(19) Europäisches Patentamt
European Patent Office
Office européen des brevets

(11) Veröffentlichungsnummer: **0 050 298**
**A2**

(12) # EUROPÄISCHE PATENTANMELDUNG

(21) Anmeldenummer: **81108273.4**

(22) Anmeldetag: **13.10.81**

(51) Int. Cl.³: **C 07 D 405/06**, C 07 D 405/14, C 07 D 409/14, A 61 K 31/41 // C07D233/54, C07D231/12, C07D249/08, C07D217/04, C07D265/30, C07D279/12, C07D317/18, C07D333/28

(30) Priorität: **16.10.80 DE 3039087**

(43) Veröffentlichungstag der Anmeldung: **28.04.82 Patentblatt 82/17**

(84) Benannte Vertragsstaaten: **AT BE CH DE FR GB IT LI NL**

(71) Anmelder: **HOECHST AKTIENGESELLSCHAFT, Postfach 80 03 20, D-6230 Frankfurt/Main 80 (DE)**

(72) Erfinder: **Blume, Ernst, Dr., Im Hainpfad 10, D-6232 Bad Soden am Taunus (DE)**
Erfinder: **Schaper, Wolfgang, Dr., Rauenthaler Weg 18, D-6000 Frankfurt am Main 71 (DE)**
Erfinder: **Raether, Wolfgang, Dr., Falkensteinstrasse 6, D-6072 Dreieich (DE)**
Erfinder: **Dittmar, Walter, Dr., Uhlandstrasse 10, D-6238 Hofheim am Taunus (DE)**

(54) **1-(1,3-Dioxolan-2-ylmethyl)-azole, ihre Salze, Verfahren zu ihrer Herstellung und ihre Verwendung.**

(57) Es werden 1-(1,3-Dioxolan-2-ylmethyl)-azole der allgemeinen Formel

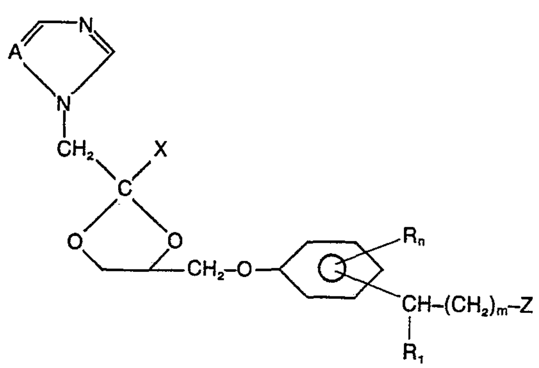

sowie ihre Stereoisomeren und ihre Salze mit physiologisch verträglichen Säuren beschrieben, die Herstellung dieser Verbindungen, sie enthaltende pharmazeutische Zubereitungen und ihre Verwendung gegen Mykosen, Protozoen und grampositive sowie gramnegative Bakterien.

EP 0 050 298 A2

HOECHST AKTIENGESELLSCHAFT HOE 80/F 237 Dr.KM/mh

1-(1,3-Dioxolan-2-ylmethyl)-azole, ihre Salze, Verfahren zu ihrer Herstellung und ihre Verwendung

Die Erfindung betrifft den in den Ansprüchen gekennzeichneten Gegenstand.

In den US-Patentschriften 3 936 470, 853 726, 4 101 664, 4 101 665, 4 101 666, 4 156 008, den BE-PSen 835 579, 837 831, den DE-OS 2 602 770, 2 803 870, 2 804 096, 2 930 029, 2 930 196, sowie in der EP-A 0 007 696 sind 1-(1,3-Dioxolan-2-ylmethyl)-1H-imidazole und -1H-1,2,4-triazole zur Bekämpfung von Pilzen und Bakterien beschrieben; ihre Wirkung und Verträglichkeit ist jedoch nicht immer ganz befriedigend. Von diesen Verbindungen unterscheiden sich die erfindungsgemäßen Verbindungen wesentlich durch die Art der Substituenten in der 4-Stellung der Dioxolangruppe.

Überraschenderweise zeigen die erfindungsgemäßen 1-(1,3-Dioxolan-2-ylmethyl)-azole eine bessere und breitere antimykotische Wirksamkeit als die bekannten 1-(1,3-Dioxolan-2-ylmethyl)-1H-imidazole und -1H-1,2,4-triazole und als das bekannte 2-S,(R)-(2,4-Dichlorphenyl)-2-(imidazol-1-yl-methyl)-4-R, (S)-/4-(4-acetylpiperazin-1-yl)-phenoxymethyl7-1,3-dioxolan (Ketoconazol).

In diesem Zusammenhang ist
unter dem Ausdruck
"Halothienyl-Rest" ein in 2- oder 3-Stellung verknüpfter Thienylrest, der in beliebiger Position mit einem Halogenatom, vorzugsweise Brom oder Chlor substituiert sein kann,
unter dem Ausdruck
"$C_1$-$C_4$-Alkyl" ein unverzweigter oder verzweigter Kohlenwasserstoffrest mit 1 - 4 Kohlenstoffatomen, wie z.B. der Methyl-, Ethyl-, Propyl-, 1-Methylethyl-, Butyl-, 2-Methylpropyl- oder 1,1-Dimethylethyl-Rest, unter dem Ausdruck "$C_1$-$C_8$-Alkyl" ein unverzweigter oder verzweigter Kohlen wasserstoffrest mit 1 - 8 Kohlenstoffatomen, wie z.B. die vorstehend genannten Reste oder eine Pentyl-, Hexyl-, Heptyl-, Oktyl- oder 1,1-Dimethyl-3,3-dimethyl-butyl-Gruppe, unter dem Ausdruck "$C_3$-$C_8$-Cycloalkyl" ein unverzweigter cyclischer Kohlenwasserstoffrest mit 3 - 8 Kohlenstoffatomen, "$C_1$-$C_4$-Alkoxy" eine Alkoxygruppe, deren Kohlenwasserstoffrest die unter dem Ausdruck "$C_1$-$C_4$-Alkyl" angegebene Bedeutung hat, unter dem Ausdruck "$C_1$-$C_5$-Alkanoyl" ein unverzweigter oder verzweigter Alkanoylrest mit 1 - 5 Kohlenstoffatomen, wie die Formyl-, Acetyl-, Propanoyl-, 2-Methylpropanoyl-, Butanoyl-, 2-Methylbutanoyl- und 2,2-Dimethylpropanoyl-Gruppe, unter dem Ausdruck "$C_3$-$C_5$-Alkenyl" ein unverzweigter oder verzweigter Alkenrest mit 3 - 5 Kohlenstoffatomen, wie z.B. der Propen-1-yl-3-, 3-Methylpropen-1-yl-3-, 1-Methylpropen-1-yl-3-, 1,1-Dimethylpropen-1-yl-3-, Buten-1-yl-4-, Penten-1-yl-5- oder der Penten-3-yl-5-Rest, unter dem Ausdruck "Halogen" ein Fluor-, Chlor-, Brom- oder Jod-atom zu verstehen.

Bevorzugt sind Verbindungen der allgemeinen Formel (I), in der X eine unsubstituierte oder mit 1 oder 2 Halogenatomen oder einem Methyl-, Methoxy- oder Trifluoromethyl-Rest substituierte Phenylgruppe, eine unsubstituierte oder mit Halogen monosubstituierte Thienylgruppe, wobei wie vorstehend Halogen vorzugsweise Fluor, Chlor oder Brom bedeuten, oder eine Naphthylgruppe bedeutet.

Unter den Verbindungen der allgemeinen Formel (I), für die $n > 1$ ist, sind diejenigen bevorzugt, in denen $R_n$ gleich oder verschieden ist und Halogen, insbesondere Chlor oder Brom, $C_1$-$C_4$-Alkyl- oder $C_1$-$C_4$-Alkoxy-Reste bedeutet.

Unter den Verbindungen der allgemeinen Formel (I), für die m 0 ist sind diejenigen bevorzugt, für die $R_1$ ein Wasserstoffatom darstellt.

Stärker bevorzugt sind Verbindungen der allgemeinen Formel (I), in denen X eine mit 1 oder 2 Halogenatomen, vorzugsweise Fluor, Chlor oder Brom substituierte Phenylgruppe oder eine Trifluormethylphenyl-, 4-Methyl- oder 4-Methoxyphenylgruppe bedeutet, $n > 3$ und $m \leq 1$ ist, wobei $R_1$ vorzugsweise Wasserstoff bedeutet, wenn m = 1 ist.

Noch stärker bevorzugt sind Verbindungen der allgemeinen Formel (I), in denen X einen 4-Fluorphenyl-, 4-Chlorphenyl-, 4-Bromphenyl-, 4-Trifluormethylphenyl-, 2,4-Dichlorphenyl-Rest, insbesondere eine 2,4-Dichlorphenyl- oder 4-Chlorphenyl-Gruppe darstellt, $n \leq 2$, m = 0 und A eine Methingruppe ist und $R_1$ Wasserstoff bedeutet.

Am stärksten bevorzugt sind solche Verbindungen der allgemeinen Formel I im Anspruch 1 sowie ihre Stereoisomeren und ihre Salze mit physiologisch verträglichen Säuren, in denen D einen 1-(1,3-Dioxolan-2-ylmethyl)-azol-Rest wie in Anspruch 1 bedeutet, wobei A Methin, X 4-Fluorphenyl, 4-Chlorphenyl, 4-Bromphenyl, 4-Trifluormethylphenyl oder 2,4-Dichlorphenyl, insbesondere 2,4-Dichlorphenyl oder 4-Chlorphenyl,
und wobei in Formel I weiterhin die einzelnen Substituenten $R_n$ unabhängig voneinander Halogen, $C_1$-$C_4$-Alkyl, Methoxy oder Allyl bedeuten, $R_1$ Wasserstoff bedeutet, n = 0 oder 2 ist, m 0 bedeutet, und Z entweder

a) einen Aminorest der allgemeinen Formel Z(a) bedeutet,

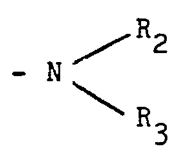

Z(a)

in der $R_2$ und $R_3$ gleich oder verschieden sind und

jeweils Wasserstoff, $C_1$-$C_8$-Alkyl, Allyl, $C_3$-$C_8$-Cycloalkyl oder eine gegebenenfalls 1 oder 2 Substituenten tragende Phenyl- bzw. Benzylgruppe bedeuten, wobei die Substituenten gleich oder verschieden sind und jeweils Halogen, $C_1$-$C_4$-Alkyl, $C_1$-$C_4$-Alkoxy oder Trifluormethyl bedeuten, oder einer der beiden Reste $R_2$ oder $R_3$ $C_1$-$C_5$-Alkanoyl bedeutet, oder

b) einen Rest der allgemeinen Formel Z(b) bedeutet,

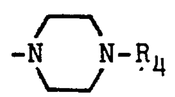 Z(b)

in der $R_4$ Wasserstoff, $C_1$-$C_4$-Alkyl, Allyl, $C_1$-$C_5$-Alkanoyl-, Methylsulfonyl, Methoxycarbonyl, $C_1$-$C_4$-Alkylaminothiocarbonyl, oder eine Phenyl-, Phenylmethyl- oder Benzoyl-Gruppe bedeutet, wobei jede der Phenylgruppen gegebenenfalls 1 oder 2 Substituenten tragen, die gleich oder verschieden sind und Halogen, Trifluormethyl, $C_1$-$C_4$-Alkyl, insbesondere $C_1$-$C_2$-Alkyl, oder $C_1$-$C_4$-Alkoxy, insbesondere $C_1$-$C_2$-Alkoxy bedeuten, oder

c) einen Piperidin-1-yl-, Morpholin-4-yl-,Thiomorpholin-4-yl-, 2,6-Dimethylmorpholin-4-yl, 2,6-Dimethylthiomorpholin-4-yl-Rest bedeutet, oder

d) einen Rest der allgemeinen Formel Z(f')

$$-NH-\overset{O}{\overset{\|}{C}}-R_5 \qquad Z(f')$$

in der $R_5$ $C_1$-$C_4$-Alkyl, oder eine gegebenenfalls 1 oder 2 Substituenten tragende Phenylgruppe darstellt, wobei die Substituenten gleich oder verschieden sind und jeweils Halogen, Trifluormethyl, $C_1$-$C_4$-Alkyl, insbesondere $C_1$-$C_2$-Alkyl, oder $C_1$-$C_4$-Alkoxy, insbesondere $C_1$-$C_2$-Alkoxy bedeuten.

Unter diesen Verbindungen gemäß der Erfindung wirken z.B. insbesondere günstig
2-S, (R)-(2,4-Dichlorphenyl)-2-(imidazol-1-ylmethyl),
4-R, (S)-/4-(4-chlorbenzoylaminomethyl)-2,6-dimethyl-phenoxymethyl/-1,3-dioxolan,

2-S, (R)-(2,4-Dichlorphenyl)-2-(imidazol-1-ylmethyl)-4-R, (S)-/4-(morpholin-4-ylmethyl)-phenoxymethyl7-1,3-dioxolan,
2-S, (R)-(2,4-Dichlorphenyl)-2-(imidazol-1-ylmethyl)-4-R, (S)-/4-(N-octylaminomethyl)-phenoxymethyl7-1,3-dioxolan,
2-S, (R)-(2,4-Dichlorphenyl)-2-(imidazol-1-ylmethyl)-4-R, (S)-/4-(4-acetylpiperazin-1-ylmethyl)-phenoxymethyl71,3-dioxolan,
2-S, (R)-(2,4-Dichlorphenyl)-(2-imidazol-1-ylmethyl)-4-R, (S)-/2,6-dimethyl-4-(N,N-dimethylaminomethyl)-phenoxymethyl7-1,3-dioxolan,
2-S, (R)-(2,4-Dichlorphenyl)-2-(imidazol-1-ylmethyl)-4-R, (S)-/2,6-dimethyl-4-(2,6-dimethylmorpholin-4-ylmethyl)-phenoxymethyl7-1,3-dioxolan,
2-S, (R)-(2,4-Dichlorphenyl)-2-(imidazol-1-ylmethyl)-4-R, (S)-/2-chlor-4-(2,6-dimethylmorpholin-4-ylmethyl)-6-methyl-phenoxymethyl7-1,3-dioxolan,
2-S, (R)-(2,4-Dichlorphenyl)-2-(imidazol-1-ylmethyl)-4-R, (S)-/2,6-dimethyl-4-(2,6-dimethylthiomorpholin-4-ylmethyl)-phenoxymethyl7-1,3-dioxolan
wie sie nachstehend in Tabelle 1 unter den Nummern 1.232, 1.238, 1.171, 1.207, 1.73, 1.239, 1.240 und 1.241 aufgeführt sind.

Die Verbindungen der Formel (I) können nach den Verfahren der Ansprüche 2, 3 oder 4 hergestellt werden.

Zur Durchführung des ersten Verfahrens (nach Anspruch 2) wird eine Verbindung der allgemeinen Formel (II)

$$D - CH_2 - E \qquad (II)$$

in der D die zur Formel (I) in Anspruch 1 angegebene Bedeutung hat und E einen reaktionsfähigen Esterrest wie Methyl-, Phenyl-, 4-Methylphenyl-, 4-Chlorphenyl-, Trifluormethylsulfonyloxy, Trifluoracetyloxy oder Halogen, insbesondere Chlor, Brom oder Jod bedeutet,
mit einer Verbindung der allgemeinen Formel (III) umgesetzt,

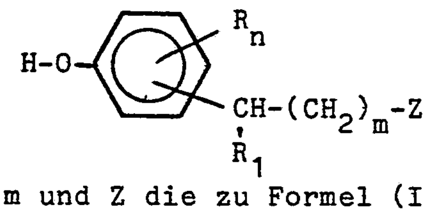

(III)

in der $R_n$, $R_1$, m und Z die zu Formel (I) in Anspruch 1 angegebenen Bedeutungen haben.

Die vorgenannte Umsetzung wird in einem Temperaturbereich von 30 bis 150°C, vorzugsweise 40 bis 100°C in Anwesenheit einer Base und in einem inerten organischen Lösungsmittel, wie N,N-Dimethylformamid, N,N-Dimethylacetamid, Dimethylsulfoxid, N-Methylpyrrolidon-2, Dioxan, Tetrahydrofuran, 4-Methyl-2-pentanon, Methanol, Ethanol, Isopropylalkohol, Propanol, Butanol, Pentanol, Tert.-butylalkohol, Methylglykol, Methylenchorid oder einem aromatischen Kohlenwasserstoff wie Benzol, Chlorbenzol, Toluol oder Xylol oder in Wasser durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -alkoholate oder -hydride wie z.B. Natriumcarbonat, Natriumhydrogencarbonat, Kaliumcarbonat, Natriumhydroxid, Natriummethylat oder Natriumhydrid, oder organische Basen z.B. tertiäre Amine wie Triethylamin, Tributylamin, Ethylmorpholin oder Pyridin, Dimethylaminopyridin, Chinolin oder 1,5-Diazabicyclo/5,4,0/undec-5-en(1,8-7) (DBU), 1-H-Imidazol oder 1-H-1,2,4-Triazol.

Die Umsetzung erfolgt ebenso unter den Bedingungen einer Phasentransferreaktion, in dem man die Reaktionspartner in einem der vorstehenden Lösungsmittel, unter kräftigem Rühren in Gegenwart eines Phasentransferkatalysators und entweder

einem gepulverten Alkalihydroxid, wie z.B. Natrium- oder Kaliumhydroxid oder einer konzentrierten wässrigen Lösung derselben, vorzugsweise in einem Temperaturbereich von 20 bis 120°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenäther, wie z.B. 12-Crown-4, 15-Crown-5, 18-Crown-6 oder Dibenzo-18-crown-6.

Falls in der vorgenannten Formel (I) der Rest Z einen Aminorest der allgemeinen Formel Z(a) in Anspruch 1 bedeutet, in der mindestens einer der Reste $R_2$ oder $R_3$ ein Wasserstoffatom, aber keiner der Reste eine $C_1$-$C_5$-Alkanoyl- oder $C_1$-$C_4$-Alkoxycarbonyl-Gruppe darstellt (vgl. nachfolgende Formel (Ia-2)) oder eine unsubstituierte 1-Piperazinylgruppe (vgl. nachfolgende Formel (I-b)), ist es vorteilhaft, in dem vorgenannten ersten Herstellungsverfahren ein Phenol der allgemeinen Formel (III) einzusetzen, dessen freie Amino- bzw. Piperazinyl-Gruppe durch eine entsprechende Schutzgruppe Sg geschützt ist (vgl. nachfolgende Formeln (III-a) und (III-b)). Dadurch wird eine N-Alkylierung vermieden. Aus den so erhaltenen Verbindungen (vgl. nachfolgende Formeln (Ia-1) und (Ia-3)) wird die Schutzgruppe Sg durch alkalische Hydrolyse abgespalten. Geeignete Schutzgruppen sind beispielsweise $C_1$-$C_4$-Alkanoyl-, insbesondere Formyl-, Acetyl- oder Trifluoracetyl- oder $C_1$-$C_4$-Alkoxycarbonyl-, insbesondere der Methoxycarbonyl-Rest.

(II) +
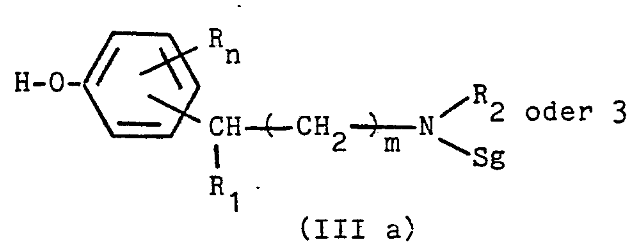

(III a)

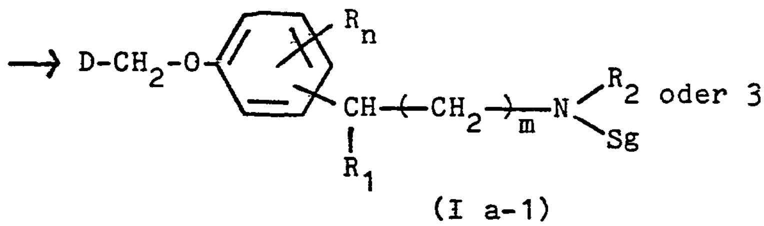


(I a-1)

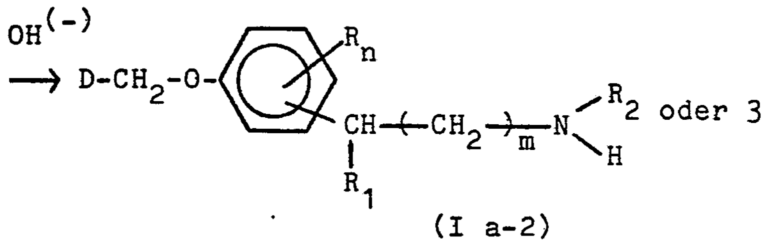


(I a-2)

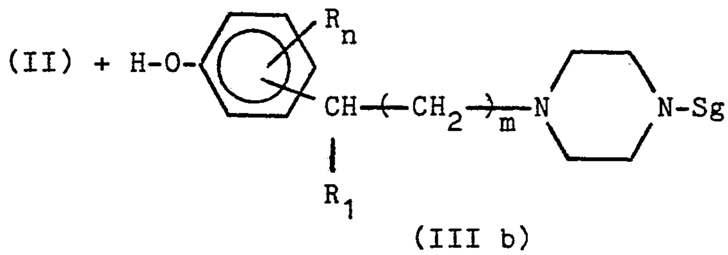


(III b)

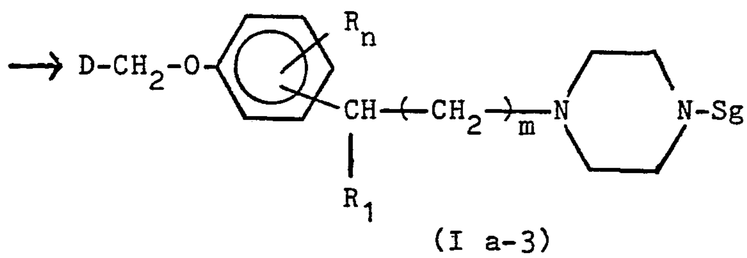

(I a-3)

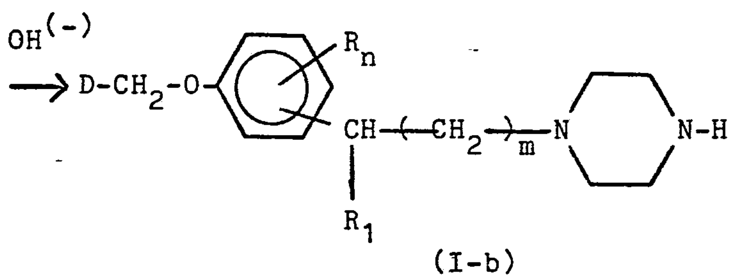


(I-b)

Eine so erhaltene Verbindung der Formel (I), die in dem Substituenten Z in Gestalt von Z(a) oder Z(b) in Anspruch 1, ein oder zwei Wasserstoffatome enthält (vgl. Formeln (Ia-2) und (Ib)), wird gegebenenfalls mit einem üblichen Acylierungsmittel acyliert oder mit einem üblichen Alkylierungsmittel alkyliert, gewünschtenfalls in Gegenwart von Schwefelkohlenstoff.

So wird vorzugsweise eine Verbindung der allgemeinen Formel (Ib)

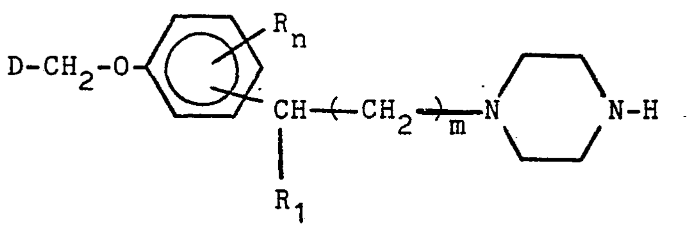
(Ib)

in der D, $R_n$, $R_1$ und m die vorstehenden Bedeutungen haben, gegebenenfalls in Gegenwart einer Base, mit einer Verbindung der allgemeinen Formel (IX)

$$R_6 - N = C = Y \qquad (IX)$$

in der Y Sauerstoff oder Schwefel bedeutet, und $R_6$ die zur Formel (Ic) genannten Bedeutungen hat, zu einer Verbindung der allgemeinen Formel (Ic)

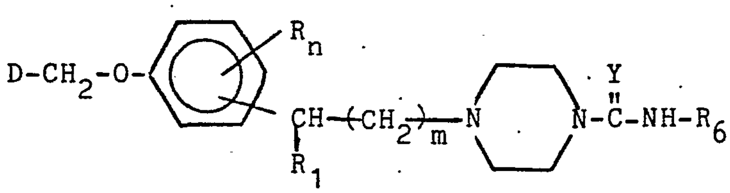
(Ic)

umgesetzt, in der D, $R_1$, $R_n$ und m die in Anspruch 1 angegebenen Bedeutungen haben,

Y Sauerstoff oder Schwefel,

$R_6$ Wasserstoff, $C_3$-$C_5$-Alkenyl, $C_1$-$C_4$-Alkyl, oder eine gegebenenfalls 1 oder 2 Substituenten tragende Phenylgruppe darstellt, wobei die Substituenten gleich oder verschieden sind und jeweils Halogen, Trifluormethyl, $C_1$-$C_4$-Alkyl, bevorzugt $C_1$-$C_2$-Alkyl, oder $C_1$-$C_4$-Alkoxy-, insbesondere $C_1$-$C_2$-Alkoxy bedeuten.

Die vorstehende Umsetzung erfolgt mit oder ohne Lösungsmittel, vorzugsweise in einem inerten Lösungsmittel gegebenenfalls in Gegenwart einer Base, wie zum ersten Herstellverfahren vorstehend angegeben, bei erhöhter Temperatur.

Falls $R_6$ Wasserstoff darstellt, ist es zweckmäßig, ein entsprechendes Alkalimetall-cyanat oder -rhodanid in einer organischen Säure wie z.B. Essigsäure oder einem Alkohol wie z.B. Ethanol, gegebenenfalls unter Zusatz von Wasser, mit einer Verbindung der allgemeinen Formel (Ib), oder dessen Hydrochlorid umzusetzen, wobei zunächst aus dem Alkalicyanat bzw. -rhodanid, Isocyan- bzw. Cyan-säure oder Rhodanwasserstoffsäure (Verbindung der allgemeinen Formel (IX), in der $R_6$ Wasserstoff bedeutet), freigesetzt wird.

Weiter wird eine Verbindung der allgemeinen Formel (Id)

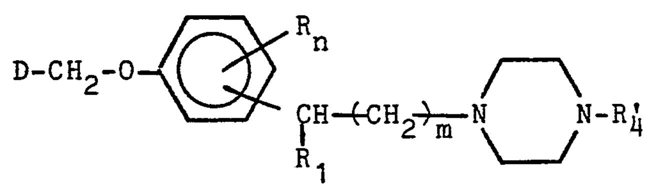

(Id)

in der D, $R_n$, $R_1$ und m die in Anspruch 1 angegebenen Bedeutungen haben, und $R_4'$ einen $C_1$-$C_5$-Alkanoyl-, Mono-, Di- oder Trihalogenmethylcarbonyl-, $C_1$-$C_4$-Alkylsulfonyl-, $C_1$-$C_4$-Alkoxycarbonyl-, $C_3$-$C_5$-Alkenylaminocarbonyl-, Mono- und Di-($C_1$-$C_4$)-Alkylaminocarbonyl-, Phenoxycarbonyl-, Phenylaminocarbonyl- bzw. Benzoyl-Rest bedeutet, wobei jede der Phenylgruppen gegebenenfalls 1 oder 2 Substituenten tragen, die gleich oder verschieden sind und Halogen, Trifluorme-

thyl-, $C_1$-$C_4$-Alkyl-, insbesondere $C_1$-$C_2$-Alkyl-, $C_1$-$C_4$-Alkoxy-, bevorzugt $C_1$-$C_2$-Alkoxy-Reste bedeuten, vorzugsweise hergestellt, indem man eine Verbindung der allgemeinen Formel (Ib) mit einem entsprechenden Acylhalogenid, das sich von der entsprechenden Carbon- oder Sulfonsäure ableitet, in Gegenwart einer geeigneten Base, in einem inerten Lösungsmittel, in einem Temperaturbereich von -10 bis 120°C umsetzt, wobei insbesondere bei $C_2$-$C_5$-Carbonsäuren auch deren Anhydride als Acylierungsmittel eingesetzt werden.

Als Lösungsmittel und Basen werden vorzugsweise die zum ersten Herstellverfahren genannten verwendet.

Bedeutet $R_4'$ eine Formylgruppe, wird die Acylierung mit Ameisensäure, bevorzugt mit Ameisensäuremethyl- oder -ethylester durchgeführt. Stellt $R_4'$ einen $C_1$-$C_4$-Alkoxy- oder einen Phenoxycarbonyl-Rest dar, wird als Acylierungsmittel ein entsprechender Ester der Kohlensäure eingesetzt.

Weiter wird eine Verbindung der allgemeinen Formel (Id')

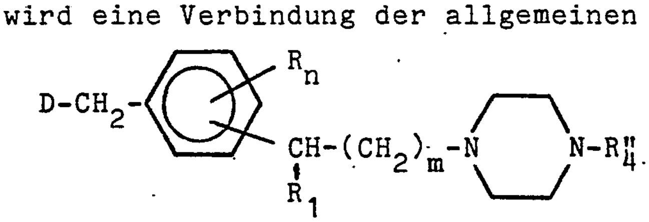

(Id')

in der D, $R_n$, $R_1$ und m die in Anspruch 1 angegebenen Bedeutungen haben, und $R_4''$ $C_1$-$C_4$-Alkyl, $C_2$-$C_5$-Alkanoylmethyl, Hydroxy-($C_2$-$C_3$)-alkyl, insbesondere Hydroxyethyl, $C_1$-$C_4$-Alkoxycarbonylmethyl, Aminocarbonylmethyl, $C_1$-$C_4$-Alkylaminocarbonylmethyl, oder Phenylmethyl darstellt, wobei die Phenylgruppe gegebenenfalls 1 oder 2 Substituenten tragen kann, die unabhängig voneinander Halogen, $C_1$-$C_4$-Alkyl, insbesondere $C_1$-$C_2$-Alkyl, oder $C_1$-$C_4$-Alkoxy, bevorzugt $C_1$-$C_2$-Alkoxy bedeuten,

vorzugsweise hergestellt, indem man eine Verbindung der allgemeinen Formel (Ib) mit einer Verbindung der allgemeinen Formel (X),

$$E - R_4'' \qquad (X)$$

in der E die zur Formel (II) angegebenen und $R_4''$ die vorstehenden Bedeutungen haben, alkyliert. Die Alkylierung erfolgt bevorzugt in einem inerten Lösungsmittel in Gegenwart einer Base wie sie zum ersten Herstellverfahren aufgeführt sind, in einem Temperaturbereich von 20 bis 120°C.

Solche Verbindungen der allgemeinen Formel (Id'), in der $R_4''$ einen Hydroxyethylrest darstellt, werden bevorzugt durch Umsetzen einer Verbindung der allgemeinen Formel (Ib) mit Ethylenoxid hergestellt, beispielsweise durch Einleiten des letzteren in eine heiße Lösung der Verbindung der allgemeinen Formel (Ib) in einem, zum ersten Herstellverfahren aufgeführten, organischen Lösungsmittel, oder man erhitzt eine Verbindung der allgemeinen Formel (Ib) mit Äthylencarbonat vorzugsweise auf 50 - 150°C, gegebenenfalls unter Zusatz eines Alkalicarbonates wie z.B. Natriumcarbonat, in Substanz oder vorzugsweise in einem inerten organischen Lösungsmittel, wie zum ersten Herstellverfahren aufgeführt, z.B. Dimethylformamid.

Solche Verbindungen der allgemeinen Formel (Id'), in der $R_4''$ $C_1$-$C_4$-Alkoxy-($C_2$-$C_3$)-alkyl, insbesondere $C_1$-$C_4$-Alkoxyethyl bedeutet, werden bevorzugt aus den vorstehend beschriebenen Verbindungen der allgemeinen Formel (Id'), in der $R_4''$ Hydroxyethyl bedeutet, durch Alkylierung mit einer Verbindung der allgemeinen Formel (X) hergestellt, in der E die zur Formel (II) angegebenen Bedeutungen hat und $R_4''$ $C_1$-$C_4$-Alkyl bedeutet. Die Alkylierung erfolgt bevorzugt in einem inerten Lösungsmittel in Gegenwart einer Base, wie sie zum ersten Herstellverfahren aufgeführt sind, in einem Temperaturbereich von 20 bis 120°C.

Solche Verbindungen der allgemeinene Formel (Id'), in der $R_4''$ entweder eine $C_1$-$C_4$-Alkyl- oder 1 oder 2 Substituenten am Phenylkern tragende Phenylmethyl-Gruppe darstellt, wobei die Substituenten vorstehende Bedeutungen haben, werden bevorzugt durch reduktive Aminierung aus einer Verbindung der allgemeinen Formel (Ib) und einem Aldehyd oder Keton hergestellt, in dem man die Reaktionspartner in einem inerten Lösungsmittel beispielsweise einem Alkohol wie zum ersten Herstellverfahren genannt, in Anwesenheit eines Hydrierungs-Katalysators wie z.B. Palladium auf Holzkohle und einer Base, wie z.B. Natriumacetat, oder katalytischen Mengen einer Säure wie z.B. Essigsäure, Chlorwasserstoffsäure, Phosphorsäure oder Schwefelsäure, mit Wasserstoff behandelt. Die Reaktion erfolgt vorzugsweise bei Normaldruck bis 150 atü in einem Temperaturbereich zwischen 20 und 150°C.

Solche Verbindungen der allgemeinen Formel (Id'), in der $R_4''$ einen $C_1$-$C_4$-Alkylaminocarbonylmethylrest bedeutet, werden vorzugsweise aus den entsprechenden Verbindungen der allgemeinen Formel (Id') hergestellt, in denen $R_4''$ eine $C_1$-$C_4$-Alkoxycarbonylmethyl-Gruppe darstellt, in dem man die letztgenannten Verbindungen mit einem $C_1$-$C_4$-Alkylamin in Substanz oder vorteilhaft in einem zum ersten Herstellverfahren angegebenen Lösungsmittel bei 20 - 150°C umsetzt.

Weiter wird eine Verbindung der allgemeinen Formel (Ie),

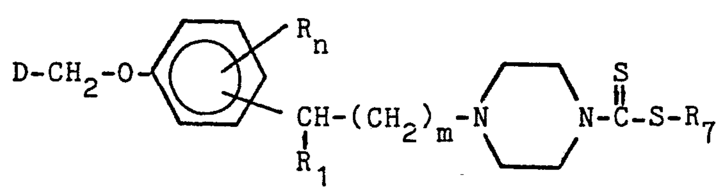

(Ie)

in der D, $R_n$, $R_1$ und m die in Anspruch 1 angegebenen Bedeutungen haben, $R_7$ einen $C_1$-$C_4$-Alkylrest bedeutet,

vorzugsweise hergestellt, in dem man eine Verbindung der allgemeinen Formel (Ib) in einem Lösungsmittel in Gegenwart einer Base und Schwefelkohlenstoff mit einer Verbindung der allgemeinen Formel (X) alkyliert, in der E die zur Formel (II) angegebenen Bedeutungen hat und $R''_4$ $C_1$-$C_4$-Alkyl bedeutet. Die verwendeten Basen, Lösungsmittel und Reaktionsbedingungen sind zum ersten Herstellverfahren angegeben.

Weiter wird eine Verbindung der allgemeinen Formel (If),

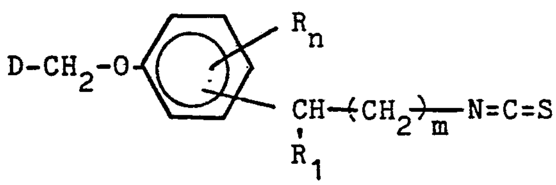

(If)

in der D, $R_n$, $R_1$ und m die in Anspruch 1 angegebenen Bedeutungen haben,
vorzugsweise hergestellt, in dem man eine Verbindung der allgemeinen Formel (Ia-4),

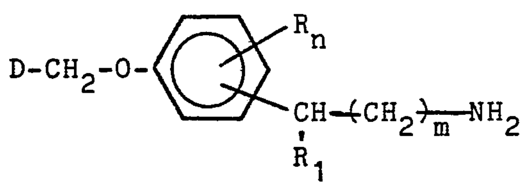

(Ia-4)

in der D, $R_n$, $R_1$ und m die in Anspruch 1 angegebenen Bedeutungen haben, in das Isothiocyanat der allgemeinen Formel (If) überführt.

Dazu wird eine Verbindung der allgemeinen Formel (Ia-4) in einem inerten Lösungsmittel in Gegenwart einer Base wie zum ersten Herstellverfahren genannt, entweder mit Thiophosgen umgesetzt, oder in Gegenwart von Schwefelkohlenstoff acyliert, wobei zweckmäßig Carbodiimide oder Chlorameisensäureester verwendet werden.

So wird z.B. eine Verbindung der allgemeinen Formel (Ia-4) mit Schwefelkohlenstoff entweder in Pyridin mit N-N'-Dicyclohexylcarbodiimid, oder in Methylenchlorid in Gegenwart von Triethylamin mit Chlorameisensäuremethylester umgesetzt.

Weiter wird eine Verbindung der allgemeinen Formel (Ig)

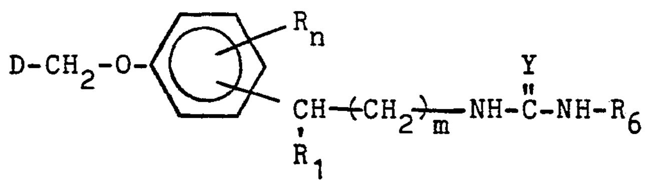

(Ig)

in der D, $R_n$, $R_1$, m und Y die in Anspruch 1 angegebenen Bedeutungen haben und $R_6$ die zu Formel (Ic) angegebenen Bedeutungen hat,
vorzugsweise hergestellt, in dem man eine Verbindung der allgemeinen Formel (Ia-4) mit
einer Verbindung der allgemeinen Formel (IX)

$$R_6 - N = C = Y \qquad (IX)$$

umsetzt, in der Y Sauerstoff oder Schwefel darstellt, und $R_6$ die zur Formel (Ic) angegebenen Bedeutungen hat.

Die Reaktionsbedingungen sind die gleichen wie vorstehend zur Herstellung von Verbindungen der Formel (Ic) durch Umsetzung von Verbindungen der Formel (Ib) mit Verbindungen der Formel (IX) angegeben.

Weiter wird eine Verbindung der allgemeinen Formel (Ih)

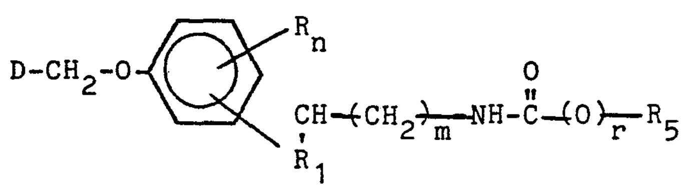

(Ih)

in der D, $R_n$, $R_1$, m, r und $R_5$ die in Anspruch 1 angegebenen Bedeutungen haben,
vorzugsweise hergestellt, in dem man eine Verbindung der allgemeinen Formel (Ia-4) mit einem Acylierungsmittel umsetzt.

Geeignete Acylierungsmittel zur Herstellung von Verbindungen der allgemeinen Formel (Ih), in der r die Zahl 0 bedeutet, sind Acyl-halogenide und -anhydride, abgeleitet von der Säure $R_5$-COOH, wobei für die Formylierung zweckmäßig Ameisensäure oder z.B. Ameisensäureethylester verwendet werden.

Geeignete Acylierungsmittel zur Herstellung von Verbindungen der allgemeinen Formel (Ih), in der r die Zahl 1 bedeutet, sind Esterchloride der Kohlensäure oder Di-$R_5$-Carbonate, wobei $R_5$ die zur Formel (Ih) angegebene Bedeutung hat.

Die Reaktionsbedingungen sind die gleichen wie vorstehend zur Herstellung von Verbindungen der Formel (Id) durch Umsetzung von Verbindungen der Formel (Ib) mit einem Acylhalogenid oder -anhydrid angegeben.

Weiter wird eine Verbindung der allgemeinen Formel (Ii)

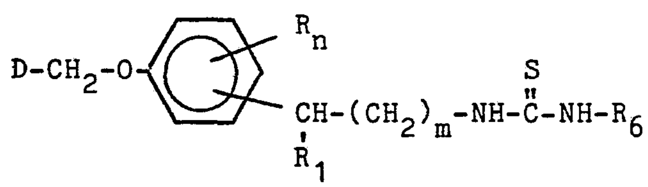

(Ii)

in der D, $R_n$, $R_1$ und m die in Anspruch 1 angegebenen Bedeutungen haben,
vorzugsweise hergestellt, in dem man eine Verbindung der allgemeinen Formel (If) mit einem Amin $R_6$-$NH_2$ umsetzt, in dem $R_6$ die zu Formel (Ic) genannten Bedeutungen hat.

Die Reaktionsbedingungen sind die gleichen wie vorstehend zur Herstellung von Verbindungen der Formel (Ic) durch Umsetzung von Verbindungen der Formel (Ib) mit Verbindungen der Formel (IX) angegeben.

Weiter wird eine Verbindung der allgemeinen Formel (Ik)

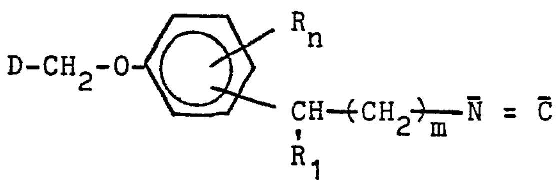

(Ik)

in der D, $R_n$, $R_1$ und m die in Anspruch 1 angegebenen Bedeutungen haben, vorzugsweise hergestellt, in dem man eine Verbindung der allgemeinen Formel (Ia-4) unter den Bedingungen einer Phasentransferreaktion (wie vorstehend beschrieben) mit Chloroform oder Bromoform umsetzt, oder in dem man eine Verbindung der allgemeinen Formel (Ia-5)

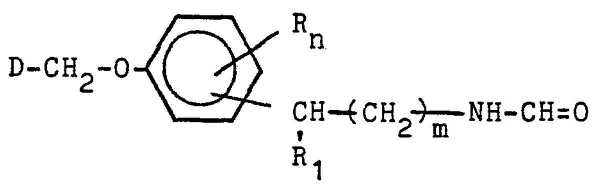

(Ia-5)

in der D, $R_n$, $R_1$ und m die in Anspruch 1 angegebenen Bedeutungen haben, mit einem Acylierungsmittel wie z.B. Phosgen, Thionylchlorid, Phosphortrichlorid, Phosphortribromid, Phosphoroxychlorid, Phosphorpentachlorid, Phosphorpentoxid, Cyanurchlorid, Benzol- oder Toluolsulfochlorid oder mit äquimolaren Mengen Triphenylphosphin und Tetrachlorkohlenstoff, jeweils in Gegenwart einer Base und in einem inerten Lösungsmittel wie zum ersten Herstellverfahren angegeben, in einem Temperaturbereich von -50 bis 50°C, umsetzt.

So wird z.B. vorteilhaft eine Verbindung der allgemeinen Formel (Ia-5) in Methylenchlorid in Gegenwart von Triäthylamin mit Phosphoroxychlorid umgesetzt.

Herstellung der Ausgangsstoffe

Zur Herstellung von Verbindungen der allgemeinen Formel II:

Die Ausgangsverbindungen der allgemeinen Formel (II), in der D die in Anspruch 1 angegebene Bedeutung hat und A eine Methingruppe bedeutet, sind in der BE-PS 837 831, der DE-OS 2 804 096 und in J.Med.Chem. 1979, 22, S. 1003 beschrieben; solche in der A Stickstoff bedeutet, können in Analogie zu den oben zitierten Literaturstellen hergestellt werden (vgl. auch Formelschema S. 23).

Zur Herstellung von Verbindungen der allgemeinen Formel (III)

Viele Ausgangsverbindungen der allgemeinen Formel (III) sind bekannt.

Diejenigen Phenole der allgemeinen Formel (III), in der $R_n$ und $R_1$ die in Anspruch 1 angegebenen Bedeutungen haben, Z einen Rest der allgemeinen Formel Z(a), Z(b) oder Z(c) in Anspruch 1 bedeutet (im folgenden Z' genannt) und m = 0 ist, sind leicht zugängliche Phenol-Mannich-Basen.

Sie werden durch Umsetzung eines entsprechenden Phenols mit einem Aldehyd der allgemeinen Formel (XI) und einer Aminoverbindung der allgemeinen Formel (XII) hergestellt (vgl. dazu H. Hellmann und G. Opitz, " -Aminoalkylierung", Verlag Chemie Weinheim (1969)),

$$R_1 - CH = O \quad (XI) \qquad\qquad H - Z' \quad (XII)$$

wobei in den vorstehenden allgemeinen Formeln (XI) und (XII) $R_1$ die in Anspruch 1 und Z' die zu Formel Z(a) , Z(b) oder Z(c) in Anspruch 1 angegebenen Bedeutungen haben.

Weiter werden sie durch Umaminierung der vorstehend beschriebenen Phenol-Mannich-Basen hergestellt, indem man bevorzugt Dimethyl- oder Diethylamino-Phenol-Mannich-Basen mit einer Aminoverbindung der allgemeinen Formel (XII), in der Z' die vorstehenden Bedeutungen hat, in Substanz oder vorzugsweise in einem inerten organischen Lösungsmittel, wie zum ersten Herstellungsverfahren angegeben, auf 50 bis 180°C erhitzt.

Diejenigen Phenole der allgemeinen Formel (III), in der m = 0, n = 0, $R_1$ die in Anspruch 1 angegebenen Bedeutungen hat und Z die Bedeutung von Z' zur Formel (XII) hat, werden vorzugsweise durch reduktive Aminierung von Hydroxybenzaldehyden oder entsprechenden Hydroxyphenylketonen mit primären oder sekundären Aminen der allgemeinen Formel (XII) hergestellt, in der Z' die vorstehenden Bedeutungen hat.

Die Reaktionsbedingungen entsprechen hierbei denen, wie sie zur Synthese von Verbindungen der allgemeinen Formel (Id') durch reduktive Aminierung von Verbindungen der allgemeinen Formel (Ib) mit Aldehyden oder Ketonen, vorstehend beschrieben sind.

Diejenigen Phenole der allgemeinen Formel (III), in denen m = 0, $R_1$ nicht Wasserstoff ist und im übrigen die in Anspruch 1 angegebenen Bedeutungen hat und Z die Bedeutung von Z'zur Formel (XII) hat, werden vorzugsweise analog zu den in Eur.J.Med.Chem. 1979, S. 227-245 beschriebenen Methoden hergestellt.

Diejenigen Phenole der allgemeinen Formel (III), in denen m die Zahl 1 oder 2 bedeutet, $R_n$ und $R_1$ die in Anspruch 1 angegebenen Bedeutungen haben, und Z die Bedeutung von Z' zur Formel (XII) hat, werden zweckmäßig durch Reduktion von entsprechenden Methoxyphenylalkylcarbonsäureamiden mit einem geeigenten Reduktionsmittel wie z.B. Lithiumaluminiumhydrid hergestellt. Die dabei entstehenden Methoxyphenylalkylamine werden zweckmäßig durch Etherspaltung mit

einer starken Säure wie z.B. Bromwasserstoffsäure in Phenole der allgemeinen Formel (III) überführt, oder man überführt wie allgemein üblich ein Methoxyphenylalkanol in einen reaktionsfähigen Ester der allgemeinen Formel (XIII)

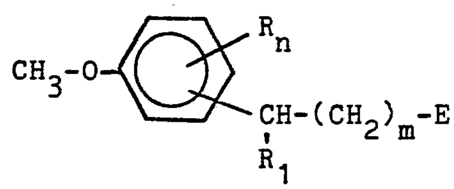

(XIII)

in der $R_n$ und $R_1$ die in Anspruch 1, E die zur Formel II angegebenen Bedeutungen haben und m die Zahl 1 oder 2 bedeutet,
und läßt auf diesen eine Aminoverbindung der allgemeinen Formel (XII) einwirken, in der Z' die zur Formel Z(a), Z(b), oder Z(c) in Anspruch 1 angegebenen Bedeutungen hat. Das so erhaltene Methoxyphenylalkylamin wird durch Etherspaltung wie vorstehend beschrieben in ein Phenol der allgemeinen Formel (III) überführt.

Wenn die phenolische Hydroxylgruppe bei vorstehend beschriebenen Synthesen zur Herstellung von Phenolen der allgemeinen Formel (III) stört, ist es zweckmäßig, diese Gruppe zunächst durch eine geeignete Schutzgruppe zu blokkieren, die später wieder abgespalten wird. Beispielsweise kann das geschützte Phenol in Form seiner Methoxyverbindung, Benzyloxyverbindung oder Acyloxyverbindung in Synthesen eingesetzt werden und anschließend die Methoxyverbindung durch Behandeln mit einer entsprechenden starken Säure, wie z.B. Bromwasserstoffsäure, die Benzyloxyverbindung durch Hydrogenolyse mit einem Katalysator wie z.B. Palladium auf Kohle und die Acyloxyverbindung durch alkalische oder saure Hydrolyse zu dem entsprechenden Phenol gespalten werden.

Zur Durchführung des zweiten Verfahrens (nach Anspruch 3) wird eine Verbindung der allgemeinen Formel (IV),

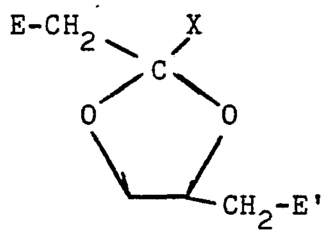

(IV)

in der X die in Anspruch 1 und E und E' die zur Formel (II) in Anspruch 2 für E angegebenen Bedeutungen haben, zunächst mit einer Verbindung der allgemeinen Formel (III) in Anspruch 2 umgesetzt und hierbei eine Verbindung der allgemeinen Formel (V) hergestellt,

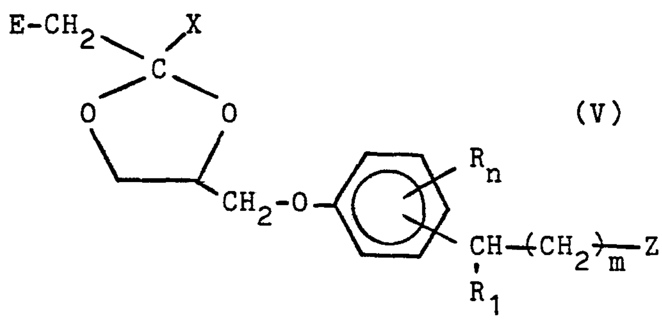

(V)

in der X, $R_n$, $R_1$, m und Z die in Anspruch 1, und E die zur Formel (II) in Anspruch 2 angegebenen Bedeutungen haben.

Verbindungen der Formel (I) werden nach diesem Verfahren dadurch hergestellt, daß man anschließend eine Verbindung der allgemeinen Formel (V) mit Verbindungen der allgemeinen Formel (VI) umsetzt,

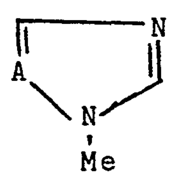

(VI)

in der A die in Anspruch 1 angegebene Bedeutung hat und Me Wasserstoff oder ein Metallatom bedeutet.
Die Reaktionsbedingungen zur Herstellung der Verbindungen der allgemeinen Formel (V) durch Umsetzung von Verbindungen der

allgemeinen Formel (IV) mit Verbindungen der allgemeinen Formel (III) sind die gleichen, wie im ersten Herstellverfahren zur Herstellung von Verbindungen der allgemeinen Formel (I) durch Umsetzung von Verbindungen der allgemeinen Formel (II) mit (III) angegeben.

Die Herstellung von Verbindungen der allgemeinen Formel (I) (nach dem zweiten Verfahren), durch Umsetzung von Verbindungen der allgemeinen Formel (V) mit Verbindungen der allgemeinen Formel (VI), erfolgt zweckmäßig in einem inerten Lösungsmittel in Gegenwart einer Base, wie zum ersten Herstellverfahren vorstehend angegeben, vorzugsweise in einem Temperaturbereich von 100 bis 190°C. Die Umsetzung erfolgt zweckmäßig in Anwesenheit eines Alkalijodides wie z.B. Natrium- oder Kaliumjodid, gegebenenfalls in einem Autoklaven unter Druck.

Die vorstehend beschriebenen Umsetzungen werden zweckmäßig als Eintopfreaktion durchgeführt, indem man zunächst bei 40 bis 100°C eine Verbindung der allgemeinen Formel (IV) mit einer Verbindung der allgemeinen Formel (III) in Gegenwart einer Base in einem inerten Lösungsmittel umsetzt. Anschließend wird ohne Isolierung der Verbindung der allgemeinen Formel (V) eine Verbindung der allgemeinen Formel (VI) und gegebenenfalls ein weiteres Moläquivalent einer Base und ein Alkalijodid (z.B. Kaliumjodid) zugegeben und auf 100 bis 190°C erhitzt.

Eine so erhaltene Verbindung der allgemeinen Formel (I), die in dem Substituenten Z in Gestalt von Z(a) oder Z(b) in Anspruch 1, ein oder zwei Wasserstoffatome enthält (vgl. Formeln (Ia-2) und (Ib), wird gegebenenfalls mit einem üblichen Acylierungsmittel acyliert oder mit einem üblichen Alkylierungsmittel alkyliert, gewünschtenfalls in Gegenwart von Schwefelkohlenstoff, wie im ersten Herstellverfahren nach Anspruch 2 beschrieben ist.

Herstellung der Ausgangsstoffe:

Zur Herstellung von Verbindungen der allgemeinen Formel (IV):

Verbindungen der allgemeinen Formel (IV), in der E und E' die zur allgemeinen Formel (II) für E angegebenen Bedeutungen haben, werden hergestellt, indem man eine Verbindung der allgemeinen Formel (XVI) (vgl. nachfolgendes Formelschema) in üblicher Weise in eine reaktionsfähige Estergruppe überführt.

So werden z.B. die Verbindungen der allgemeinen Formel (IV) (vgl. Formelschema) hergestellt, in der E die zur allgemeinen Formel (II) in Anspruch 2 angebenen Bedeutungen hat und E' Methylsulfonyloxy bedeutet, indem man eine Verbindung der allgemeinen Formel (XVI), in der X die in Anspruch 1 und E die zur allgemeinen Formel (II) angebenen Bedeutungen hat, mit Methylsulfonylchlorid bei -10 bis +50°C umsetzt (vgl. nachfolgendes Formelschema).
Die vorstehende Umsetzung erfolgt zweckmäßig in einem inerten Lösungsmittel in Gegenwart einer Base, wie zum ersten Herstellverfahren angegeben.
Solche Verbindungen der allgemeinen Formel (IV), in der E' Chlor oder Brom bedeutet, werden z.B. durch Umsetzung von Verbindungen der allgemeinen Formel (XVI) (vgl. nachfolgendes Formelschema) mit entsprechenden Halogenierungsmitteln wie z.B. Thionylchlorid, Phosphorpenta-chlorid oder -bromid, Phosphoroxy-chlorid oder -bromid, hergestellt.
Die vorstehende Umsetzung erfolgt gegebenenfalls in einem inerten Lösungsmittel, wie zum ersten Herstellverfahren vorstehend angegeben, bei 0 bis 100°C.
Weiter können solche Verbindungen der allgemeinen Formel (IV), in der E die zur allgemeinen Formel (II) angegebenen Bedeutungen hat und E' Chlor oder Brom bedeutet, hergestellt werden, indem man eine Verbindung der allgemeinen Formel (XIV) (vergl. Formelschema) mit 1-Chlor-2,3-propandiol oder 1-Brom-2,3-propandiol umsetzt.

Die Umsetzung erfolgt in einem inerten Lösungsmittel, wie vorstehend beim ersten Herstellverfahren angegeben, in Anwesenheit einer geeigneten starken Säure, wie z.B. 4-Methylphenylsulfonsäure, Benzolsulfonsäure, Naphthalin-1,5-disulfonsäure, Eisessig oder Schwefel- oder Phosphor-säure in einem Temperaturbereich von 80 bis 180°C.
Vorteilhaft wird ein Lösungsmittelgemisch verwendet, bestehend aus einem inerten Lösungsmittel, das mit Wasser ein azeotropes Gemisch bildet, wie z.B. Benzol, Toluol, Xylol, Chlorbenzol oder Cyclohexan und einem Alkohol, wie vorstehend beim ersten Herstellverfahren angegeben.

Zur Herstellung von Verbindungen der allgemeinen Formel (XVI):

Verbindungen der allgemeinen Formel (XVI), in der X die in Anspruch 1 und E die zur allgemeinen Formel (II) angegebenen Bedeutungen haben,
werden hergestellt, indem man eine Verbindung der allgemeinen Formel (XIV), in der X und E die vorstehenden Bedeutungen haben, mit Glycerin umgesetzt (vgl. nachfolgendes Formelschema).

Die Reaktionsbedingungen sind die gleichen wie zur Herstellung von Verbindungen der allgemeinen Formel (IV) durch Umsetzung von Verbindungen der allgemeinen Formel (XIV) mit 1-Chlor-2,3-propandiol vorstehend angegeben.
Weiter können Verbindungen der allgemeinen Formel (XVI), in der X die in Anspruch 1 angegebene Bedeutung hat und E Halogen bedeutet, hergestellt werden, indem man eine Verbindung der allgemeinen Formel (XV), in der X die in Anspruch 1 angegebene Bedeutung hat, mit Glycerin umsetzt und die so erhaltene Verbindung der allgemeinen Formel (XVII) (vgl. nachfolgendes Formelschema) mit Halogen umsetzt, z.B. mit Brom, in Analogie zu J.Med.Chem. 1979, 22, S. 1003.

Das 1. und 2. Herstellverfahren werden durch folgendes Formelschema erläutert.

Formelschema

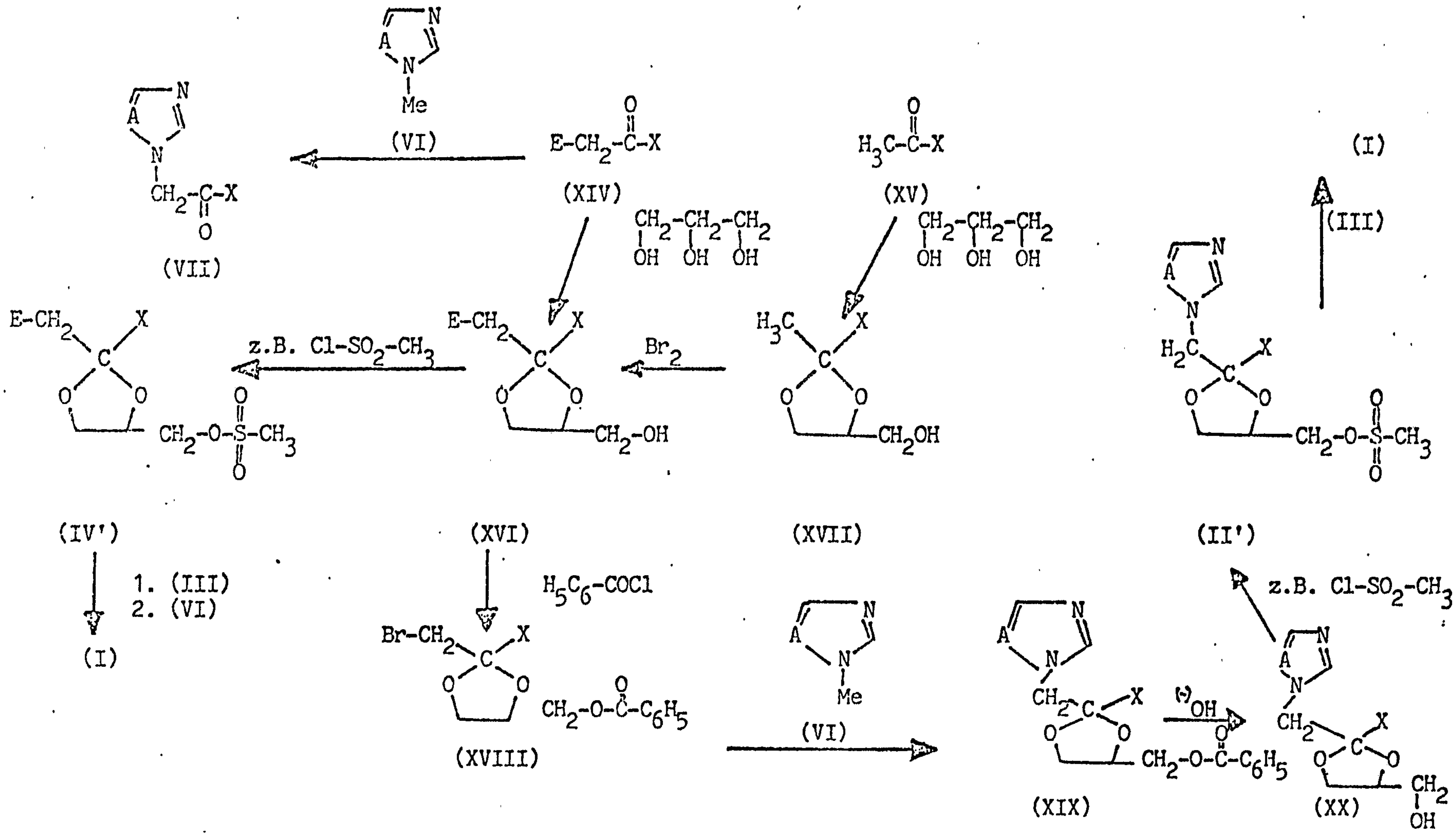


(VI) (VII) (XIV) (XV) (I) (III) (IV') (XVI) (XVII) (II') (I) (XVIII) (VI) (XIX) (XX)

Zur Durchführung des dritten Verfahrens (nach Anspruch 4) wird eine Verbindung der allgemeinen Formel (VII),

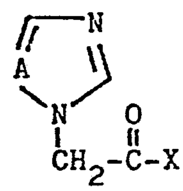

(VII)

in der A und X die in Anspruch 1 angebenen Bedeutungen haben, mit einer Verbindung der allgemeinen Formel (VIII) oder dem entsprechenden Epoxid der allgemeinen Formel (VIII')

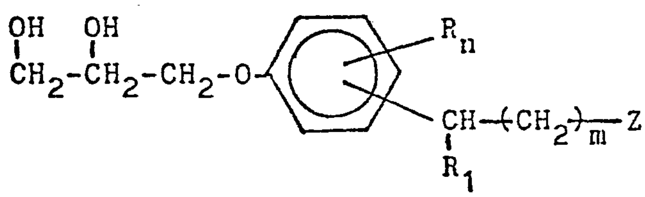

(VIII)

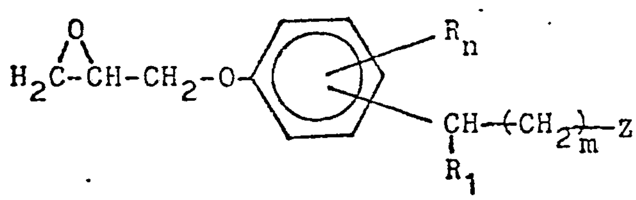

(VIII')

umgesetzt.

In den vorstehenden allgemeinen Formeln (VIII) und (VIII') haben $R_n$, $R_1$, m und Z die in Anspruch 1 angegebenen Bedeutungen.

Die Umsetzung von Verbindungen der allgemeinen Formel (VII) mit Verbindungen der allgemeinen Formel (VIII) wird im allgemeinen unter denselben Bedingungen durchgeführt, wie im zweiten Herstellverfahren bei der Herstellung von Verbindungen der allgemeinen Formel (IV) durch Umsetzung von Verbindungen der allgemeinen Formel (XIV) mit 1-Chlor-2,3-propandiol beschrieben. Bei der entsprechenden Reaktion von Verbindungen der allgemeinen Formel (VII) mit Verbindungen

der allgemeinen Formel (VIII') ist es zweckmäßig, in einem inerten Lösungsmittel, wie beim ersten Herstellverfahren beschrieben, in Anwesenheit einer geeigneten starken Säure, wie beim zweiten Herstellverfahren erläutert, zu arbeiten.

Eine so erhaltene Verbindung der allgemeinen Formel (I), die in dem Substituenten Z in Gestalt von Z(a) oder Z(b) in Anspruch 1, ein oder zwei Wasserstoffatome enthält (vgl. Formeln (Ia-2) und(Ib)), wird gegebenenfalls mit einem üblichen Acylierungsmittel acyliert oder mit einem üblichen Alkylierungsmittel alkyliert, gewünschtenfalls in Gegenwart von Schwefelkohlenstoff, wie im ersten Herstellverfahren nach Anspruch 2 beschrieben.

Herstellung der Ausgangsstoffe:

Zur Herstellung von Verbindungen der allgemeinen Formel (VII):

Verbindungen der allgemeinen Formel (VII), in der A und X die in Anspruch 1 angegebenen Bedeutungen haben, werden zweckmäßig durch Umsetzung von Verbindungen der allgemeinen Formel (XIV) mit (VI) hergestellt, sofern sie nicht bekannt sind.
Die Umsetzung erfolgt in einem inerten Lösungsmittel, gegebenenfalls in Anwesenheit einer Base, in einem Temperaturbereich von -10 bis +100°C. Die verwendeten Basen und Lösungsmittel sind zum ersten Herstellverfahren angegeben.

Zur Herstellung von Verbindungen der allgemeinen Formel (VIII):

Verbindungen der allgemeinen Formel (VIII), in der $R_n$, $R_1$, m und Z die in Anspruch 1 angegebenen Bedeutungen haben, werden durch Umsetzung von Verbindungen der allgemeinen Formel (III) mit 1-Halogen-2,3-propandiol hergestellt, in analoger Weise wie in Org. Synth. Collect. Vol. I, S. 296 beschrieben.

Zur Herstellung von Verbindungen der allgemeinen Formel (VIII'):

Verbindungen der allgemeinen Formel (VIII'), in der $R_n$, $R_1$, m und Z die in Anspruch 1 angegebenen Bedeutungen haben, können durch Umsetzung von Verbindungen der allgemeinen Formel (III) mit Epichlorhydrin hergestellt werden, in analoger Weise wie in J. Chem. Soc. 1954, S. 1571, beschrieben.

Die Verbindungen der allgemeinen Formel (I) weisen ihre wesentlichen Eigenschaften auch in Form ihrer Salze auf. Zur Herstellung von Säureadditionssalzen kommen alle physiologisch verträglichen Säuren in Frage. Hierzu gehören vorzugsweise die Halogenwasserstoffsäuren, wie z.B. Chlorwasserstoff- und Bromwasserstoff-säure, sowie Salpetersäure, ferner Phosphorsäure oder Schwefelsäure. Bevorzugte organische Säuren sind mono- und bifunktionelle Carbonsäuren und Hydroxycarbonsäuren wie z.B. Essigsäure, Maleinsäure, Oxalsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure, Methyl- und Phenylsulfonsäure sowie 1,5-Naphthalindisulfonsäure.
Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure oder Salpetersäure, erhalten werden und in bekannter Weise, z.B. durch Abfiltrieren, isoliert und gegebenenfalls durch Waschen oder Umkristallisation mit einem inerten organischen Lösungsmittel gereinigt werden.

Aus der allgemeinen Formel (I) ist ersichtlich, daß die erfindungsgemäßen Verbindungen mindestens zwei asymmetrische Kohlenstoffatome aufweisen, die sich in 2- und 4-Stellung des Dioxolanringes befinden. Diese Verbindungen können demnach in Form verschiedener Stereoisomeren vorliegen.

Die diastereomeren Racemate (cis- oder trans-Form gemäß den in C.A., Bd. 76 (1972), Index Guide, Section IV, 203 beschriebenen Regeln) der Verbindung der allgemeinen Formel (I) können in üblicher Weise getrennt werden. Geeignete Methoden sind beispielsweise die selektive Kristallisation und die Chromatographie, z.B. die Säulenchromatographie.

Da die stereochemische Konfiguration bereits in den Zwischenprodukten der allgemeinen Formel (II) bzw. (V) vorgegeben ist, kann die Trennung in die cis- und trans-Form bereits auf dieser Stufe oder bevorzugt noch früher, beispielsweise auf der Stufe der Zwischenprodukte der allgemeinen Formel (IV) erfolgen. Die Trennung ist nach vorstehend beschriebenen Methoden leicht möglich. Die cis- und trans-diastereomeren Racemate können ihrerseits in üblicher Weise in ihre optischen Antipoden cis(+), cis(-) bzw. trans(+) und trans(-) getrennt werden.

Die neuen Verbindungen der Formel (I) sind Chemotherapeutika und besitzen eine dem eingangs erwähnten "Ketoconazol" überlegene Wirkung und Verträglichkeit gegen Pilzinfektionen. Sie sind in vitro sehr gut wirksam gegen Hautpilze, wie z.B. Trichophyton mentagrophytes, Microsporum canis, Epidermophytes floccosum; Schimmelpilze, wie z.B. Aspergillus niger oder Hefen, wie z.B. Candida albicans, C.tropicalis, Torulpsis glabrata und Trichosporon cutaneum oder Trichomonas vaginalis oder T.fetus oder grampositive und gramnegative Bakterien.

Auch in vivo, z.B. bei der experimentellen Nierencandidose der Maus besitzen die Verbindungen nach oraler oder parenteraler Anwendung einen sehr guten systemischen Effekt z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als geeignete Anwendungsform der erfindungsgemäßen Verbindung kommen beispielsweise in Frage, Tabletten oder Kapseln, Suspensionen, Lösungen, Gelees, Cremes oder Salben sowie Aerosole in Form von Spray.
Die Anwendungskonzentration für Lösungen, Gelees, Cremes oder Salben sowie Aerosole in Form von Spray beträgt im allgemeinen zwischen 0,1 - 3 Gewichtsprozenten.
Die orale Anwendung erfolgt in pharmazeutisch üblichen Zubereitungen, beispielsweise in Form von Tabletten oder Kapseln, die pro Tagesdosis 50 - 200 mg des Wirkstoffes in Mischung mit einem gebräuchlichen Trägerstoff und/oder Konstituents enthalten.
Für die lokale Anwendung können z.B. Suspensionen, Lösungen, Gelees, Cremes, Salben oder Suppositorien verwendet werden.
Für die parenterale Anwendung kommen geeignete Suspensionen oder Lösungen in Frage in einer Anwendungskonzentration zwischen 0,1 - 5 Gewichtsprozenten.

Die Beispiele erläutern die Erfindung.

<u>Beispiele für das erste Herstellverfahren nach Anspruch 2:</u>

<u>Beispiel 1</u>

Cis-2-(2,4-Dichlorphenyl)-2-(1H-imidazol-1-ylmethyl)-4-[4-chlor-2-(piperidin-1-ylmethyl)-phenoxymethyl]-1,3-dioxolan und Dinitrat

a) Herstellung der freien Verbindung

Zur Suspension aus 0,3 g = 10 mmol NaH (80 %ige Öldispersion) in 20 ml abs. Dimethylformamid gibt man unter Rühren und Kühlen mit einem Eisbad 2,25 g = 10 mmol 4-Chlor-2-(piperidin-1-ylmethyl)-phenol portionsweise so zu, daß die Temperatur 20°C nicht übersteigt, rührt bei 20°C nach bis die Wasserstoffentwicklung beendet ist, trägt anschließend 4,07 g = 10 mmol Cis-2-(2,4-Dichlorphenyl)-2-(1H-imidazol-1-ylmethyl)-1,3-dioxolan-4-ylmethylmethansulfonat portionsweise ein und heizt 8

Stunden auf 80°C. Nach dem Abkühlen wird das Lösungsmittel im Ölpumpenvakuum am Rotationsverdampfer abgezogen, der Rückstand in 100 ml Wasser aufgenommen, zweimal mit 30 ml Methylenchlorid extrahiert, die vereinigten Methylenchloridphasen mit 30 ml Wasser nachgewaschen, über Natriumsulfat getrocknet und im Vakuum eingedampft. Der erhaltene Rückstand (5,4 g) wird säulenchromatographisch an Kieselgel (Kieselgel S, Riedel-de Haën) mit einer Mischung aus Methylenchlorid : Ethanol, 10 : 1 als Laufmittel gereinigt. Die nach Dünnschichtchromatogramm (Kieselgel, Methylenchlorid : Ethanol, 10 : 1) einheitlichen Fraktionen werden vereinigt und das Lösungsmittel abgezogen. Man erhält 4,6 g (86 % d.Th.) als hochviskoses, nicht kristallines Öl.

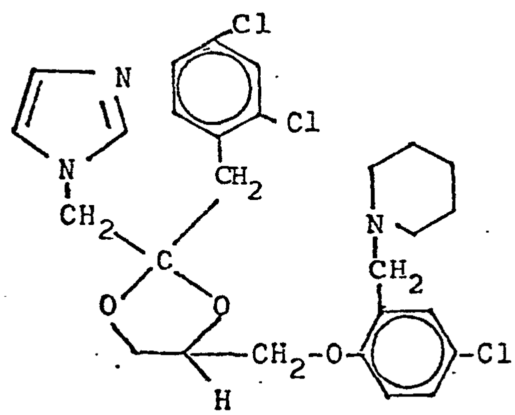

| | | | | |
|---|---|---|---|---|
| Analyse: | Ber.: | C: 58,17 | Gef.: | C: 57,5 |
| $C_{26}H_{28}Cl_3N_3O_3$ | | H: 5,26 | | H: 4,9 |
| | | N: 7,83 | | N: 7,6 |

MG: 536,86

Bei gleicher Arbeitsweise wie vorstehend beschrieben können auch Dimethylsulfoxid, Dimethylacetamid oder N-Methylpyrrolidon-2, sowie Gemische dieser Lösungsmittel mit z.B. Tetrahydrofuran, Dioxan, Dimethoxyethan oder Toluol verwendet werden.

b) Herstellung des Dinitrates:

2 g = 3,7 mmol des vorstehend beschriebenen Öles löst man in 60 ml Essigester und tropft unter Rühren bei 20°C die Lösung von 0,31 ml = 0,47 g = 7,4 mmol 100 %ige $HNO_3$ in 20 ml Essigester, läßt 15 Stunden bei 20°C stehen, zieht das Lösungsmittel im Vakuum am Rotationsverdampfer ab und kristallisiert den Rückstand aus Acetonitril/Essigester um. Man erhält 1,2 g (49 % d.Th.) cis-2-(2,4-Dichlorphenyl)-2-(1H-imidazol-1-ylmethyl)-4-/4-chlor-2-(piperidin-1-ylmethyl)-phenoxymethyl/-1,3-dioxolan als Dinitrat.

(Fp.: 140°C Zersetzung).

| | | | | |
|---|---|---|---|---|
| Analyse: | Ber.: | C: 47,11 | Gef.: | C: 46,8 |
| $C_{26}H_{30}Cl_3N_5O_9$ | | H: 4,56 | | H: 4,7 |
| | | N: 10,56 | | N: 10,4 |

MG: 662,89.

Beispiel 2

gemäß Beispiel 1, jedoch unter Verwendung äquivalenter Mengen der entsprechenden Verbindungen der allgemeinen Formel (II) und (III) können die folgenden, in Tabelle 1 aufgeführten, Verbindungen der allgemeinen Formel (I) hergestellt werden.

0050298

## Tabelle 1

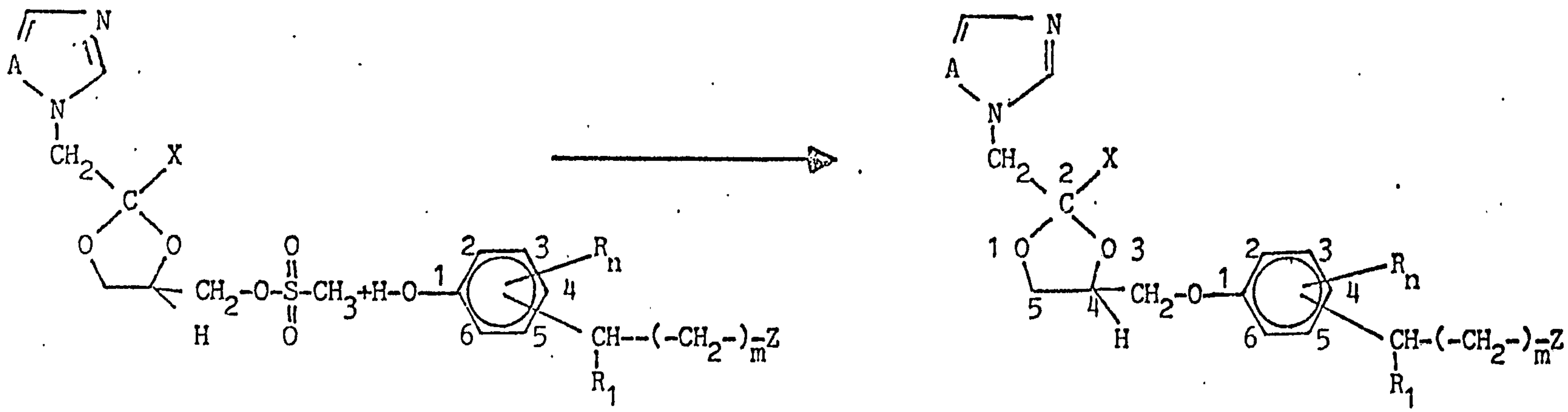

(II) (III) (I)

a) v gibt in der Tabelle die Position des Restes $-\underset{R_1}{CH}-(CH_2-)_m-Z$ an

b) Cis- und Trans- bezieht sich auf den Azolylmethyl-Rest und den Phenoxy-Rest in der 2- bzw. 4-Stellung des Dioxolanringes

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.1 | CH | $2,4-Cl_2C_6H_3$ | 0 | | 2 | H | 0 | $-N(CH_3)_2$ | Cis | Base | | | |
| 1.2 | CH | $2,4-Cl_2C_6H_3$ | 0 | | 3 | H | 0 | $-N(CH_3)_2$ | Cis | Base | 74-76 | C: 59,78<br>H: 5,45<br>N: 9,08 | C: 59,2<br>H: 5,4<br>N: 8,7 |
| 1.3 | CH | $2,4-Cl_2C_6H_3$ | 0 | | 4 | H | 0 | $-N(CH_3)_2$ | Cis | Base | | C: 59,75<br>H: 5,45<br>N: 9,09 | C: 60,0<br>H: 5,2<br>N: 8,8 |
| 1.4 | CH | $2,4-Cl_2C_6H_3$ | 1 | 4-Cl | 2 | H | 0 | $-N(CH_3)_2$ | Cis | Base | | C: 55,61<br>H: 4,87<br>N: 8,46 | C: 55,3<br>H: 5,3<br>N: 8,2 |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.5 | CH | $2,4Cl_2C_6H_3$ | 1 | 4-Cl | 2 | H | 0 | 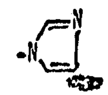 | Cis | Base | 124-126 | C: 55,45<br>H: 4,07<br>N: 10,77 | C: 54,9<br>H: 4,2<br>N: 10,4 |
| 1.6 | CH | $2,4Cl_2C_6H_3$ | 1 | 4-Cl | 2 | H | 0 | 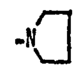 | Cis | Base | - | C: 57,40<br>H: 5,01<br>N: 8,04 | C: 56,8<br>H: 5,0<br>N: 7,8 |
| 1.7 | CH | $2,4Cl_2C_6H_3$ | 1 | 4-Cl | 2 | H | 0 | 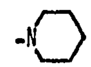 | Cis | $2 \cdot HNO_3$ | 140 | C: 47,11<br>H: 4,56<br>N: 10,56 | C: 46,8<br>H: 4,7<br>N: 10,4 |
| 1.8 | CH | $2,4Cl_2C_6H_3$ | 1 | 4-Cl | 2 | H | 0 | 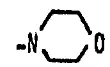 | Cis | Base | - | C: 55,73<br>H: 4,86<br>N: 7,80 | C: 55,1<br>H: 4,5<br>N: 7,6 |
| 1.9 | CH | $2,4Cl_2C_6H_3$ | 1 | 4-Cl | 2 | H | 0 | 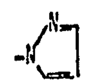 | Cis | Base | 107 | C: 55,46<br>H: 4,07<br>N: 10,78 | C: 55,9<br>H: 4,4<br>N: 11,1 |
| 1.10 | CH | $2,4Cl_2C_6H_3$ | 1 | 4-Br | 2 | H | 0 | $-N(CH_3)_2$ | Cis | Base | | | |
| 1.11 | CH | $2,4Cl_2C_6H_3$ | 1 | 4-F | 2 | H | 0 | $-N(CH_3)_2$ | Cis | Base | | | |
| 1.12 | CH | $2,4Cl_2C_6H_3$ | 1 | $4-CF_3$ | 2 | H | 0 | $-N(CH_3)_2$ | Cis | Base | | | |
| 1.13 | CH | $2,4Cl_2C_6H_3$ | 1 | $4-CH_3$ | 2 | H | 0 | $-N(CH_3)_2$ | Cis | Base | | | |
| 1.14 | CH | $2,4Cl_2C_6H_3$ | 1 | 4-tert.-butyl | 2 | H | 0 | $-N(CH_3)_2$ | Cis | Base | | | |
| 1.15 | CH | $2,4Cl_2C_6H_3$ | 1 | 4-tert.-butyl | 2 | H | 0 | 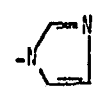 | Cis | Base | 149-150 | C: 62,16<br>H: 5,59<br>N: 10,25 | C: 62,0<br>H: 5,8<br>N: 10,3 |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.16 | CH | $2,4Cl_2C_6H_3$ | 1 | 4-tert.-butyl | 2 | H | 0 | 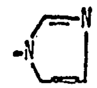 | Cis | Dinitrat | 230 | C: 50,38<br>H: 4,83<br>N: 12,60 | C: 50,4<br>H: 4,9<br>N: 12,4 |
| 1.17 | CH | $2,4Cl_2C_6H_3$ | 1 | 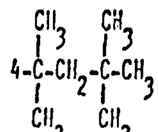 | 2 | H | 0 | $-N(CH_3)_2$ | Cis | Base | | | |
| 1.18 | CH | $2,4Cl_2C_6H_3$ | 1 | 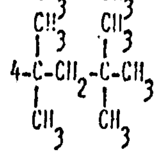 | 2 | H | 0 | 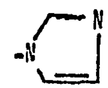 | Cis | Base | 147 | C: 64,27<br>H: 6,40<br>N: 9,37 | C: 63,9<br>H: 6,4<br>N: 9,3 |
| 1.19 | CH | $2,4Cl_2C_6H_3$ | 1 | $4-O-CH_3$ | 2 | H | 0 | $-N(CH_3)_2$ | Cis | Base | | | |
| 1.20 | CH | $2,4Cl_2C_6H_3$ | 1 | $4-O-CH_3$ | 2 | H | 0 | 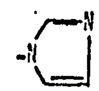 | Cis | Base | 114 | C: 58,26<br>H: 4,69<br>N: 10,87 | C: 57,8<br>H: 5,0<br>N: 10,5 |
| 1.21 | CH | $2,4Cl_2C_6H_3$ | 1 | $4-O-C_4H_9$ | 2 | H | 0 | $-N(CH_3)_2$ | Cis | Base | | | |
| 1.22 | CH | $2,4Cl_2C_6H_3$ | 3 | $2,4,5\ Cl_3$ | 6 | H | 0 | $-N(CH_3)_2$ | Cis | Base | | | |
| 1.23 | CH | $2,4Cl_2C_6H_3$ | 1 | $-\overset{O}{\overset{\|}{C}}-O-C_2H_5$ | 2 | H | 0 | 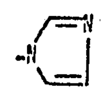 | Cis | Base | - | C: 58,18<br>H: 4,70<br>N: 10,05 | C: 58,2<br>H: 5,0<br>N: 9,7 |
| 1.24 | CH | $2,4Cl_2C_6H_3$ | 1 | $-\overset{O}{\overset{\|}{C}}-O-CH_3$ | 2 | H | 0 | $-N(CH_3)_2$ | Cis | Base | | | |
| 1.25 | CH | $2,4Cl_2C_6H_3$ | 1 | $-\overset{O}{\overset{\|}{C}}-OH$ | 2 | H | 0 | 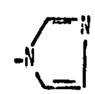 | Cis | Base | - | C: 56,76<br>H: 4,19<br>N: 10,59 | C: 56,5<br>H: 4,0<br>N: 10,2 |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.26 | CH | $2,4Cl_2C_6H_3$ | 1 | $2-CH_2-N(CH_3)_2$ | 4 | H | 0 | 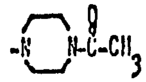 | Cis | Base | - | C: 59,80<br>H: 6,19<br>N: 11,62 | C: 59,5<br>H: 6,0<br>N: 11,1 |
| 1.27 | CH | $2,4Cl_2C_6H_3$ | 1 | $4-NO_2$ | 2 | H | 0 | $-N(CH_3)_2$ | Cis | Base | | | |
| 1.28 | CH | $2,4Cl_2C_6H_3$ | 2 | $3,4-C_4H_4$ | 2 | H | 0 | 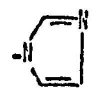 | Cis | Base | 143-144 | C: 62,01<br>H: 4,51<br>N: 10,46 | C: 62,7<br>H: 4,6<br>N: 10,4 |
| 1.29 | CH | $2,4Cl_2C_6H_3$ | 2 | $3,4-C_4H_4$ | 2 | H | 0 | $-N(CH_3)_2$ | Cis | Base | - | C: 63,28<br>H: 5,31<br>N: 8,20 | C: 63,1<br>H: 5,2<br>N: 8,0 |
| 1.30 | CH | $2,4Cl_2C_6H_3$ | 2 | $3,4-C_4H_4$ | 2 | 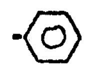 | 0 | $-N(CH_3)_2$ | Cis | Base | | | |
| 1.31 | CH | $2,4Cl_2C_6H_3$ | 2 | $3,4-C_4H_4$ | 2 | 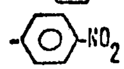 | 0 | $-N(CH_3)_2$ | Cis | Base | | | |
| 1.32 | CH | $2,4Cl_2C_6H_3$ | 2 | $3,4-C_4H_4$ | 2 | 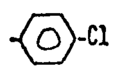 | 0 | $-N(CH_3)_2$ | Cis | Base | 75-80 | C: 63,62<br>H: 4,85<br>N: 6,75 | C: 63,7<br>H: 5,1<br>N: 6,8 |
| 1.33 | CH | $2,4Cl_2C_6H_3$ | 2 | $3,4-C_4H_4$ | 2 | 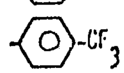 | 0 | $-N(CH_3)_2$ | Cis | Base | | | |
| 1.34 | N | $2,4Cl_2C_6H_3$ | 2 | $3,4-C_4H_4$ | 2 | H | 0 | $-N(CH_3)_2$ | Cis | Base | | | |
| 1.35 | N | $2,4Cl_2C_6H_3$ | 2 | $3,4-C_4H_4$ | 2 | 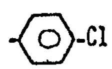 | 0 | $-N(CH_3)_2$ | Cis | Base | | | |
| 1.36 | CH | $2,4Cl_2C_6H_3$ | 1 | 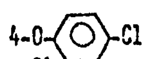 | 2 | H | 0 | $-N(CH_3)_2$ | Cis | Base | | | |
| 1.37 | CH | $2,4Cl_2C_6H_3$ | 1 | 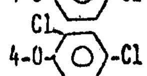 | 2 | H | 0 | $-N(CH_3)_2$ | Cis | Base | | | |
| 1.38 | CH | $2,4Cl_2C_6H_3$ | 1 | 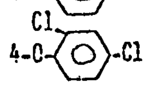 | 2 | H | 0 | 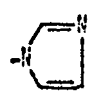 | Cis | Base | - | C: 55,75<br>H: 3,74<br>N: 8,67 | C: 55,9<br>H: 4,0<br>N: 8,7 |
| 1.39 | CH | $2,4Cl_2C_6H_3$ | 1 | 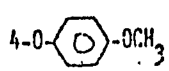 | 2 | H | 0 | $-N(CH_3)_2$ | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.40 | CH | $2,4Cl_2C_6H_3$ | 1 | 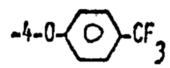 | 2 | H | 0 | $-N(CH_3)_2$ | Cis | Base | | | |
| 1.41 | CH | $2,4Cl_2C_6H_3$ | 1 | 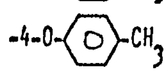 | 2 | H | 0 | $-N(CH_3)_2$ | Cis | Base | | | |
| 1.42 | CH | $2,4Cl_2C_6H_3$ | 2 | $4,6-Cl_2$ | 2 | H | 0 | 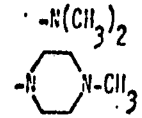 | Cis | Base | - | C: 53,26<br>H: 4,81<br>N: 9,56 | C: 52,9<br>H: 4,8<br>N: 9,3 |
| 1.43 | CH | $2,4Cl_2C_6H_3$ | 2 | $4,6-Cl_2$ | 2 | H | 0 | 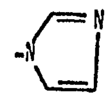 | Cis | Base | - | C: 52,03<br>H: 3,64<br>N: 10,11 | C: 51,7<br>H: 3,8<br>N: 9,8 |
| 1.44 | CH | $2,4Cl_2C_6H_3$ | 2 | $4,6-Cl_2$ | 2 | H | 0 | 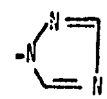 | Cis | Base | - | C: 49,77<br>H: 3,45<br>N: 12,62 | C: 50,0<br>H: 3,6<br>N: 12,3 |
| 1.45 | CH | $2,4Cl_2C_6H_3$ | 2 | 2,6-diiso-propyl | 4 | H | 0 | 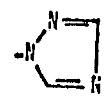 | Cis | Base | - | C: 61,06<br>H: 5,83<br>N: 12,28 | C: 61,0<br>H: 5,9<br>H: 12,1 |
| 1.46 | CH | $2,4Cl_2C_6H_3$ | 2 | 2,6-diiso-propyl | 4 | H | 0 | $-N(CH_3)_2$ | Cis | Base | | C: 63,74<br>H: 6,82<br>N: 7,69 | C: 63,5<br>H: 6,8<br>N: 7,5 |
| 1.47 | CH | $2,4Cl_2C_6H_3$ | 2 | $2-CH_3$<br>$6-CH_2-CH=CH_2$ | 4 | H | 0 | $-N(CH_3)_2$ | Cis | Base | | | |
| 1.48 | CH | $2,4Cl_2C_6H_3$ | 2 | 2-Cl<br>$6-CH_2-CH=CH_2$ | 4 | H | 0 | $-N(CH_3)_2$ | Cis | Base | | | |
| 1.49 | CH | $2,4Cl_2C_6H_3$ | 2 | 2,6-diethyl | 4 | H | 0 | $-N(CH_3)_2$ | Cis | Base | | | |
| 1.50 | CH | $2,4Cl_2C_6H_3$ | 2 | 2,6-ditert.-butyl | 4 | H | 0 | $-N(CH_3)_2$ | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.51 | CH | $2,4Cl_2C_6H_3$ | 0 | | 4 | $-C_4H_9$ | 0 | 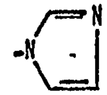 | Cis | Base | | | |
| 1.52 | CH | $2,4Cl_2C_6H_3$ | 0 | | 4 | $-C_3H_7$ | 0 | $-N(C_2H_5)_2$ | Cis | Base | | | |
| 1.53 | CH | $2,4Cl_2C_6H_3$ | 2 | 2,6-di-tert.-butyl | 4 | H | 0 | $-N(C_2H_5)_2$ | Cis | Base | | | |
| 1.54 | CH | $2,4Cl_2C_6H_3$ | 2 | 2,6-diiso-propyl | 4 | H | 0 | $-N(C_2H_5)_2$ | Cis | Base | | | |
| 1.55 | CH | $2,4Cl_2C_6H_3$ | 2 | 2,6-di-methoxy | 4 | H | 0 | $-N(CH_3)_2$ | Cis | Base | | | |
| 1.56 | CH | $2,4Cl_2C_6H_3$ | 2 | 2,6-di-methoxy | 4 | H | 0 | $-N(C_2H_5)_2$ | Cis | Base | | | |
| 1.57 | CH | $2,4Cl_2C_6H_3$ | 2 | 2,6-di-methoxy | 4 | H | 0 | 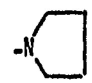 | Cis | Base | | | |
| 1.58 | CH | $2,4Cl_2C_6H_3$ | 2 | 2,6-di-ethyl | 4 | H | 0 | $-N(C_2H_5)_2$ | Cis | Base | | | |
| 1.59 | N | $2,4Cl_2C_6H_3$ | 2 | 2,6-di-ethyl | 4 | H | 0 | $-N(CH_3)_2$ | Cis | Base | | | |
| 1.60 | N | $2,4Cl_2C_6H_3$ | 2 | 2,6-diiso-propyl | 4 | H | 0 | $-N(CH_3)_2$ | Cis | Base | | | |
| 1.61 | N | $2,4Cl_2C_6H_3$ | 2 | 2,6-di-tert.-butyl | 4 | H | 0 | $-N(CH_3)_2$ | Cis | Base | | | |
| 1.62 | CH | $4-ClC_6H_4$ | 2 | 2,6-di-ethyl | 4 | H | 0 | $-N(C_2H_5)_2$ | | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.63 | CH | $4-ClC_6H_4$ | 2 | 2,6-diisopropyl | 4 | H | 0 | $-N(C_2H_5)_2$ | | Base | | | |
| 1.64 | CH | $2,4-C_6H_3$ | 2 | 2,6-diethyl | 4 | H | 0 | 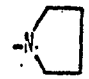 | Cis | Base | | | |
| 1.65 | CH | $2,4-C_6H_3$ | 2 | 2,6-diisopropyl | 4 | H | 0 | 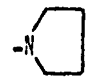 | Cis | Base | | | |
| 1.66 | CH | $2,4-C_6H_3$ | 2 | 2,6-diethyl | 4 | H | 0 | 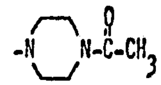 | Cis | Base | | | |
| 1.67 | CH | $2,4-C_6H_3$ | 2 | 2,6-diisopropyl | 4 | H | 0 | 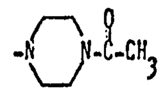 | Cis | Base | | | |
| 1.68 | CH | $2,4-C_6H_3$ | 2 | 2,6-dimethoxy | 4 | H | 0 | 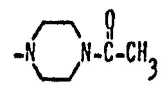 | Cis | Base | | | |
| 1.69 | CH | $2,4-Cl_2C_6H_3$ | 2 | 2,6-dibrom | 4 | H | 0 | $-N(CH_3)_2$ | Cis | Base | | | |
| 1.70 | CH | $2,4-Cl_2C_6H_3$ | 2 | 2,6-dibrom | 4 | H | 0 | $-N(C_2H_5)_3$ | Cis | Base | | | |
| 1.71 | CH | $2,4-Cl_2C_6H_3$ | 2 | 2,6-dibrom | 4 | H | 0 | 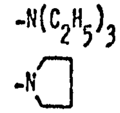 | Cis | Base | | | |
| 1.72 | CH | $2,4-Cl_2C_6H_3$ | 2 | 2,6-dibrom | 4 | H | 0 | $-N(-CH_2-CH=CH_2)_2$ | Cis | Base | | | |
| 1.73 | CH | $2,4-Cl_2C_6H_3$ | 2 | 2,6-dimethyl | 4 | H | 0 | $-N(CH_3)_2$ | Cis | Base | - | C: 61,23<br>H: 5,96<br>N: 8,57 | C: 60,8<br>H: 6,3<br>N: 8,7 |
| 1.74 | CH | $2,4-Cl_2C_6H_3$ | 2 | 2,6-dimethyl | 4 | H | 0 | $-N(C_2H_5)_2$ | Cis | Base | - | C: 62,54<br>H: 6,41<br>N: 8,10 | C: 62,0<br>H: 6,4<br>N: 7,8 |

| Vorbdgs. Nr. | A | X | n | $R_n$ | a) v | $R_1$ | m | Z | b) 2,4-Isomere | Base oder Salze | Fp.:[°C] | Analyse Ber.: | Gef.: |
|---|---|---|---|---|---|---|---|---|---|---|---|---|---|
| 1.75 | CH | 2,4-$Cl_2C_6H_3$ | 2 | 2,6-dimethyl | 4 | H | 0 | $-N(C_4H_9)_2$ | Cis | Base | | | |
| 1.76 | CH | 2,4-$Cl_2C_6H_3$ | 2 | 2,6-dimethyl | 4 | H | 0 | $-N(-CH_2-CH=CH_2)_2$ | Cis | Base | | C: 64,32<br>H: 5,96<br>N: 7,76 | C: 64,3<br>H: 6,2<br>N: 7,6 |
| 1.77 | CH | 2,4-$Cl_2C_6H_3$ | 2 | 2,6-dimethyl | 4 | H | 0 | $-NH-C_4H_9$ | Cis | Base | | | |
| 1.78 | CH | 2,4-$Cl_2C_6H_3$ | 2 | 2,6-dimethyl | 4 | H | 0 | $-NH-C_8H_{17}$ | Cis | Base | | | |
| 1.79 | CH | 2,4-$Cl_2C_6H_3$ | 2 | 2,6-dimethyl | 4 | H | 0 | $CH_3$<br>\|<br>$-N-C_4H_9$ | Cis | Base | | | |
| 1.80 | CH | 2,4-$Cl_2C_6H_3$ | 2 | 2,6-dimethyl | 4 | H | 0 | 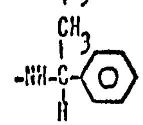 | Cis | Base | | | |
| 1.81 | CH | 2,4-$Cl_2C_6H_3$ | 2 | 2,6-dimethyl | 4 | $H_3C$ | 0 | $-N(CH_3)_2$ | Cis | Base | | | |
| 1.82 | CH | 2,4-$Cl_2C_6H_3$ | 2 | 2,6-dimethyl | 4 | H | 0 | $-N(C_3H_7)_2$ | Cis | Base | | | |
| 1.83 | CH | 2,4-$Cl_2C_6H_3$ | 2 | 2,6-dimethyl | 4 | H | 0 | $-N[-CH(CH_3)_2-]_2$ | Cis | Base | | | |
| 1.84 | CH | 2,4-$Cl_2C_6H_3$ | 2 | 2,6-dimethyl | 4 | H | 0 | -NH-tert.-butyl | Cis | Base | | | |
| 1.85 | CH | 2,4-$Cl_2C_6H_3$ | 2 | 2,6-dimethyl | 4 | H | 0 | $CH_3$<br>\|<br>$-N-CH_2-CH=CH_2$ | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.86 | CH | $2,4-Cl_2C_6H_3$ | 2 | 2,6-dimethyl | 4 | H | 0 | 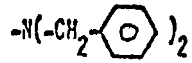 | Cis | Base | | | |
| 1.87 | CH | $2,4-Cl_2C_6H_3$ | 2 | 2,6-dimethyl | 4 | H | 0 | 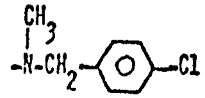 | Cis | Base | | | |
| 1.88 | CH | $2,4-Cl_2C_6H_3$ | 2 | 2,6-dimethyl | 4 | H | 0 | 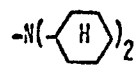 | Cis | Base | | | |
| 1.89 | CH | $2,4-Cl_2C_6H_3$ | 2 | 2,6-dimethyl | 4 | H | 0 | 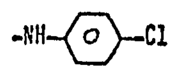 | Cis | $\cdot 2HNO_3$ | 159 | C: 49,83<br>H: 4,33<br>N: 10,02 | C: 49,6<br>H: 4,3<br>N: 10,0 |
| 1.90 | CH | $2,4-Cl_2C_6H_3$ | 2 | 2,6-dimethyl | 4 | H | 0 | 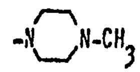 | Cis | Base | | | |
| 1.91 | CH | $2,4-Cl_2C_6H_3$ | 2 | 2,6-dimethyl | 4 | H | 0 | 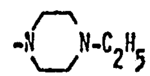 | Cis | Base | | | |
| 1.92 | CH | $2,4-Cl_2C_6H_3$ | 2 | 2,6-dimethyl | 4 | H | 0 | 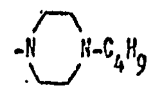 | Cis | Base | | | |
| 1.93 | CH | $2,4-Cl_2C_6H_3$ | 2 | 2,6-dimethyl | 4 | H | 0 | 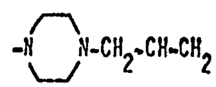 | Cis | Base | | | |
| 1.94 | CH | $2,4-Cl_2C_6H_3$ | 2 | 2,6-dimethyl | 4 | H | 0 | 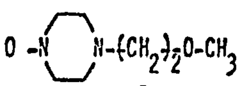 | Cis | Base | | | |
| 1.95 | CH | $2,4-Cl_2C_6H_3$ | 2 | 2,6-dimethyl | 4 | H | 0 | 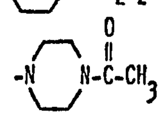 | Cis | Base | - | C: 60,74<br>H: 5,98<br>N: 9,77 | C: 60,2<br>H: 6,2<br>N: 9,8 |
| 1.96 | CH | $2,4-Cl_2C_6H_3$ | 2 | 2,6-dimethyl | 4 | H | 0 | 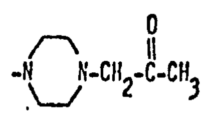 | Cis | Base | | | |

| Verbdgs. Nr. | A | X | n | $R_n$ | a) v | $R_1$ | m | Z | b) 2,4-Iso-mere | Base oder Salze | Fp.: [°C] | Analyse Ber.: | Gef.: |
|---|---|---|---|---|---|---|---|---|---|---|---|---|---|
| 1.97 | CH | 2,4-$Cl_2C_6H_3$ | 2 | 2,6-di-methyl | 4 | H | 0 | 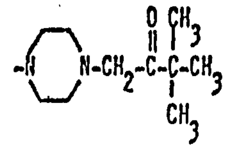 | Cis | Base | | | |
| 1.98 | CH | 2,4-$Cl_2C_6H_3$ | 2 | 2,6-di-methyl | 4 | H | 0 | 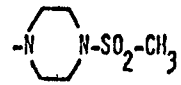 | Cis | Base | - | C: 55,17<br>H: 5,62<br>N: 9,19 | C: 54,7<br>H: 5,8<br>N: 9,3 |
| 1.99 | CH | 2,4-$Cl_2C_6H_3$ | 2 | 2,6-di-methyl | 4 | H | 0 | 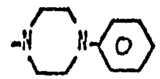 | Cis | Base | - | C: 65,23<br>H: 5,97<br>N: 9,22 | C: 64,9<br>H: 6,2<br>N: 9,1 |
| 1.100 | CH | 2,4-$Cl_2C_6H_3$ | 2 | 2,6-di-methyl | 4 | H | 0 | 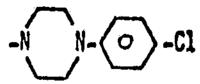 | Cis | Base | - | C: 61,74<br>H: 5,50<br>N: 8,73 | C: 61,0<br>H: 5,5<br>N: 8,6 |
| 1.101 | CH | 2,4-$Cl_2C_6H_3$ | 2 | 2,6-di-methyl | 4 | H | 0 | 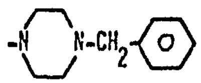 | Cis | Base | | | |
| 1.102 | CH | 2,4-$Cl_2C_6H_3$ | 2 | 2,6-di-methyl | 4 | H | 0 | 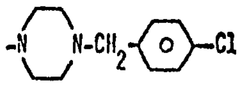 | Cis | Base | | C: 62,25<br>H: 5,68<br>N: 8,54 | C: 61,9<br>H: 5,6<br>N: 8,4 |
| 1.103 | CH | 2,4-$Cl_2C_6H_3$ | 2 | 2,6-di-methyl | 4 | H | 0 | 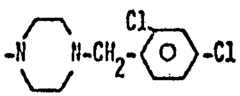 | Cis | Base | | | |
| 1.104 | CH | 2,4-$Cl_2C_6H_3$ | 2 | 2,6-di-methyl | 4 | H | 0 | 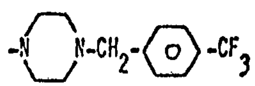 | Cis | Base | | | |
| 1.105 | CH | 2,4-$Cl_2C_6H_3$ | 2 | 2,6-di-methyl | 4 | H | 0 | 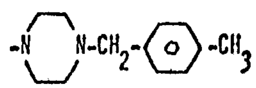 | Cis | Base | | | |
| 1.106 | CH | 2,4-$Cl_2C_6H_3$ | 2 | 2,6-di-methyl | 4 | H | 0 | 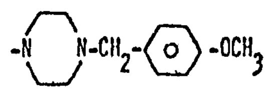 | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.107 | CH | $2,4-Cl_2C_6H_3$ | 2 | 2,6-dimethyl | 4 | H | 0 | 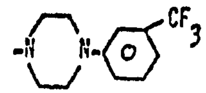 | Cis | Base | | | |
| 1.108 | CH | $2,4-Cl_2C_6H_3$ | 2 | 2,6-dimethyl | 4 | H | 0 | 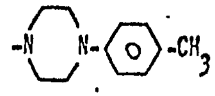 | Cis | Base | | | |
| 1.109 | CH | $2,4-Cl_2C_6H_3$ | 2 | 2,6-dimethyl | 4 | H | 0 | 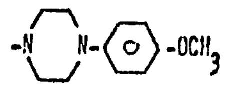 | Cis | Base | - | C: 64,05<br>H: 6,01<br>N: 8,79 | C: 63,6<br>H: 6,1<br>N: 8,4 |
| 1.110 | CH | $2,4-Cl_2C_6H_3$ | 2 | 2,6-dimethyl | 4 | H | 0 | 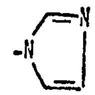 | Cis | Base | | | |
| 1.111 | CH | $2,4-Cl_2C_6H_3$ | 2 | 2,6-dimethyl | 4 | H | 0 | 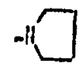 | Cis | Base | - | C: 62,79<br>H: 6,05<br>N: 8,14 | C: 62,2<br>H: 6,2<br>N: 7,8 |
| 1.112 | CH | $2,4-Cl_2C_6H_3$ | 2 | 2,6-dimethyl | 4 | H | 0 | 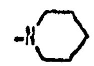 | Cis | Base | - | C: 63,39<br>H: 6,27<br>N: 7,92 | C: 63,1<br>H: 6,3<br>N: 7,6 |
| 1.113 | CH | $2,4-Cl_2C_6H_3$ | 2 | 2,6-dimethyl | 4 | H | 0 | 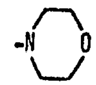 | Cis | Base | - | C: 60,90<br>H: 5,87<br>N: 7,89 | C: 60,2<br>H: 5,8<br>N: 7,5 |
| 1.114 | CH | $2,4-Cl_2C_6H_3$ | 2 | 2,6-dimethyl | 4 | H | 0 | 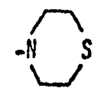 | Cis | Base | - | C: 59,12<br>H: 5,70<br>N: 5,11 | C: 58,6<br>H: 6,1<br>N: 4,7 |
| 1.115 | CH | $2,4-Cl_2C_6H_3$ | 2 | 2,6-dimethyl | 4 | H | 0 | 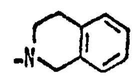 | Cis | Base | | | |
| 1.116 | CH | $2,4-Cl_2C_6H_3$ | 2 | 2,6-dimethyl | 4 | H | 0 | 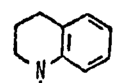 | Cis | Base | | | |
| 1.117 | CH | $2,4-Cl_2C_6H_3$ | 2 | 2,6-dimethyl | 4 | H | 0 | 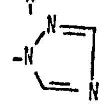 | Cis | Base | - | C: 58,37<br>H: 4,90<br>N: 13,61 | C: 57,5<br>H: 4,6<br>N: 12,9 |

0050298

| Verbdgs. Nr. | A | X | n | $R_n$ | a) y | $R_4$ | m | Z | b) 2,4-Isomere | Base oder Salze | Fp.: [°C] | Analyse Ber.: | Gef.: |
|---|---|---|---|---|---|---|---|---|---|---|---|---|---|
| 1.118 | N | $2,4\text{-}Cl_2C_6H_3$ | 2 | 2,6-dimethyl | 4 | H | 0 | $-N(C_2H_5)_2$ | Cis | Base | | | |
| 1.119 | N | $2,4\text{-}Cl_2C_6H_3$ | 2 | 2,6-dimethyl | 4 | H | 0 | 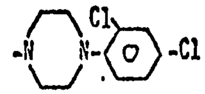 | Cis | Base | | | |
| 1.120 | N | $2,4\text{-}Cl_2C_6H_3$ | 2 | 2,6-dimethyl | 4 | H | 0 | 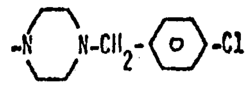 | Cis | Base | | | |
| 1.121 | N | $4\text{-}BrC_6H_4$ | 2 | 2,6-dimethyl | 4 | H | 0 | $-N(C_2H_5)_2$ | | Base | | | |
| 1.122 | CH | $4\text{-}CH_3\text{-}C_6H_4$ | 2 | 2,6-dimethyl | 4 | H | 0 | $-N(C_2H_5)_2$ | | Base | | | |
| 1.123 | N | $2,4\text{-}Cl_2C_6H_3$ | 2 | 2,6-dimethyl | 4 | H | 0 | 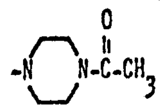 | Cis | Base | | | |
| 1.124 | CH | 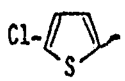 | 2 | 2,6-dimethyl | 4 | H | 0 | $-N(C_2H_5)_2$ | | Base | | | |
| 1.125 | CH | 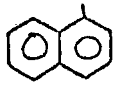 | 2 | 2,6-dimethyl | 4 | H | 0 | $-N(C_2H_5)_2$ | | Base | | | |
| 1.126 | CH | $2,4\text{-}Cl_2C_6H_3$ | 2 | 2-Cl; 6-$CH_3$ | 4 | H | 0 | $-N(C_2H_5)_2$ | Cis | Base | | | |
| 1.127 | CH | $2,4\text{-}Cl_2C_6H_3$ | 2 | 2-Cl; 6-$CH_3$ | 4 | H | 0 | $-N(C_4H_9)_2$ | Cis | Base | | | |
| 1.128 | CH | $2,4\text{-}Cl_2C_6H_3$ | 2 | 2-Cl; 6-$CH_3$ | 4 | H | 0 | $-N(-CH_2\text{-}CH\text{-}CH_2)_2$ | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.129 | CH | $2,4-Cl_2C_6H_3$ | 2 | 2-Cl; 6-$CH_3$ | 4 | H | 0 | $-N(C_3H_7)_2$ | Cis | Base | | | |
| 1.130 | CH | $2,4-Cl_2C_6H_3$ | 2 | 2-Cl; 6-$CH_3$ | 4 | H | 0 | $-N[-CH(CH_3)_2]_2$ | Cis | Base | | | |
| 1.131 | CH | $2,4-Cl_2C_6H_3$ | 2 | 2-Cl; 6-$CH_3$ | 4 | H | 0 | $-N(CH_3)-CH_2-CH=CH_2$ | Cis | Base | | | |
| 1.132 | CH | $2,4-Cl_2C_6H_3$ | 2 | 2-Cl; 6-$CH_3$ | 4 | H | 0 | 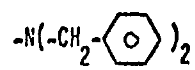 | Cis | Base | | | |
| 1.133 | CH | $2,4-Cl_2C_6H_3$ | 2 | 2-Cl; 6-$CH_3$ | 4 | H | 0 | 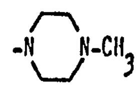 | Cis | Base | - | C: 57,30<br>H: 5,52<br>N: 9,90 | C: 57,3<br>H: 5,3<br>N: 9,2 |
| 1.134 | CH | $2,4-Cl_2C_6H_3$ | 2 | 2-Cl; 6-$CH_3$ | 4 | H | 0 | 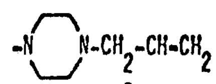 | Cis | Base | - | | |
| 1.135 | CH | $2,4-Cl_2C_6H_3$ | 2 | 2-Cl; 6-$CH_3$ | 4 | H | 0 | 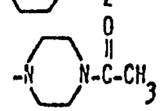 | Cis | Base | - | C: 56,63<br>H: 5,26<br>N: 9,43 | C: 55,9<br>H: 5,1<br>N: 9,0 |
| 1.136 | CH | $2,4-Cl_2C_6H_3$ | 2 | 2-Cl; 6-$CH_3$ | 4 | H | 0 | 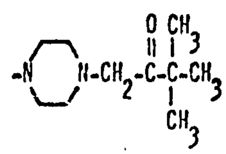 | Cis | Base | | | |
| 1.137 | CH | $2,4-Cl_2C_6H_3$ | 2 | 2-Cl; 6-$CH_3$ | 4 | H | 0 | 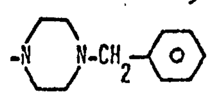 | Cis | Base | | | |
| 1.138 | CH | $2,4-Cl_2C_6H_3$ | 2 | 2-Cl; 6-$CH_3$ | 4 | H | 0 | 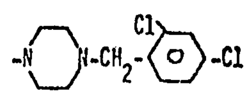 | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.139 | CH | $2,4-Cl_2C_6H_3$ | 2 | 2-Cl; $6-CH_3$ | 4 | H | 0 | 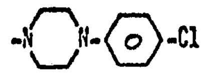 | Cis | Base | | | |
| 1.140 | CH | $2,4-Cl_2C_6H_3$ | 2 | 2-Cl; $6-CH_3$ | 4 | H | 0 | 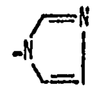 | Cis | Base | - | C: 56,25 H: 4,34 N: 10,50 | C: 55,9 H: 4,1 N: 10,1 |
| 1.141 | CH | $2,4-Cl_2C_6H_3$ | 2 | 2-Cl; $6-CH_3$ | 4 | H | 0 | 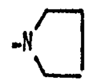 | Cis | Base | | | |
| 1.142 | CH | $2,4-Cl_2C_6H_3$ | 2 | 2-Cl; $6-CH_3$ | 4 | H | 0 | 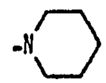 | Cis | Base | | | |
| 1.143 | CH | $2,4-Cl_2C_6H_3$ | 2 | 2-Cl; $6-CH_3$ | 4 | H | 0 | 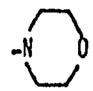 | Cis | Base | - | C: 56,48 H: 5,1 N: 7,6 | C: 57,1 H: 5,2 N: 7,7 |
| 1.144 | CH | $2,4-Cl_2C_6H_3$ | 2 | 2-Cl; $6-CH_3$ | 4 | H | 0 | 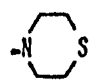 | Cis | Base | - | C: 54,89 H: 4,96 N: 7,38 | C: 54,5 H: 5,2 N: 7,5 |
| 1.145 | CH | $2,4-Cl_2C_6H_3$ | 2 | 2-Cl; $6-CH_3$ | 4 | H | 0 | 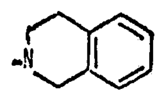 | Cis | Base | - | C: 62,17 H: 5,05 N: 7,01 | C: 61,8 H: 5,0 N: 6,9 |
| 1.146 | N | $2,4-Cl_2C_6H_3$ | 2 | 2-Cl; $6-CH_3$ | 4 | H | 0 | $-N(CH_3)_2$ | Cis | Base | - | C: 57,87 H: 5,49 N: 11,74 | C: 57,7 H: 5,6 N: 11,9 |
| 1.147 | CH | $4-Cl-C_6H_4$ | 2 | 2-Cl; $6-CH_3$ | 4 | H | 0 | $-N(CH_3)_2$ | | Base | - | C: 60,56 H: 5,71 N: 8,82 | C: 60,6 H: 5,7 N: 8,9 |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.148 | CH | $2,4-Cl_2C_6H_3$ | 2 | 2,6-dichlor | 4 | H | 0 | $-N(CH_3)_2$ | Cis | Base | - | C: 53,06<br>H: 4,27<br>N: 7,73 | C: 52,5<br>H: 4,6<br>N: 7,5 |
| 1.149 | CH | $2,4-Cl_2C_6H_3$ | 2 | 2,6-dichlor | 4 | H | 0 | $-N(C_2H_5)_2$ | Cis | Base | - | | |
| 1.150 | CH | $2,4-Cl_2C_6H_3$ | 2 | 2,6-dichlor | 4 | H | 0 | $-N(-CH_2-CH-CH_2)_2$ | Cis | Base | - | | |
| 1.151 | CH | $2,4-Cl_2C_6H_3$ | 2 | 2,6-dichlor | 4 | H | 0 | $-\overset{CH_3}{N}-C_4H_9$ | Cis | Base | - | | |
| 1.152 | CH | $2,4-Cl_2C_6H_3$ | 2 | 2,6-dichlor | 4 | H | 0 | 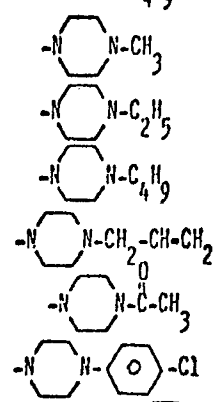 | Cis | Base | | | |
| 1.153 | CH | $2,4-Cl_2C_6H_3$ | 2 | 2,6-dichlor | 4 | H | 0 | | Cis | Base | | | |
| 1.154 | CH | $2,4-Cl_2C_6H_3$ | 2 | 2,6-dichlor | 4 | H | 0 | | Cis | Base | | | |
| 1.155 | CH | $2,4-Cl_2C_6H_3$ | 2 | 2,6-dichlor | 4 | H | 0 | | Cis | Base | | | |
| 1.156 | CH | $2,4-Cl_2C_6H_3$ | 2 | 2,6-dichlor | 4 | H | 0 | | Cis | Base | - | C: 52,78<br>H: 4,59<br>N: 9,12 | C: 52,7<br>H: 4,6<br>N: 9,0 |
| 1.157 | CH | $2,4-Cl_2C_6H_3$ | 2 | 2,6-dichlor | 4 | H | 0 | | Cis | Base | | | |
| 1.158 | CH | $2,4-Cl_2C_6H_3$ | 2 | 2,6-dichlor | 4 | H | 0 | 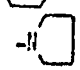 | Cis | Base | | | |
| 1.159 | CH | $2,4-Cl_2C_6H_3$ | 2 | 2,6-dichlor | 4 | H | 0 | 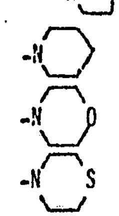 | Cis | Base | | | |
| 1.160 | CH | $2,4-Cl_2C_6H_3$ | 2 | 2,6-dichlor | 4 | H | 0 | | Cis | Base | | | |
| 1.161 | CH | $2,4-Cl_2C_6H_3$ | 2 | 2,6-dichlor | 4 | H | 0 | | Cis | Base | | | |
| 1.162 | CH | $2,4-Cl_2C_6H_3$ | 2 | 2,6-dichlor | 4 | H | 0 | 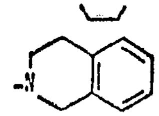 | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.163 | CH | $2,4-Cl_2C_6H_3$ | 2 | 2,6-dichlor | 4 | H | 0 | 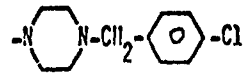 | Cis | Base | - | | |
| 1.164 | CH | $4-Br-C_6H_5$ | 2 | 2,6-dichlor | 4 | H | 0 | $-N(C_2H_5)_2$ | | Base | | | |
| 1.165 | CH | $4-Cl-C_6H_5$ | 2 | 2,6-dichlor | 4 | H | 0 | 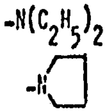 | | Base | | | |
| 1.166 | CH | $2,4-Cl_2C_6H_3$ | 0 | | | H | 0 | $-N(CH_3)_2$ | Cis | Base | - | C: 59,75<br>H: 5,45<br>N: 9,10 | C: 59,3<br>H: 5,1<br>N: 9,3 |
| 1.167 | CH | $2,4-Cl_2C_6H_3$ | 0 | | 4 | H | 0 | $-N(C_2H_5)_2$ | Cis | Base | - | C: 61,23<br>H: 5,96<br>N: 8,57 | C: 59,6<br>H: 5,5<br>N: 8,7 |
| 1.168 | CH | $2,4-Cl_2C_6H_3$ | 0 | | 4 | H | 0 | $-N(C_4H_9)_2$ | Cis | Base | | | |
| 1.169 | CH | $2,4-Cl_2C_6H_3$ | 0 | | 4 | H | 0 | $-N(-CH_2-CH-CH_2)_2$ | Cis | Base | - | C: 63,04<br>H: 5,68<br>N: 8,17 | C: 63,1<br>H: 5,6<br>N: 7,8 |
| 1.170 | CH | $2,4-Cl_2C_6H_3$ | 0 | | 4 | H | 0 | $-NH-C_4H_9$ | Cis | Base | | | |
| 1.171 | CH | $2,4-Cl_2C_6H_3$ | 0 | | 4 | H | 0 | $-NH-C_8H_{17}$ | Cis | Base | 70-71 | C: 63,73<br>H: 6,82<br>N: 7,69 | C: 63,5<br>H: 6,7<br>N: 7,8 |
| 1.172 | CH | $2,4-Cl_2C_6H_3$ | 0 | | 4 | H | | $-NH-C(=O)-CH_3$ | Cis | Base | 157-158 | C: 57,99<br>H: 4,87<br>N: 8,82 | C: 58,0<br>H: 4,9<br>N: 8,8 |
| 1.173 | CH | $2,4-Cl_2C_6H_3$ | 0 | | 4 | H | 0 | 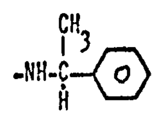 | Cis | Base | | | |
| 1.174 | CH | $2,4-Cl_2C_6H_3$ | 0 | | 4 | $H_3C$ | 0 | $-N(CH_3)_2$ | Cis | Base | - | C: 60,51<br>H: 5,71<br>N: 8,82 | C: 60,3<br>H: 5,6<br>N: 8,9 |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.175 | CH | $2,4-Cl_2C_6H_3$ | 0 | | 4 | H | 0 | 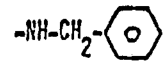 | Cis | Base | | | |
| 1.176 | CH | $2,4-Cl_2C_6H_3$ | 0 | | 4 | H | 1 | $-NH-C_4H_9$ | Cis | Base | | | |
| 1.177 | CH | $2,4-Cl_2C_6H_3$ | 0 | | 4 | H | 1 | 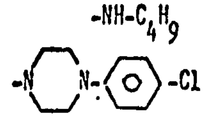 | Cis | Base | | | |
| 1.178 | CH | $2,4-Cl_2C_6H_3$ | 0 | | 4 | H | 1 | 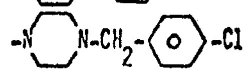 | Cis | Base | | | |
| 1.179 | CH | $2,4-Cl_2C_6H_3$ | 0 | | 4 | H | 1 | 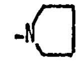 | Cis | Base | | | |
| 1.180 | CH | $2,4-Cl_2C_6H_3$ | 0 | | 4 | $C_3H_7$ | 0 | $-N(C_2H_5)_2$ | Cis | Base | | | |
| 1.181 | CH | $2,4-Cl_2C_6H_3$ | 0 | | 4 | H | 0 | 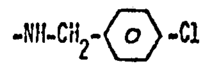 | Cis | Base | | | |
| 1.182 | CH | $2,4-Cl_2C_6H_3$ | 0 | | 4 | H | 0 | 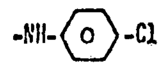 | Cis | Base | | C: 59,52<br>H: 4,44<br>N: 7,71 | C: 59,1<br>H: 4,2<br>N: 7,6 |
| 1.183 | CH | $2,4-Cl_2C_6H_3$ | 0 | | 4 | H | 0 | 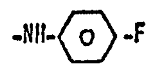 | Cis | Base | | | |
| 1.184 | CH | $2,4-Cl_2C_6H_3$ | 0 | | 4 | H | 0 | 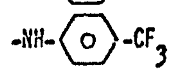 | Cis | Base | | | |
| 1.185 | CH | $2,4-Cl_2C_6H_3$ | 0 | | 4 | H | 0 | 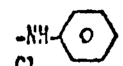 | Cis | Base | | | |
| 1.186 | CH | $2,4-Cl_2C_6H_3$ | 0 | | 2 | H | | 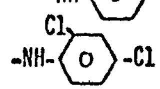 | Cis | Base | | | |
| 1.187 | CH | $2,4-Cl_2C_6H_3$ | 0 | | 4 | H | | 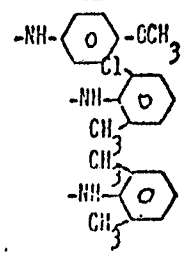 | Cis | Base | | | |
| 1.188 | CH | $2,4-Cl_2C_6H_3$ | 0 | | 4 | H | | | Cis | Base | | | |
| 1.189 | CH | $2,4-Cl_2C_6H_3$ | 0 | | 2 | H | | | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.190 | CH | 2,4-$Cl_2C_6H_3$ | 0 | | | H | 0 | 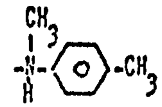 | Cis | Base | | | |
| 1.191 | CH | 2,4-$Cl_2C_6H_3$ | 0 | | | H | 0 | 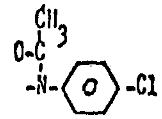 | Cis | Base | | | |
| 1.192 | CH | 2,4-$Cl_2C_6H_3$ | 0 | | 4 | H | 0 | 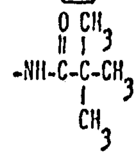 | Cis | $\cdot HNO_3$ | 158 | C: 53,71<br>H: 5,20<br>N: 9,64 | C: 53,5<br>H: 5,2<br>N: 9,3 |
| 1.193 | CH | 2,4-$Cl_2C_6H_3$ | 0 | | 4 | H | 0 | $-NH-\overset{O}{C}-O-CH_3$ | Cis | Base | | | |
| 1.194 | CH | 2,4-$Cl_2C_6H_3$ | 0 | | 4 | H | 0 | $-NH-\overset{O}{C}-O-C_2H_5$ | Cis | Base | | | |
| 1.195 | CH | 2,4-$Cl_2C_6H_3$ | 0 | | 4 | H | 1 | 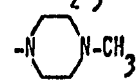 | Cis | Base | 115-116 | C: 61,02<br>H: 6,07<br>N: 10,54 | C: 60,9<br>H: 6,2<br>N: 10,4 |
| 1.196 | CH | 2,4-$Cl_2C_6H_3$ | 0 | | 4 | H | 2 | 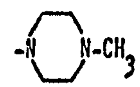 | Cis | Base | Öl | C: 61,65<br>H: 6,28<br>N: 10,27 | C: 60,8<br>H: 6,1<br>N: 10,2 |
| 1.197 | CH | 2,4-$Cl_2C_6H_3$ | 0 | | 4 | H | 2 | $-N(CH_3)_2$ | Cis | Base | Öl | C: 61,23<br>H: 5,96<br>N: 8,57 | C: 60,8<br>H: 5,9<br>N: 8,4 |
| 1.198 | CH | 2,4-$Cl_2C_6H_3$ | 0 | | 4 | H | 2 | $-N(C_2H_5)_2$ | Cis | Base | | | |
| 1.199 | CH | 2,4-$Cl_2C_6H_3$ | 0 | | 4 | H | 1 | $-N(C_2H_5)_2$ | Cis | Base | | | |
| 1.200 | CH | 2,4-$Cl_2C_6H_3$ | 0 | | 4 | H | 1 | $-N(C_3H_7)_2$ | Cis | Base | | | |

| Verbdgs. Nr. | A | X | n | $R_n$ | a) v | $R_1$ | m | Z | b) 2,4-Isomere | Base oder Salze | Fp.: [°C] | Analyse Ber.: | Gef.: |
|---|---|---|---|---|---|---|---|---|---|---|---|---|---|
| 1.201 | CH | $2,4-Cl_2C_6H_3$ | 0 | | 4 | H | 1 | 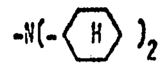 | Cis | Base | | | |
| 1.202 | CH | $2,4-Cl_2C_6H_3$ | 0 | | 4 | H | 0 | 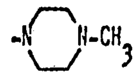 | Cis | Base | | | |
| 1.203 | N | $2,4-Cl_2C_6H_3$ | 0 | | 4 | H | 0 | 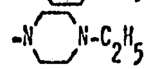 | Cis | Base | | | |
| 1.204 | CH | $2,4-Cl_2C_6H_3$ | 0 | | 4 | H | 0 | 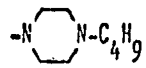 | Cis | Base | | | |
| 1.205 | CH | $2,4-Cl_2C_6H_3$ | 0 | | 4 | H | 0 | 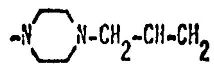 | Cis | Base | - | C: 61,88<br>H: 5,94<br>N: 10,31 | C: 61,6<br>H: 6,1<br>N: 10,0 |
| 1.206 | CH | | | | | | | | | | | | |
| 1.207 | CH | $2,4-Cl_2C_6H_3$ | 0 | | 4 | H | 0 | 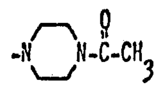 | Cis | Base | - | C: 59,45<br>H: 5,54<br>N: 10,27 | C: 59,1<br>H: 5,6<br>N: 10,4 |
| 1.208 | CH | $2,4-Cl_2C_6H_3$ | 0 | | 4 | H | 0 | 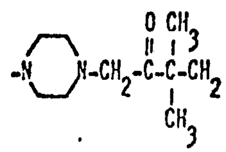 | Cis | Base | | | |
| 1.209 | N | $2,4-Cl_2C_6H_3$ | 0 | | 4 | H | 0 | 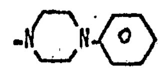 | Cis | Base | | | |
| 1.210 | CH | $2,4-Cl_2C_6H_3$ | 0 | | 4 | H | 0 | 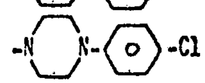 | Cis | Base | | | |
| 1.211 | CH | $2,4-Cl_2C_6H_3$ | 0 | | 4 | H | 0 | 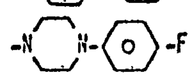 | Cis | Base | | | |
| 1.212 | CH | $2,4-Cl_2C_6H_3$ | 0 | | 4 | H | 0 | 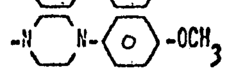 | Cis | Base | | | |
| 1.213 | CH | $2,4-Cl_2C_6H_3$ | 0 | | 4 | H | 0 | 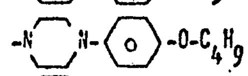 | Cis | Base | | | |
| 1.214 | CH | $2,4-Cl_2C_6H_3$ | 0 | | 4 | H | 0 | 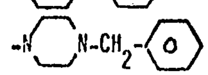 | Cis | Base | | | |
| 1.215 | CH | $2,4-Cl_2C_6H_3$ | 0 | | 4 | H | 0 | 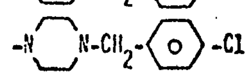 | Cis | Base | - | C: 61,20<br>H: 5,30<br>N: 8,92 | C: 60,9<br>H: 5,3<br>N: 8,7 |

| Verbdgs. Nr. | A | X | n | $R_n$ | a) v | $R_q$ | m | Z | b) 2,4-Isomere | Base oder Salze | Fp.: [°C] | Analyse Ber.: | Gef.: |
|---|---|---|---|---|---|---|---|---|---|---|---|---|---|
| 1.216 | CH | $2,4-Cl_2C_6H_3$ | 0 | | 4 | H | 0 | 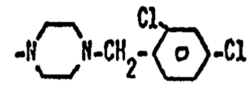 | Cis | Base | | | |
| 1.217 | CH | $2,4-Cl_2C_6H_3$ | 0 | | 4 | H | 0 | 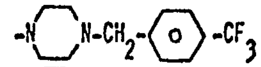 | Cis | Base | | | |
| 1.218 | CH | $2,4-Cl_2C_6H_3$ | 0 | | 4 | H | 0 | 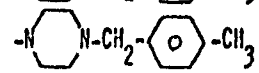 | Cis | Base | | | |
| 1.219 | CH | $2,4-Cl_2C_6H_3$ | 0 | | 4 | H | 0 | 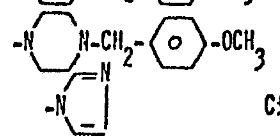 | Cis | Base | | | |
| 1.220 | CH | $2,4-Cl_2C_6H_3$ | 0 | | 4 | H | 0 | | Cis | Base | - | C: 59,44<br>H: 4,57<br>N: 11,55 | C: 58,9<br>H: 4,7<br>N: 11,1 |
| 1.221 | CH | $2,4-Cl_2C_6H_3$ | 0 | | 4 | H | 0 | 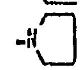 | Cis | Base | - | C: 61,48<br>H: 5,57<br>N: 8,60 | C: 61,1<br>H: 5,4<br>N: 8,4 |
| 1.222 | CH | $2,4-Cl_2C_6H_3$ | 0 | | 4 | H | 0 | 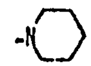 | Cis | Base | - | C: 62,15<br>H: 5,82<br>N: 8,36 | C: 61,7<br>H: 5,9<br>N: 8,0 |
| 1.223 | N | $2,4-Cl_2C_6H_3$ | O | | 4 | H | O | 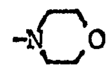 | Cis | Base | 107-108 | C: 59,53<br>H: 5,40<br>N: 8,33 | C: 59,4<br>H: 5,4<br>N: 8,2 |
| 1.224 | CH | $2,4-Cl_2C_6H_3$ | O | | 4 | H | O | 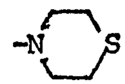 | Cis | Base | - | C: 57,69<br>H: 5,23<br>N: 8,07 | C: 57,5<br>H: 5,4<br>N: 7,7 |
| 1.225 | CH | $2,4-Cl_2C_6H_3$ | O | | 4 | H | O | 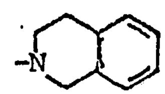 | Cis | Base | - | C: 65,46<br>H: 5,31<br>N: 7,63 | C: 65,1<br>H: 5,2<br>N: 7,2 |
| 1.226 | CH | $2,4-Cl_2C_6H_3$ | O | | 4 | H | O | $-N(CH_3)-CH_2-C_6H_5$ | Cis | Base | - | C: 64,69<br>H: 5,43<br>N: 7,80 | C: 64,8<br>H: 5,2<br>N: 7,8 |
| 1.227 | CH | $2,4-Cl_2C_6H_3$ | O | | 4 | H | O | $-N(CH_3)-C_6H_5$ | Cis | Base | - | C: 64,69<br>H: 5,43<br>N: 7,80 | C: 64,6<br>H: 5,6<br>N: 7,6 |

| Verbdgs. Nr. | A | X | n | $R_n$ | a) v | $R_1$ | m | Z | b) 2,4-Isomere | Base oder Salze | Fp.: /°C/ | Analyse Ber.: | Analyse Gef.: |
|---|---|---|---|---|---|---|---|---|---|---|---|---|---|
| 1.228 | CH | $2,4-Cl_2C_6H_3$ | 0 | - | 4 | H | O | 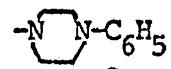 | Cis | Base | - | C: 64,25<br>H: 5,57<br>N: 9,67 | C: 63,5<br>H: 5,5<br>N: 9,5 |
| 1.229 | CH | $2,4-Cl_2C_6H_3$ | 0 | - | 4 | H | O | 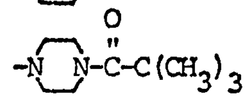 | Cis | Base | - | C: 61,33<br>H: 6,18<br>N: 9,54 | C: 61,0<br>H: 6,1<br>N: 9,2 |
| 1.230 | CH | $2,4-Cl_2C_6H_3$ | 0 | - | 4 | H | O | 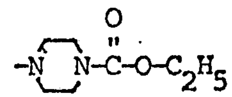 | Cis | Base | - | C: 58,44<br>H: 5,60<br>N: 9,74 | C: 57,7<br>H: 5,7<br>N: 9,3 |
| 1.231 | CH | $2,4-Cl_2C_6H_3$ | 2 | 2,6-dimethyl | 4 | H | O | $-NH-C_8H_{17}$ | Cis | | - | | |
| 1.232 | CH | $2,4-Cl_2C_6H_3$ | 2 | 2,6-dimethyl | 4 | H | O | 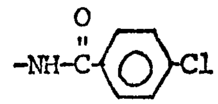 | Cis | Base | - | C: 59,96<br>H: 4,70<br>N: 6,70 | C: 60,3<br>H: 4,6<br>N: 6,5 |
| 1.233 | CH | $2,4-Cl_2C_6H_3$ | 2 | 2,6-dimethyl | 4 | H | O | 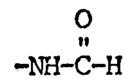 | Cis | Base | - | C: 58,78<br>H: 5,14<br>N: 8,57 | C: 58,5<br>H: 5,2<br>N: 8,3 |
| 1.234 | CH | $2,4-Cl_2C_6H_3$ | 2 | 2,6-dimethyl | 4 | H | O | 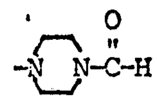 | Cis | Base | - | C: 60,11<br>H: 5,76<br>N: 10,01 | C: 59,9<br>H: 5,6<br>N: 9,7 |
| 1.235 | CH | $2,4-Cl_2C_6H_3$ | 2 | 2,6-dimethyl | 4 | H | O | 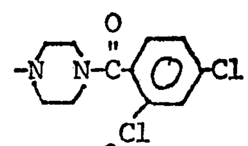 | Cis | Base | - | C. 57,24<br>H: 4,95<br>N: 8,09 | C: 57,2<br>H: 4,9<br>N: 7,8 |
| 1.236 | CH | $2,4-Cl_2C_6H_3$ | 2 | 2,6-dimethyl | 4 | H | O | 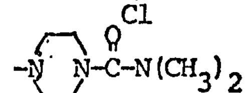 | Cis | Base | - | C: 59,80<br>H: 6,19<br>N: 11,62 | C: 60,0<br>H: 6,3<br>N: 11,4 |
| 1.237 | CH | $2,4-Cl_2C_6H_3$ | | 2,6-dimethyl | 4 | H | O | 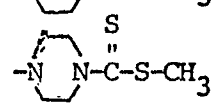 | Cis | Base | - | | |
| 1.238 | CH | $2,4-Cl_2C_6H_3$ | 0 | - | 4 | H | O | 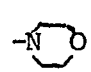 | Cis | Base | 110 | C: 59,53<br>H: 5,40<br>N: 8,33 | C: 59,4<br>H: 5,4<br>N: 8,2 |

| Verbds. Nr. | A | X | n | $R_n$ | a) v | $R_1$ | m | Z | b) 2,4-Isomere | Base oder Salze | Fp.: /¯°C_7 | Analyse Ber. | Analyse Gef. |
|---|---|---|---|---|---|---|---|---|---|---|---|---|---|
| 1.239 | CH | $2,4\text{-}Cl_2C_6H_3$ | 2 | 2,6-dimethyl | 4 | H | 0 | 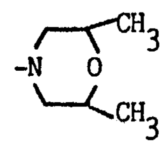 | Cis | Base | - | C: 62,14<br>H: 6,29<br>N: 7,50 | C: 61,6<br>H: 6,4<br>N: 7,3 |
| 1.240 | CH | $2,4\text{-}Cl_2C_6H_3$ | 2 | 2-chlor-6-methyl | 4 | H | 0 | 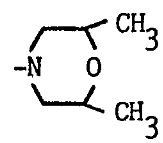 | Cis | Base | - | C: 57,89<br>H: 5,55<br>N: 7,23 | C: 58,0<br>H: 5,4<br>N: 7,0 |
| 1.241 | CH | $2,4\text{-}Cl_2C_6H_3$ | 2 | 2,6-dimethyl | 4 | H | 0 | 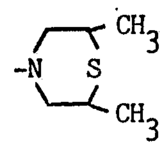 | Cis | Base | - | C: 60,41<br>H: 6,12<br>N: 7,29 | C: 60,1<br>H: 5,9<br>N: 6,9 |
| 1.242 | CH | $2,4\text{-}Cl_2C_6H_5$ | 2 | 2-chor-6-methyl | 4 | H | 0 | 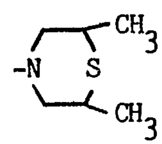 | Cis | Base | - | C: 56,33<br>H: 5,40<br>N: 7,04 | C: 56,1<br>H: 5,2<br>N: 6,7 |

Beispiel 3:

Cis-2-(2,4-Dichlorphenyl)-2-(1H-imidazol-1-ylmethyl)-4-/4-chlor-2-(4-acetylpiperazin-1-ylmethyl)-phenoxymethyl7-1,3-dioxolan

Ein Gemisch aus
4,07 g = 10 mmol Cis-2-(2,4-Dichlorphenyl-2-(1H-imidazol-1-ylmethyl)-1,3-dioxolan-4-ylmethylmethansulfonat
2,7 g = 10 mmol 4-Chlor-2-(4-acetylpiperazin-1-ylmethyl)-phenol und
2,8 g = 20 mmol Kaliumcarbonat in 30 ml Dimethylsulfoxid erhitzt man 16 Stunden auf 100°C, läßt abkühlen, gießt die Mischung in 150 ml Wasser, schüttelt 2mal mit 30 ml Methylenchlorid aus, wäscht die vereinigten Methylenchloridphasen mit 30 ml Wasser nach, trocknet über Natriumsulfat und zieht im Vakuum das Solvens ab. Der Rückstand (5,3 g) wird säulenchromatographisch an Kieselgel mit einer Mischung aus Methylenchlorid : Ethanol, 10 : 1 als Laufmittel gereinigt und anschließend im Ölpumpenvakuuum über $P_4O_{10}$ bei 50°C getrocknet. Man erhält 3,4 g (58 % d.Th.) als hochviskoses Öl.

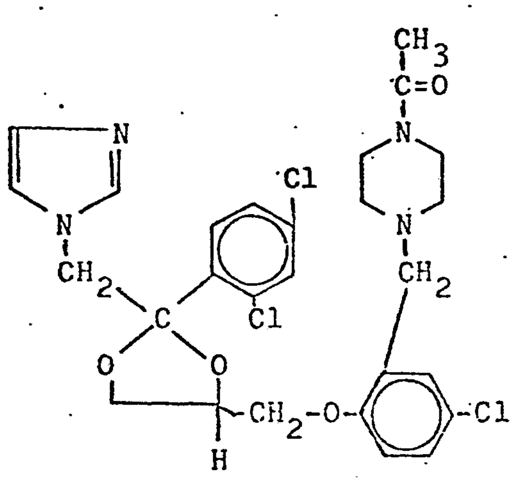

| | | | | |
|---|---|---|---|---|
| Analyse: | Ber.: | C: 55,92 | Gef.: | C: 55,2 |
| $C_{27}H_{29}Cl_3N_4O_4$ | | H: 5,04 | | H: 5,1 |
| | | N: 9,66 | | N: 9,1 |

MG: 579,88.

Bei gleicher Arbeitsweise wie vorstehend beschrieben können auch Dimethylformamid, Dimethylacetamid, Methyl-isobutylketon oder N-Methylpyrrolidon-2 als Lösungsmittel verwendet werden.

Beispiel 4

Cis-2-(2,4-Dichlorphenyl)-2-(1H-imidazol-1-ylmethyl)-4-/4-(N,N-diethylaminomethyl)-phenoxymethyl7-1,3-dioxolan

Zu einer frisch hergestellten Alkoholatlösung aus 0,23 g = 10 mmol Natrium in 20 ml abs. Ethanol tropft man unter Rühren bei 20°C die Lösung von 1,8 g = 10 mmol 4-(N,N-Diethylaminomethyl)-phenol in 10 ml abs. Ethanol, rührt 10 min. nach, gibt 4,07 g = 10 mmol Cis-2-(2,4-Dichlorphenyl)-2-(1H-imidazol-1-ylmethyl)-1,3-dioxolan-4-ylmethylmethansulfonat portionsweise zu und kocht anschließend 8 Stunden am Rückfluß. Nach dem Abkühlen wird das Lösungsmittel im Vakuum am Rotationsverdampfer abgezogen, der Rückstand in 50 ml Methylenchlorid und 20 ml 40 %iger Natronlauge aufgenommen, die Methylenchloridphase zweimal mit Wasser gewaschen, über Natriumsulfat getrocknet und im Vakuum abgezogen. Der Rückstand (4,2 g) wird säulenchromatographisch an Kieselgel mit einer Mischung aus Methylenchlorid : Ethanol, 20 : 1 als Laufmittel gereinigt und anschließend wie in Beispiel 3 getrocknet. Man erhält 3,1 g (63 % d.Th.) als hochviskoses Öl.

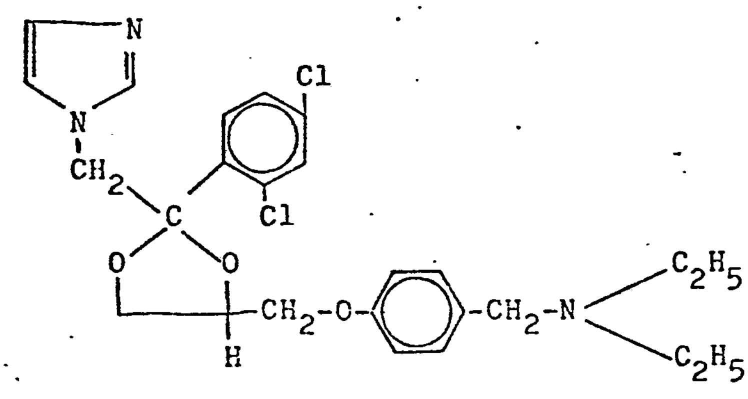

| | | | | | |
|---|---|---|---|---|---|
| Analyse: | Ber.: | C: 61,23 | Gef.: | C: 59,6 |
| $C_{25}H_{29}Cl_2N_3O_3$ | | H: 5,96 | | H: 5,5 |
| | | N: 8,57 | | N: 8,2 |

MG: 490,43.

Beispiel 5

Cis-2-(2,4-Dichlorphenyl)-2-(1H-imidazol-1-ylmethyl)-4-/4-(N,N-dimethylaminoethyl)-phenoxymethyl7-1,3-dioxolan

Zu einer Alkoholatlösung aus 0,46 g = 20 mmol Natrium in 30 ml abs. Ethanol gibt man unter Rühren bei 20°C nacheinander 2,46 g = 10 mmol 4-(N,N-Dimethylaminoethyl)-phenol-Hydrobromid und 4,07 g = 10 mmol Cis-2-(2,4-Dichlorphenyl)-2-(1H-imidazol-1-ylmethyl)-1,3-dioxolan-4-ylmethylmethansulfonat portionsweise zu und kocht anschließend 8 Stunden am Rückfluß. Die Aufarbeitung erfolgt wie in Beispiel 4 beschrieben. Man erhält 2,6 g (55 % d.Th.) als hochviskoses Öl.

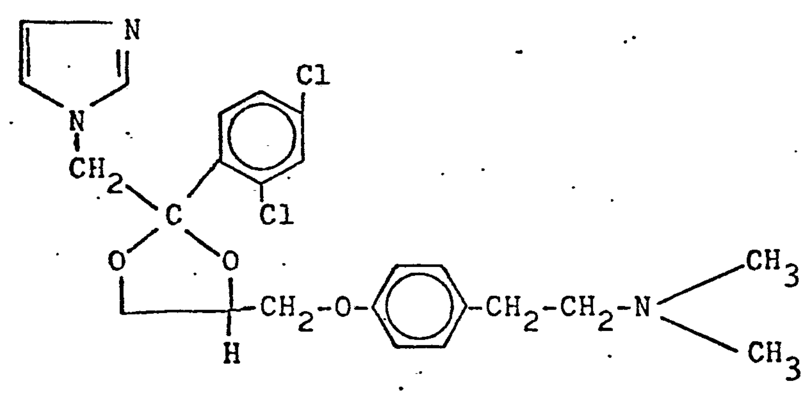

| | | | | |
|---|---|---|---|---|
| Analyse: | Ber.: | C: 60,51 | Gef.: | C: 60,1 |
| $C_{24}H_{27}Cl_2N_3O_3$ | | H: 5,71 | | H: 5,7 |
| | | N: 8,82 | | N: 8,4 |

MG: 476,41.

Beispiel 6

Cis-2-(2,4-Dichlorphenyl)-2-(1H-imidazol-1-ylmethyl)-4-/2,6-dimethyl-4-(4-phenylpiperazin-1-ylmethyl)-phenoxymethyl]-1,3-dioxolan und Dinitrat

a) Herstellung der freien Verbindung

Zu 10 ml 50 %iger Natronlauge
40 ml Toluol und
0,3 g = 0,9 mmol Tetrabutylammoniumbromid gibt man unter Rühren 3 g = 10 mmol 2,6-Dimethyl-4-(4-phenylpiperazin-1-ylmethyl)-phenol und anschließend 4,07 g = 10 mmol Cis-2-(2,4-Dichlorphenyl)-2-(1H-imidazol-1-ylmethyl)-1,3-dioxolan-4-yl-methylmethansulfonat portionsweise zu und kocht anschließend bei intensiver Rührung 12 Stunden am Rückfluß, versetzt die abgekühlte Mischung mit 30 ml Wasser, trennt die wäßrige Phase ab, wäscht die organische Phase mit Wasser nach, trocknet über $Na_2SO_4$ und zieht im Vakuum am Rotationsverdampfer das Lösungsmittel ab. Das Rohprodukt (6,1 g) wird säulenchromatographisch an Kieselgel mit einer Mischung aus Methylenchlorid : Ethanol, 10 : 1 als Laufmittel gereinigt, oder wird direkt, wie anschließend beschrieben, zur Salzbildung verwendet. Man erhält 5,1 g (85 % d.Th.) als hochviskoses, nicht kristallines Öl.

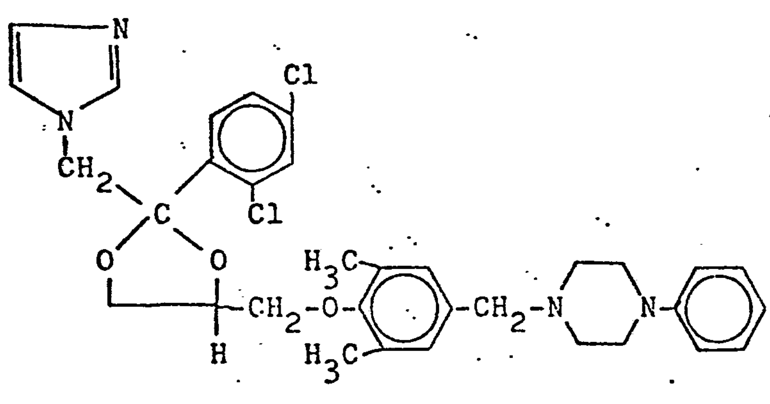

| | | | | |
|---|---|---|---|---|
| Analyse: | Ber.: | C: 65,23 | Gef.: | C: 64,9 |
| $C_{33}H_{36}Cl_2N_4O_3$ | | H: 6,0 | | H: 6,2 |
| | | N: 9,22 | | N: 9,1 |

MG: 607,58.

b) Fällung des Dinitrates:

Zur Lösung von 2 g = 3,3 mmol des vorstehend beschriebenen Öls in 60 ml Essigester tropft man unter Rühren bei 20°C die Lösung von 0,28 ml = 0,41 g = 6,6 mmol 100 %ige $HNO_3$ in 20 ml Essigester, läßt 15 Stunden bei Raumtemperatur stehen, saugt ab und kristallisiert den Rückstand aus Acetonitril um. Man erhält 1,3 g (53 % d.Th.) vom Fp.: 145°C (Zersetzung), Cis-2-(2,4-dichlorphenyl)-2-(1H-imidazol-1-ylmethyl)-4-/2,6-dimethyl-4-(4-phenylpiperazin-1-ylmethyl)-phenoxymethyl7-1,3-dioxolan, als Dinitrat.

| | | | | |
|---|---|---|---|---|
| Analyse: | Ber.: | C: 54,03 | Gef.: | C: 53,8 |
| $C_{33}H_{38}Cl_2N_6O_9$ | | H: 5,22 | | H: 5,3 |
| | | N: 19,63 | | N: 19,4 |

MG: 733,59.

Beispiel 7

Cis-2-(2,4-Dichlorphenyl)-2-(1H-imidazol-1-ylmethyl)-4-/2-chlor-4-(N,N-dimethylaminomethyl)-6-methylphenoxymethyl7-1,3-dioxolan

Zu 10 ml 50 %iger Natronlauge, 40 ml Benzol und 0,3 g = 1,3 mmol Benzyltriethylammoniumchlorid gibt man unter Rührung 2 g = 10 mmol 2-Chlor-4-(N,N-dimethylaminomethyl)-6-methylphenol und anschließend 4,07 g = 10 mmol Cis-2-(2,4-Dichlorphenyl)-2-(1H-imidazol-1-ylmethyl)-1,3-dioxolan-4-ylmethylmethansulfonat portionsweise zu und kocht anschließend bei intensiver Rührung 8 Stunden am Rückfluß. Die Aufarbeitung erfolgt wie in Beispiel 6a beschrieben. Man erhält 3,8 g (75 % d.Th.) als hochviskoses, nicht kristallines Öl.

| | | | | |
|---|---|---|---|---|
| Analyse: | Ber.: | C: 56,42 | Gef.: | C: 55,8 |
| $C_{24}H_{26}Cl_3N_3O_3$ | | H: 5,13 | | H: 5,2 |
| | | N: 8,22 | | N: 8,0 |

MG: 510,85.

Beispiel 8

Cis-2-(2,4-Dichlorphenyl)-2-(1H-imidazol-1-ylmethyl)-4-/2-chlor-6-methyl-4-(piperazin-1-ylmethyl)-phenoxymethyl7-1,3-dioxolan

Die Lösung von 29,7 g = 50 mmol der Verbindung 1.135 (vgl. Tab. 1) und 8 g = 0,2 mol Natriumhydroxid in einem Gemisch aus 100 ml Ethanol und 10 ml Wasser kocht man 20 Stunden am Rückfluß, zieht das Lösungsmittel im Vakuum am Rotationsverdampfer ab, nimmt den Rückstand in Wasser auf, extrahiert mit Äther, trocknet die organische Phase über Natriumsulfat und arbeitet auf, wie im Beispiel 1a beschrieben. Man erhält 15,2 g (55 % d. Th.) als hochviskoses, nicht kristallines Öl.

| | | | |
|---|---|---|---|
| Analyse: | Ber.: C: 56,57 | Gef.: | C: 56,3 |
| $C_{26}H_{29}Cl_3N_4O_3$ | H: 5,30 | | H: 5,2 |
| | N: 10,15 | | N: 9,8 |

MG: 551,9.

Beispiel 9

gemäß Beispiel 8, jedoch unter Einsatz äquivalenter Mengen von Verbindungen der Formel (Ia-3), werden die folgenden Verbindungen der Formel (Ib) hergestellt.

Tabelle 2:

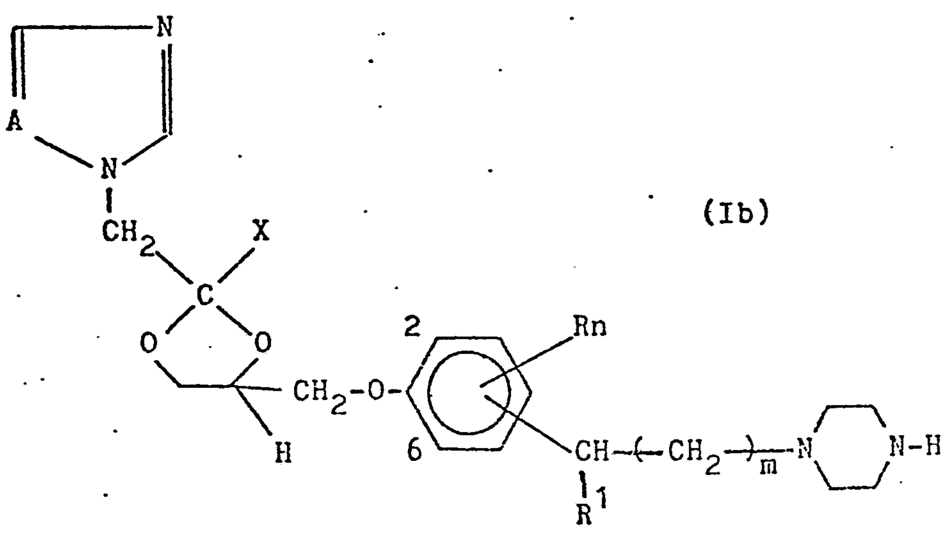

(Ib)

Für 2,4-Isomere und v gelten die Erklärungen der Tabelle 1.

| Verb.. dgs. Nr. | A | X | n | Rn | v | $R_1$ | m | 2,4-Isomere | Fp.: /°C/ | Analyse Ber.: | Gef. |
|---|---|---|---|---|---|---|---|---|---|---|---|
| 2.1 | CH | 2,4-$Cl_2C_6H_3$ | 2 | 2,6-di methoxy | 4 | H | 0 | Cis | | | |
| 2.2 | CH | 2,4-$Cl_2C_6H_3$ | 2 | 2,6-di-methyl | 4 | H | 0 | Cis | | C: 60,90<br>H: 6,25<br>N: 10,52 | C: 60,7<br>H: 6,2<br>N: 10,5 |
| 2.3 | N | 2,4-$Cl_2C_6H_3$ | 2 | 2,6-di-methyl | 4 | H | 0 | Cis | | | |
| 2.4 | N | 2,4-$Cl_2C_6H_3$ | 2 | 2-chlor 6-methyl | 4 | H | 0 | Cis | | | |
| 2.5 | CH | 2,4-$Cl_2C_6H_3$ | 2 | 2,6-di-chlor | 4 | H | 0 | Cis | | C: 52,46<br>H: 4,58<br>N: 9,79 | C: 52,3<br>H: 4,4<br>N: 9,8 |
| 2.6 | CH | 2,4-$Cl_2C_6H_3$ | 0 | - | 4 | H | 0 | Cis | | C: 59,65<br>H: 5,61<br>N: 11,13 | C: 59,2<br>H: 5,7<br>N: 10,8 |

Beispiel 10

Cis-2-(2,4-Dichlorphenyl)-2-(imidazol-1-ylmethyl)-4-/2-chlor-4-(4-ethylthiocarbamoylpiperazin-1-ylmethyl)-6-methylphenoxymethyl/-1,3-dioxolan

Zur Lösung von 5,5 g = 10 mmol der Verbindung aus Beispiel 8, in 20 ml Toluol und 0,1 ml Triäthylamin, tropft man unter Rühren bei 20°C die Lösung von 0,9 g = 10 mmol Ethylisothiocyanat in 10 ml Toluol und kocht anschließend 5 Stunden am Rückfluß. Die abgekühlte Lösung wird mit Wasser gewaschen, mit Natriumsulfat getrocknet und aufgearbeitet, wie in Beispiel 1a beschrieben.

Man erhält 5,2 g (81 % d.Th.) als hochviskoses, nicht kristallines Öl.

| Analyse: | Ber.: | Gef.: |
|---|---|---|
| $C_{29}H_{34}Cl_3N_5O_3S$ | C: 54,51<br>H: 5,36<br>N: 10,96 | C: 54,0<br>H: 5,3<br>N: 10,8 |

MG: 639,01

## Beispiel 11

Gemäß Beispiel 10, jedoch unter Einsatz äquivalenter Mengen von Verbindungen der Formel (Ib) und (IX), werden die folgenden Verbindungen (Ic) hergestellt:

Tabelle 3

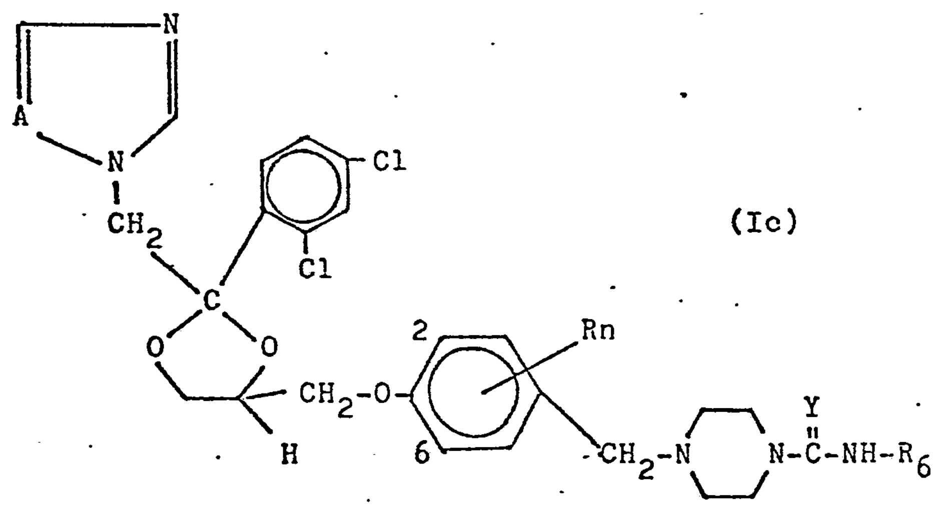

(Ic)

a) Für 2,4-Isomere gilt die Erklärung der Tabelle 1.

| Verb. dgs. Nr. | n | $R_n$ | Y | $R_6$ | 2,4-Isomere | Fp.: [°C] | Analyse Ber.: | Gef. |
|---|---|---|---|---|---|---|---|---|
| 3.1 | 2 | 2-Cl<br>6-$CH_3$ | S | $-CH_2-CH=CH_2$ | Cis | - | C: 59,65<br>H: 5,61<br>N: 11,13 | C: 59,2<br>H: 5,4<br>N: 10,9 |
| 3.2 | 2 | 2,6-dimethyl | S | $-CH_2-CH=CH_2$ | Cis | | | |
| 3.3 | 2 | 2,6-dimethyl | S | tert.-butyl | Cis | | | |
| 3.4 | 2 | 2-Cl<br>6-$CH_3$ | O | $-CH_3$ | Cis | - | C: 55,32<br>H: 5,14<br>N: 11,52 | C: 54,9<br>H: 5,3<br>N: 11,2 |

| Verb. dgs. Nr. | n | $R_n$ | Y | $R_6$ | 2,4-Isomere | Fp.: /°C/ | Analyse Ber.: | Gef.: |
|---|---|---|---|---|---|---|---|---|
| 3.5 | 0 | - | O | $-C_4H_9$ | Cis | | | |
| 3.6 | 2 | 2,6-dichlor | S | $4-ClC_6H_4$ | Cis | | | |
| 3.7 | 2 | 2-Cl 6-$CH_3$ | O | $-C_6H_5$ | Cis | | | |
| 3.8 | 0 | - | O | $3-CF_3C_6H_4$ | Cis | | | |
| 3.9 | 2 | 2-Cl 6-$CH_3$ | O | $2,6-Cl_2C_6H_3$ | Cis | | | |
| 3.10 | 2 | 2,6-dimethyl | O | $4-CH_3C_6H_4$ | Cis | | | |

Beispiel 12

Cis-2-(2,4-Dichlorphenyl)-2-(1H-imidazol-1-ylmethyl)-4-/4-(4-carbamoylpiperazin-1-ylmethyl)-2-chlor-6-methylphenoxymethyl/-1,3-dioxolan

Ein Gemisch aus 5,5 g = 10 mmol der Verbindung aus Beispiel 8, 1,2 g = 15 mmol Kaliumcyanat und 20 ml Eisessig rührt man 24 Stunden bei 20°C, zieht im Vakuum am Rotationsverdampfer das Lösungsmittel ab, nimmt in Wasser auf, extrahiert mit Methylenchlorid, wäscht die organische Phase mit 2 n Sodalösung nach und arbeitet auf, wie in Beispiel 1a beschrieben. Man erhält 1,8 g (30 % d.Th.) als hochviskoses, nicht kristallines Öl.

| | | | | |
|---|---|---|---|---|
| Analyse: | Ber.: | C: 54,51 | Gef.: | C: 54,1 |
| $C_{27}H_{30}Cl_3N_5O_4$ | | H: 5,08 | | H: 5,1 |
| | | N: 11,77 | | N: 11,5 |

MG: 594,93

Beispiel 13

Cis-2-(2,4-Dichlorphenyl)-2-(1H-imidazol-1-ylmethyl)-4-/2-chlor-4-(4-methoxycarbonylpiperazin-1-ylmethyl)-6-methylphenoxymethyl/-1,3-dioxolan und Dinitrat

a) Herstellung der freien Verbindung

Zur Lösung von 5,5 g = 10 mmol der Verbindung aus Beispiel 8, in 20 ml Methylenchlorid und 10,1 g = 10 mmol Triethylamin, tropft man unter Rühren und Kühlen bei -10°C die Lösung von 0,94 g = 10 mmol Chlorameisensäuremethylester in 10 ml Methylenchlorid, läßt auf Raumtemperatur aufwärmen und kocht anschließend 3 Stunden am Rückfluß. Die abgekühlte Lösung wird mit Wasser gewaschen, mit Natriumsulfat getrocknet und aufgearbeitet, wie im Beispiel 1a beschrieben. Man erhält 5,1 g (85 % d.Th.) als hochviskoses, nicht kristallines Öl.

| | | | | |
|---|---|---|---|---|
| Analyse: | Ber.: | C: 55,14 | Gef.: | C: 54,8 |
| $C_{28}H_{31}Cl_3N_4O_5$ | | H: 5,12 | | H: 5,2 |
| | | N: 9,18 | | N: 8,9 |
| MG: 609,90 | | | | |

b) Herstellung des Dinitrates

gemäß Beispiel 1b, jedoch unter Einsatz äquivalenter Mengen der Verbindung aus Beispiel 13 a und Salpetersäure erhält man 1,5 g (55 % d.Th.) vom Fp.: 130°C, Cis-2-(2,4-Dichlorphenyl)-2-(1H-imidazol-1-ylmethyl)-4-[2-chlor-4-(4-methoxycarbonylpiperazin-1-ylmethyl)-6-methylphenoxymethyl]-1,3-dioxolan als Dinitrat.

| | | | | |
|---|---|---|---|---|
| Analyse: | Ber.: | C: 45,70 | Gef.: | C: 45,4 |
| $C_{28}H_{33}Cl_3N_6O_{11}$ | | H: 4,52 | | H: 4,8 |
| | | N: 11,42 | | N: 11,2 |
| MG: 735,93 | | | | |

Beispiel 14

Gemäß Beispiel 13 a, jedoch unter Einsatz äquivalenter Mengen von Verbindungen der Formel (Ib) und einem Acylchlorid, das sich von der entsprechenden Carbon- oder Sulfonsäure ableitet, werden die folgenden Verbindungen (Id) hergestellt:

Tabelle 4:

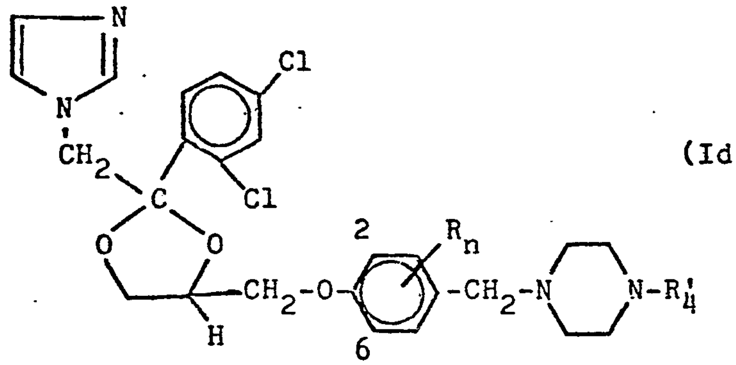

(Id)

Für 2,4-Isomere gilt die Erklärung der Tabelle 1

| Verbdgs. Nr. | n | Rn | R4 | 2,4-Isomere | Analyse Ber. | Analyse Gef. |
|---|---|---|---|---|---|---|
| 4.1 | 2 | 2-Cl ; 6-$CH_3$ | $-\overset{\overset{O}{\|}}{C}-$tert.-butyl | Cis | | |
| 4.2 | 2 | 2,6-dimethyl | $-\overset{\overset{O}{\|}}{C}-$tert.-butyl | Cis | C: 62,44<br>H: 6,55<br>N: 9,10 | C: 62,0<br>H: 6,3<br>N: 8,9 |
| 4.3 | 2 | 2,6-dimethyl | $-\overset{\overset{O}{\|}}{C}-CH_2Cl$ | Cis | C: 57,29<br>H: 5,47<br>N: 9,22 | C: 57,1<br>H: 5,3<br>N: 9,0 |
| 4.4 | 2 | 2,6-dichlor | $-\overset{\overset{O}{\|}}{C}-CF_3$ | Cis | | |
| 4.5 | 2 | 2,6-dichlor | $-SO_2-C_2H_5$ | Cis | | |
| 4.6 | 2 | 2-Cl ; 6-$CH_3$ | $-\overset{\overset{O}{\|}}{C}-N(CH_3)_2$ | Cis | | |
| 4.7 | 2 | 2-Cl ; 6-$CH_3$ | $-\overset{\overset{O}{\|}}{C}-N(C_4H_9)_2$ | Cis | | |
| 4.8 | 0 | - | 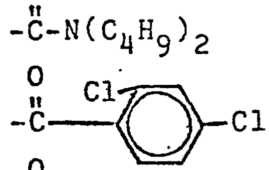 | Cis | | |
| 4.9 | 2 | 2,6-dimethyl | 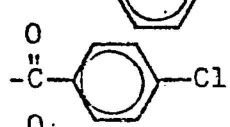 | Cis | | |
| 4.10 | 2 | 2,6-dichlor | 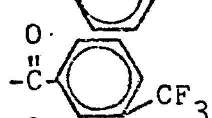 | Cis | | |
| 4.11 | 2 | 2,6-dimethyl | 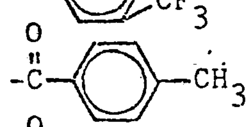 | Cis | | |
| 4.12 | 0 | - | 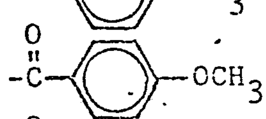 | Cis | | |
| 4.13 | 2 | 2,6-dichlor | 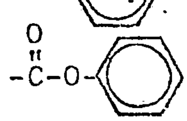 | Cis | | |

Beispiel 15

Cis-2-(2,4-Dichlorphenyl)-2-(1H-imidazol-1-ylmethyl)-4-/2-chlor-4-(4-formylpiperazin-1-ylmethyl)-6-methylphenoxymethyl7-1,3-dioxolan

Die Lösung von 5,5 g = 10 mmol der Verbindung aus Beispiel 8 in 20 ml Ameisensäureethylester kocht man 24 Stunden am Rückfluß und zieht nach dem Abkühlen das Lösungsmittel im Vakuum am Rotationsverdampfer ab. Der Rückstand wird säulenchromatographiert, wie in Beispiel 1a beschrieben. Man erhält 4,9 g (85 % d.Th.) als hochviskoses, nicht kristallines Öl.

| | | | | |
|---|---|---|---|---|
| Analyse: | Ber.: | C: 55,92 | Gef.: | C: 55,7 |
| $C_{27}H_{29}Cl_3N_4O_4$ | | H: 5,04 | | H: 5,1 |
| | | N: 9,66 | | N: 9,5 |

MG: 579,91

Beispiel 16

Cis-2-(2,4-Dichlorphenyl)-2-(1H-imidazol-1-ylmethyl)-4-/2,6-dimethyl-4-(4-tert.-butoxycarbonylmethylpierpazin-1-ylmethyl)-phenoxymethyl7-1,3-dioxolan

Ein Gemisch aus 5,3 g = 10 mmol der Verbindung 2.2 (vgl. Tabelle 2), 2,8 g = 20 mmol Kaliumcarbonat und 1,5 g = 10 mmol Chloressigsäure-tert.-butylester in 20 ml Dimethylformamid wird 6 Stunden bei 80°C gerührt. Die Aufarbeitung erfolgt wie in Beispiel 1a beschrieben. Man erhält 3,5 g (55 % d.Th.) als hochviskoses, nicht kristallines Öl.

| | | | | |
|---|---|---|---|---|
| Analyse: | Ber.: | C: 61,39 | Gef.: | C: 61,2 |
| $C_{33}H_{42}Cl_2N_4O_5$ | | H: 6,56 | | H: 6,6 |
| | | N: 8,68 | | N: 8,6 |

MG: 645,63

Beispiel 17

Gemäß Beispiel 16, jedoch unter Einsatz äquivalenter Mengen der Verbindungen aus Beispiel 8 und Brommethyl-tert.-butylketon bzw. Chloressigsäure-N-butylamid, werden folgende Verbindungen (Id') hergestellt:
Cis-2-(2,4-Dichlorphenyl)-2-(1H-imidazol-1-ylmethyl)-4-[2-chlor-6-methyl-4-(4-tert.-butylcarboxymethylpiperazin-1-ylmethyl)-phenoxymethyl]-1,3-dioxolan.
Man erhält 4 g (62 % d.Th.) als hochviskoses, nicht kristallines Öl.

| | | | | |
|---|---|---|---|---|
| Analyse: | Ber.: | C: 59,13 | Gef.: | C: 58,9 |
| $C_{32}H_{39}Cl_3N_4O_4$ | | H: 6,05 | | H: 5,8 |
| | | N: 8,62 | | N: 8,5 |
| MG: 650,05 | | | | |

Cis-2-(2,4-Dichlorphenyl)-2-(1H-imidazol-1-ylmethyl)-4-[2-chlor-4-(4-butylaminocarboxymethylpiperazin-1-ylmethyl)-6-methylphenoxymethyl]-1,3-dioxolan
Man erhält 2,1 g (32 % d.Th.) als hochviskoses, nicht kristallines Öl.

| | | | | |
|---|---|---|---|---|
| Analyse: | Ber.: | C: 57,79 | Gef.: | C: 57,5 |
| $C_{32}H_{40}Cl_3N_5O_4$ | | H: 6,06 | | H: 5,8 |
| | | N: 10,53 | | N: 10,1 |
| MG: 665,06 | | | | |

Beispiel 18

Cis-2-(2,4-Dichlorphenyl)-2-(1H-imidazol-1-ylmethyl)-4-[2,6-dimethyl-4-(4-hydroxyethylpiperazin-1-ylmethyl)-phenoxymethyl]-1,3-dioxolan

Durch die siedende Lösung von 10,6 g = 20 mmol der Verbindung 2.2 (vgl. Tabelle 2) in 50 ml Ethanol leitet man 1 Stunde lang Ethylenoxid, läßt abkühlen, zieht im Vakuum das Lösungsmittel ab und arbeitet den Rückstand auf, wie in Beispiel 3 beschrieben. Man erhält 4,8 g (42 % d.Th.) als hochviskoses, nicht kristallines Öl.

| | | | | |
|---|---|---|---|---|
| Analyse: | Ber.: | C: 60,52 | Gef.: | C: 60,6 |
| $C_{29}H_{36}Cl_2N_4O_4$ | | H: 6,31 | | H: 6,4 |
| | | N: 9,74 | | N: 9,5 |
| MG: 575,54 | | | | |

Beispiel 19

Cis-2-(2,4-Dichlorphenyl)-2-(1H-imidazol-1-ylmethyl)-4-[2,6-dimethyl-4-(4-butyloxyethylpiperazin-1-ylmethyl)-phenoxy-methyl]-1,3-dioxolan

Zur Suspension aus 0,3 g = 10 mmol Natriumhydrid (80 %ige Öldispersion) in 15 ml abs. Dimethylformamid tropft man unter Rühren und Kühlen die Lösung von 5,75 g = 10 mmol der vorstehend in Beispiel 18 beschriebenen Verbindung in 10 ml abs. Dimethylformamid so zu, daß die Temperatur 20°C nicht übersteigt. Bis zum Ende der Waserstoffentwicklung wird nachgerührt, bei 20°C die Lösung von 1,37 g = 10 mmol Butylbromid in 5 ml abs. Dimethylformamid zugetropft und anschließend 6 Stunden bei 80°C nachgerührt. Nach dem Abkühlen wird aufgearbeitet, wie in Beispiel 1a beschrieben. Man erhält 3,8 g (60 % d.Th.) als hochviskoses, nicht kristallines Öl.

| | | | | |
|---|---|---|---|---|
| Analyse: | Ber.: | C: 62,75 | Gef.: | C: 62,5 |
| $C_{33}H_{44}Cl_2N_4O_4$ | | H: 7,02 | | H: 7,2 |
| | | N: 8,87 | | N: 8,6 |
| MG: 631,65 | | | | |

Beispiel 20

Cis-2-(2,4-Dichlorphenyl)-2-(1H-imidazol-1-ylmethyl)-4-[4-(4-butylpiperazin-1-ylmethyl)-phenoxymethyl]-1,3-dioxolan

Ein Gemisch aus 5,03 g = 10 mmol der Verbindung 2.6 (vgl. Tabelle 2), 0,72 g = 10 mmol Butanal, 0,05 ml konz. Schwefelsäure und 1 g Raney-Nickel in 50 ml abs. Ethanol wird in einem Autoclaven bei 60°C und 100 atm. Wasserstoff 3 Stunden gerührt. Nach Abkühlung und Entspannung wird der Katalysator abfiltriert, das Lösungsmittel im Vakuum am Rotationsverdampfer abgezogen, der Rückstand in Methylenchlorid aufgenommen, mit 2 n Sodalösung gewaschen, mit Natriumsulfat getrocknet und aufgearbeit, wie in Beispiel 1a beschrieben. Man erhält 2,8 g (50 % d.Th.) als hochviskoses, nicht kristallines Öl.

| | | | | |
|---|---|---|---|---|
| Analyse: | Ber.: | C: 62,31 | Gef.: | C: 62,0 |
| $C_{29}H_{36}Cl_2N_4O_3$ | | H: 6,49 | | H: 6,5 |
| | | N: 10,02 | | N: 9,8 |
| MG: 559,11 | | | | |

Beispiel 21

Cis-2-(2,4-Dichlorphenyl)-2-(1H-imidazol-1-ylmethyl)-4-[2-chlor-6-methyl-4-(4-methylthiothiocarbonylpiperazin-1-ylmethyl)-phenoxymethyl]-1,3-dioxolan

Zur Lösung von 5,5 g = 10 mmol der Verbindung aus Beispiel 8, in 30 ml Ethanol und 1 g = 10 mmol Triethylamin, tropft man unter Rühren bei 20°C 0,76 g = 10 mmol Schwefelkohlenstoff, rührt 30 Minuten nach, tropft anschließend bei 20°C 1,26 g = 10 mmol Dimethylsulfat zu, rührt 1 Stunde bei 20°C nach und kocht dann 1 Stunde am Rückfluß. Nach dem Abkühlen zieht man im Vakuum das Lösungsmittel ab, nimmt den Rückstand in Methylenchlorid auf, wäscht mit Wasser, trocknet die organische Phase mit Natriumsulfat und arbeitet auf, wie in Beispiel 1a beschrieben. Man erhält 3,8 g (60 % d.Th.) als hochviskoses, nicht kristallines Öl.

| | | | | |
|---|---|---|---|---|
| Analyse: | Ber.: | C: 52,38 | Gef.: | C: 52,1 |
| $C_{28}H_{31}Cl_3N_4O_3S$ | | H: 4,86 | | H: 4,8 |
| | | N: 8,72 | | N: 8,6 |
| MG: 642,04 | | | | |

Beispiel 22

Cis-2-(2,4-Dichlorphenyl)-2-(1H-imidazol-1-ylmethyl)-4-/4-(4-butylthiothiocarbonylpiperazin-1-ylmethyl)-2,6-dimethylphenoxymethyl7-1,3-dioxolan

Gemäß Beispiel 21, jedoch unter Einsatz äquivalenter Mengen der Verbindung 2.2 (vgl. Tabelle 2), Schwefelkohlenstoff und n-Butylbromid erhält man die vorstehend genannte Verbindung. Man erhält 4,1 g (62 % d.Th.) als hochviskoses, nicht kristallines Öl.

| | | | | |
|---|---|---|---|---|
| Analyse: | Ber.: | C: 57,91 | Gef.: | C: 57,8 |
| $C_{32}H_{40}Cl_2N_4O_3S_2$ | | H: 6,07 | | H: 6,0 |
| | | N: 8,44 | | N: 8,5 |
| MG: 663,70 | | | | |

Beispiel 23

a) Herstellung der Aminoverbindung:
Cis-2-(2,4-Dichlorphenyl)-2-(1H-imidazol-1-ylmethyl)-4-(4-aminomethyl-phenoxymethyl)-1,3-dioxolan

Ein Gemisch aus 47,6 g = 0,1 mol der Verbindung 1.172 (vgl. Tabelle 1), 40 g = 0,5 mol 50 %ige Natronlauge und 400 ml Methylglykol kocht man unter Rühren 12 Stunden am Rückfluß, läßt abkühlen, zieht im Ölpumpenvakuum das Lösungsmittel ab, nimmt den Rückstand in Methylenchlorid auf, wäscht dreimal mit Wasser nach, trocknet die organische Phase mit Natriumsulfat und arbeitet auf, wie in Beispiel 1a beschrieben. Man erhält 18 g (41 %d.Th.) als hochviskoses, nicht kristallines Öl.

| | | | | |
|---|---|---|---|---|
| Analyse: | Ber.: | C: 58,07 | Gef.: | C: 58,2 |
| $C_{21}H_{21}Cl_2N_3O_3$ | | H: 4,87 | | H: 5,0 |
| | | N: 9,67 | | N: 9,6 |
| MG: 434,32 | | | | |

b) Herstellung des Isothiocyanates:
Cis-2-(2,4-Dichlorphenyl)-2-(1H-imidazol-1-ylmethyl)-4-(4-isothiocyanatomethyl-phenoxymethyl)-1,3-dioxolan.

Zur Lösung von 4,3 g = 10 mmol der vorstehenden Verbindung aus Beispiel 23 a in 30 ml Pyridin, tropft man unter Rühren und Kühlen bei -5°C 1 g = 13 mmol Schwefelkohlenstoff, rührt 30 Minuten nach und gibt anschließend 2,1 g = 10 mmol N,N'-Dicyclohexylcarbodiimid portionsweise so zu, daß die Temperatur -5°C nicht übersteigt. Man rührt bei dieser Temperatur 2 Stunden und anschließend bei 25°C 1 Stunde nach und zieht im Ölpumpenvakuum das Lösungsmittel ab. Der Rückstand wird in 20 ml Eisessig aufgenommen und unter Rühren und Kühlen bei 10°C mit 50 ml Wasser versetzt. Der gebildete Niederschlag wird abgesaugt, das Filtrat unter Rühren und Kühlen bei 10°C mit Natriumcarbonat neutralisiert und mit Methylenchlorid ausgeschüttelt. Die Methylenchloridphase wird über Natriumsulfat getrocknet und aufgearbeitet, wie in Beispiel 1a beschrieben. Man erhält 2,6 g (55 % d.Th.) als hochviskoses, nicht kristallines Öl.

| | | | | |
|---|---|---|---|---|
| Analyse: | Ber.: | C: 55,47 | Gef.: | C: 55,3 |
| $C_{22}H_{19}Cl_2N_3O_3S$ | | H: 4,02 | | H: 4,0 |
| | | N: 8,82 | | N: 8,6 |
| MG: 476,38 | | | | |

Beispiel 24

Gemäß Beispiel 10, jedoch unter Einsatz äquivalenter Mengen der Verbindung aus Beispiel 23a und entsprechender Verbindungen der Formel IX, können die folgenden Verbindungen (I g) hergestellt werden.

Tabelle 5:

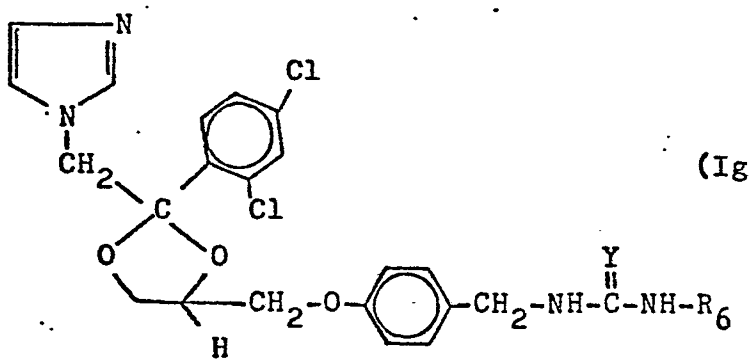

(Ig)

Für 2,4-Isomere gilt die Erklärung der Tabelle 1

| Verbdgs.-Nr. | 2,4-Isomere | Y | $R_6$ | Analyse Ber.: | Gef.: | Fp.: [°C] |
|---|---|---|---|---|---|---|
| 5.1. | Cis | S | $-CH_2-CH=CH_2$ | | | |
| 5.2. | Cis | S | -tert.-butyl | | | |
| 5.3. | Cis | S | 4-Cl $C_6H_4$ | C:55,69<br>H: 4,17<br>N: 9,28 | C:55,7<br>H: 4,3<br>N: 9,3 | |
| 5.4. | Cis | O | $2,4-Cl_2C_6H_3$ | | | |
| 5.5. | Cis | O | $C_6H_5$ | | | |

Beispiel 25

Gemäß Beispiel 13a, jedoch unter Einsatz äquivalenter Mengen der Verbindung aus Beispiel 23a und einem Acylchlorid, das sich von der entsprechenden Carbonsäure ableitet, können die folgenden Verbindungen (Ih) hergestellt werden.

Tabelle 6:

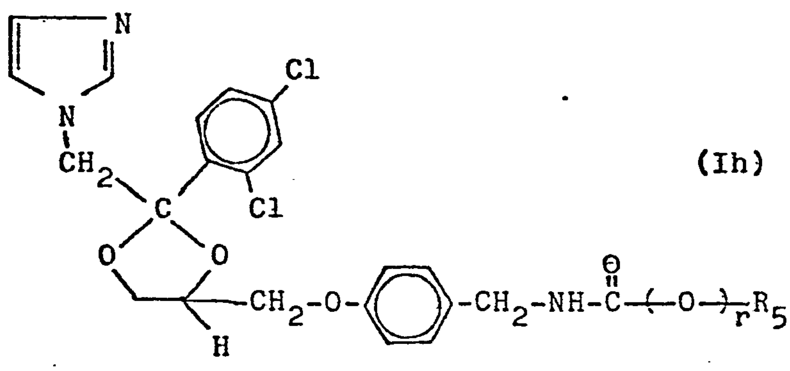

(Ih)

Für 2,4-Isomere gilt die Erklärung der Tabelle 1

| Verbdgs.-Nr. | r | $R_5$ | 2,4-Isomere | Fp.: [°C] | Analyse Ber.: | Gef.: |
|---|---|---|---|---|---|---|
| 6.1. | 0 | $-CHCl_2$ | Cis | | | |
| 6.2. | 0 | $2,4-Cl_2C_6H_3$ | Cis | | | |
| 6.3. | 0 | $3-CF_3C_6H_4$ | Cis | | | |
| 6.4. | 0 | $4-Cl\ C_6H_4$ | Cis | | C:58,70<br>H: 4,22<br>N: 7,33 | C: 58,5<br>H: 4,2<br>N: 7,1 |
| 6.5. | 0 | $4-CH_3C_6H_4$ | Cis | | | |

Beispiel 26

a) Herstellung der N-Formylverbindung:
Cis-2-(2,4-Dichlorphenyl)-2-(1H-imidazol-1-ylmethyl)-4-/4-(N-formylaminomethyl)-phenoxymethyl/-1,3-dioxolan

Gemäß Beispiel 15, jedoch unter Einsatz äquivalenter Mengen der Verbindung aus Beispiel 23a wird die vorstehend genannte Verbindung hergestellt. Man erhält 4,1 g (90 % d.Th.) als hochviskoses, nicht kristallines Öl.

| | | | | |
|---|---|---|---|---|
| Analyse: | Ber.: | C: 57,16 | Gef.: | C: 57,2 |
| $C_{22}H_{21}Cl_2N_3O_4$ | | H: 4,58 | | H: 4,4 |
| | | N: 9,09 | | N: 8,9 |

MG: 462,33

b) Herstellung des Isonitrils:
Zur Lösung von 4,62 g = 10 mmol der Verbindung aus Beispiel 26a in 20 ml Methylenchlorid und 2,5 g = 25 mmol Triethylamin, tropft man unter Rühren und Kühlen bei -10°C die Lösung von 1,54 g = 10 mmol Phosphoroxychlorid in 5 ml Methylenchlorid, läßt innerhalb 1 Stunde auf 20°C aufwärmen und rührt 30 Minuten nach. Anschließend gibt man die Lösung von 2,6 g = 25 mmol Natriumcarbonat in 20 ml Wasser zu, rührt bei 20°C 30 Minuten nach, trennt die organische Phase ab, trocknet sie mit Natriumcarbonat und zieht im Vakuum das Lösungsmittel ab. Der erhaltene Rückstand (4,2 g) wird säulenchromatographisch an Aluminiumoxid (neutral) und Methylenchlorid als Laufmittel gereinigt. Man erhält 2,9 g (65 % d.Th.) als hochviskoses, nicht kristallines Öl.

| | | | | |
|---|---|---|---|---|
| Analyse: | Ber.: | C: 59,47 | Gef.: | C: 59,5 |
| $C_{22}H_{19}Cl_2N_3O_3$ | | H: 4,31 | | H: 4,2 |
| | | N: 9,46 | | N: 9,5 |

MG: 444,30

Herstellung der Ausgangsstoffe

Zur Herstellung von Verbindungen der allgemeinen Formel (II):

Beispiel 27

Die folgenden Verbindungen der allgemeinen Formel (II) werden in Analogie zu J.Med.Chem. 1979, 22, S. 1003 und DE-OS 28 04 096, Beispiel 38 hergestellt (vgl. dazu die Reaktionsfolge (XV)⟶(XVII)⟶(XVI)⟶(XVIII)⟶(XIX)⟶(XX)⟶(II') im allgemeinen Formelschema).

Tabelle 7:

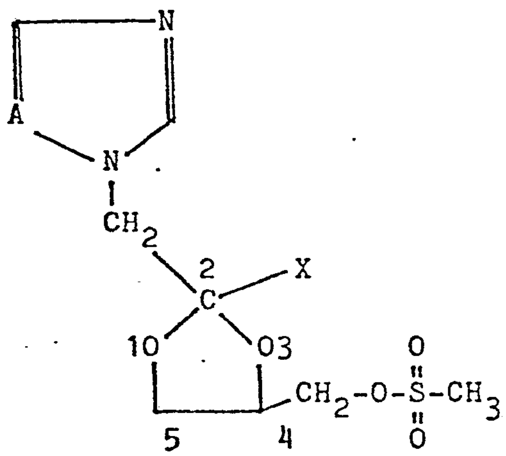

(II)

Für 2,4-Isomere gilt die Erklärung der Tabelle 1

| Verbdgs. Nr. | A | X | 2,4-Isomere | Fp.: [°C] |
|---|---|---|---|---|
| 7.1. | CH | 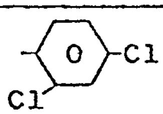 | Cis | 91-92 |
| 7.2. | CH | 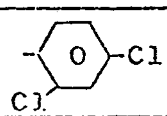 | Trans | |
| 7.3. | N | 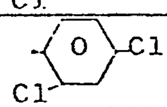 | Cis | 98 |
| 7.4. | N | 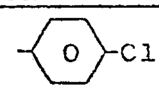 | Cis | 116 |

Zur Herstellung von Verbindungen der allgemeinen Formel (III):

Beispiel 28

4-(4-Acetylpiperazin-1-ylmethyl)-2,6-dimethylphenol

Die Lösung von 35,8 g = 0,2 mol 2,6-Dimethyl-4-(N,N-dimethylaminomethyl)-phenol und 25,6 g = 0,2 mol Acetylpiperazin in 100 ml Xylol wird 5 Stunden am Rückfluß gekocht, wobei durch die Lösung Stickstoff geleitet wird. Nach Abkühlung wird der Niederschlag abgesaugt, mit Essigester gewaschen und über Phosphorpentoxid im Vakuum getrocknet. Man erhält 46,1 g (88 % d.Th.) vom Fp.: 150 - 151°C.

Beispiel 29

Gemäß Beispiel 28, jedoch unter Einsatz äquivalenter Mengen einer entsprechenden N,N-Dimethylaminomethyl-phenol-Mannich-Base und einer Aminoverbindung der allgemeinen Formel XII, werden die folgenden Verbindungen der allgemeinen Formel (III) hergestellt:

Tabelle 8:

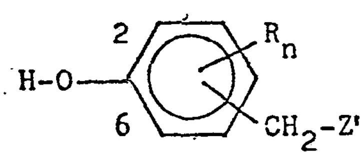

(III)

a) v gibt in der Tabelle die Position des Restes $-CH_2-Z'$ an.

| Verbdgs.-Nr. | a) v | n | $R_n$ | Z' | Fp.: [°C] |
|---|---|---|---|---|---|
| 8.1. | 2 | 1 | 4-Cl | 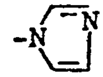 | 171 |
| 8.2. | 2 | 1 | 4-Cl | 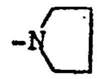 | 63-66 |
| 8.3. | 2 | 1 | 4-Cl | 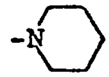 | 54-55 |
| 8.4. | 2 | 1 | 4-Cl | 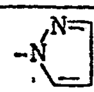 | 170-172 |
| 8.5. | 2 | 1 | 4-tert.-butyl | 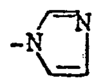 | 157 |
| 8.6. | 2 | 1 | $4-O-CH_3$ | 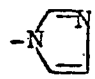 | 165 |
| 8.7. | 2 | 1 | $4-\overset{O}{\overset{\|}{C}}-O-C_2H_5$ | 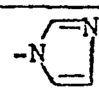 | 194 |
| 8.8. | 2 | 1 | 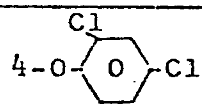 | 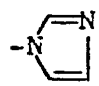 | 230-232 |
| 8.9. | 2 | 2 | $4,6-Cl_2$ | 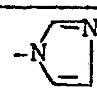 | 181-183 |
| 8.10. | 2 | 2 | $4,6-Cl_2$ | 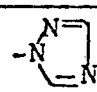 | 187-188 |
| 8.11. | 1 | 2 | 4-Cl | 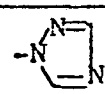 | 190 |
| 8.12. | 2 | 1 | 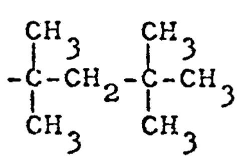 | 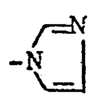 | 176 |
| 8.13. | 2 | 2 | $4,6-Cl_2$ | 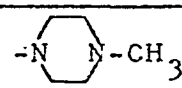 | 105 |
| 8.14. | 4 | 2 | 2,6-di-methyl | $-N(C_2H_5)_2$ | 58-59 |

| Verbdgs.-Nr. | v | n | $R_n$ | Z' | Fp.: [°C] |
|---|---|---|---|---|---|
| 8.15. | 4 | 2 | 2,6-di-methyl | 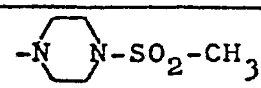 | 166-167 |
| 8.16. | 4 | 2 | 2,6-di-methyl | 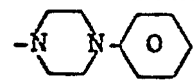 | 115-116 |
| 8.17. | 4 | 2 | 2-chlor 6-methyl | $-N(CH_3)_2$ | 110-112 |
| 8.18. | 4 | 2 | 2-chlor 6-methyl | 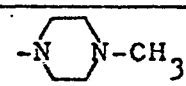 | 105 |
| 8.19. | 4 | 2 | 2-chlor 6-methyl | 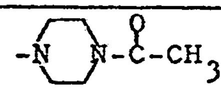 | 150-152 |
| 8.20. | 4 | 2 | 2-chlor 6-methyl | 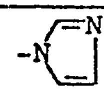 | 163-165 |
| 8.21. | 4 | 2 | 2-chlor 6-methyl | 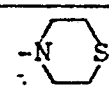 | 108-110 |
| 8.22. | 4 | 2 | 2-chlor 6-methyl | 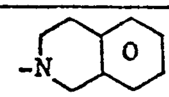 | 116 |
| 8.23. | 4 | 2 | 2,6-dichlor | $-N(CH_3)_2$ | 180-183 |
| 8.24. | 4 | 2 | 2,6-dichlor | 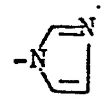 | 204 |

| Verbdgs.-Nr. | v | n | $R_n$ | Z' | Fp.: [°C] |
|---|---|---|---|---|---|
| 8.25 | 4 | 2 | 2,6-dimethyl | 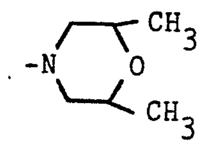 | * 119 - 131 |
| 8.26 | 4 | 2 | 2-chlor-6-methyl | 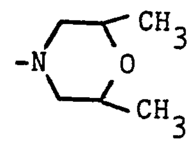 | * 124 - 136 |
| 8.27 | 4 | 2 | 2,6-dimethyl | 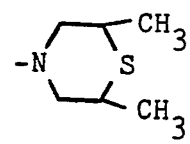 | * 125 - 138 |
| 8.28 | 4 | 2 | 2-chlor-6-methyl | 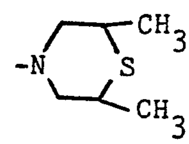 | * 125 - 138 |
| 8.29 | 4 | 2 | - | 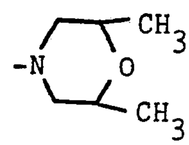 | * 98 - 106 |
| 8.30 | 4 | 2 | 2,6-dimethyl | 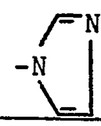 | 171 - 172 |

* Gemisch der cis- und trans-Isomeren

Beispiel 30
4-(4-Acetylpiperazin-1-ylmethyl)-phenol

Ein Gemisch aus 22,4 g = 0,2 mol p-Hydroxybenzaldehyd, 25,6 g = 0,2 mol Acetylpiperazin, 200 ml Methanol, 10 g Raney-Nickel und 0,1 ml konz. Schwefelsäure wird in einem Autoclaven bei 80°C und 100 atm. Wasserstoff 4 Stunden gerührt. Nach Abkühlung und Entspannung wird der Katalysator abfiltriert, das Lösungsmittel im Vakuum am Rotationsverdampfer abgezogen, der Rückstand in Methylenchlorid aufgenommen, mit 2 n Sodalösung gewaschen, die organische Phase mit Natriumsulfat getrocknet, das Lösungsmittel wie vorstehend abgezogen und der Rückstand aus einer Mischung aus Essigester und Diisopropylether umkristallisiert. Man erhält 33,4 g (71 % d.Th.) vom Fp.: 130 - 132°C.

Beispiel 31
4-Hydroxybenzylamin

In 500 ml Ethanol wird bei 10°C Ammoniak bis zur Sättigung eingeleitet, 122,1 g = 1 mol p-Hydroxybenzaldehyd, 20 g Raney-Nickel und 0,1 ml konz. Schwefelsäure zugegeben und die Mischung in einem Autoclaven bei Raumtemperatur und 100 atm. Wasserstoff 6 Stunden gerührt. Nach Entspannung wird der Katalysator abfiltriert, das Filtrat mit Aktivkohle aufgekocht, filtriert und eingeengt. Der Rückstand wird aus einer Mischung aus Ethanol und Ether umkristallisiert. Man erhält 104 g (85 % d.Th.) vom Fp.: 104°C.

Beispiel 32
a) Herstellung der Bisacetylverbindung:
Essigsäure(4-acetoxybenzyl)-amid

Zur Lösung von 12,3 g = 0,1 mol 4-Hydroxybenzylamin in 20 ml Pyridin tropft man unter Rühren 22,44 g = 0,22 mol Acetanhydrid so zu, daß die Temperatur 60°C nicht übersteigt, rührt anschließend bei dieser Temperatur

1 Stunde nach, zieht im Vakuum das Lösungsmittel ab, nimmt den Rückstand in Wasser auf, extrahiert mit Methylenchlorid, trocknet die organische Phase über Natriumsulfat, zieht im Vakuum das Lösungsmittel ab und kristallisiert den Rückstand aus einer Mischung aus Toluol und Äther um. Man erhält 14,65 g (73 % d.Th.) vom Fp.: 82 - 83°C.

b) Herstellung der Monoacetylverbindung:
Essigsäure-(4-hydroxybenzyl)-amid

Zur Lösung von 8,8 g 50 %iger Natronlauge = 0,11 mmol in 300 ml Ethanol trägt man unter Rühren bei 25°C 20,7 g = 0,1 mol Essigsäure-(4-acetoxybenzyl)-amid portionsweise ein, rührt 2 Stunden nach und zieht im Vakuum das Lösungsmittel ab. Der Rückstand wird in einer Mischung aus Methylenchlorid : Ethanol wie 1 : 1 aufgenommen, über Kieselgel filtriert und das Filtrat eingeengt. Der Rückstand wird aus Essigester umkristallisiert. Man erhält 14 g (85 % d.Th.) vom Fp.: 137°C.

Beispiel 33

a) Herstellung von 4-(Piperidin-1-ylethyl)-anisol:

Zur Suspension von 4,6 g = 0,12 mol Lithiumaluminiumhydrid in 250 ml abs. Ether tropft man unter Rühren die Lösung von 28 g = 0,12 mol 4-Methoxyphenylessigsäurepiperidid in 100 ml abs. Ether so zu, daß der Ether schwach siedet und kocht anschließend 6 Stunden am Rückfluß. Unter Rühren und Kühlen werden 12 ml = 0,67 mol Wasser vorsichtig zugetropft, die Rührung abgeschaltet und nach 16 Stunden abgesaugt, der Filterrückstand mit Ether gewaschen, das Filtrat im Vakuum abgezogen und der Rückstand im Ölpumpenvakuum destilliert. Man erhält 20,0 g (76 % d.Th.) vom $Kp_{0,2}$ 96 - 98°C.

b) Herstellung von 4-(Piperidin-1-ylethyl)-phenol

Die Lösung von 11 g = 50 mmol 4-(Piperidin-1-ylethyl)-anisol in einer Mischung aus 50 ml Eisessig und 50 ml 48 %iger Bromwasserstoffsäure wird 4 Stunden am Rückfluß gekocht, die Lösung im Vakuum abgezogen und der Rückstand aus Ethanol umkristallisiert. Man erhält 9 g vom Fp.: 265°C als Hydrobromid. Das Salz wird in 30 ml Wasser gelöst und die freie Base mit konzentrierter, wäßriger Ammoniaklösung ausgefällt und abgesaugt. Man erhält 5,5 g (54 % d.Th.) vom Fp.: 168 - 169°C.

Beispiel 34

Gemäß Beispiel 33a und b, jedoch unter Einsatz äquivalenter Mengen entsprechender Methoxyphenylessig- bzw.-propionsäureamiden, Lithiumaluminiumhydrid und Eisessig-Bromwasserstoffsäure werden die folgenden Hydroxyphenylalkylamine erhalten:

4-(4-Methylpiperazin-1-ylethyl)-phenol (Fp.: 125 - 126°C)
4-(N,N-Dimethylaminopropyl)-phenol (Fp.: 109 - 110°C)
4-(4-Methylpiperazin-1-ylpropyl)-phenol (Fp.: 137 - 138°C)

Beispiele für das zweite Herstellverfahren nach Anspruch 3:

Beispiel 35

a) Herstellung einer Verbindung der allgemeinen Formel (V)

2-Brommethyl-2-(4-chlorphenyl)-4-[2-chlor-4-(N,N-dimethylaminomethyl)-6-methylphenoxymethyl]-1,3-dioxolan

Zur Suspension aus 0,6 g = 20 mmol Natriumhydrid (80 %ige Öldispersion) in 30 ml abs. Dimethylformamid gibt man unter Rühren und Kühlen mit einem Eisbad 4 g = 20 mmol 2-Chlor-4-(N,N-dimethylaminomethyl)-6-methylphenol portionsweise so zu, daß die Temperatur 20°C nicht übersteigt. Man rührt bei 20°C nach bis die Wasserstoffentwicklung beendet ist, trägt anschließend 8,26 g = 20 mmol 2-Brommethyl-2-(4-chlorphenyl)-1,3-dioxolan-4-ylmethyl-methansulfonat (Verbindung der Formel (IV)) portionsweise ein, rührt 7 Stunden bei 60°C nach und arbeitet auf, wie in Beispiel 1a beschrieben. Man erhält 7 g (71 % d.Th.) als hochviskoses, nicht kristallines Öl.

| | | | | |
|---|---|---|---|---|
| Analyse: | Ber.: | C: 51,56 | Gef.: | C: 51,6 |
| $C_{21}H_{24}BrCl_2NO_3$ | | H: 4,94 | | H: 5,0 |
| | | N: 2,86 | | N: 2,5 |
| MG: 489,24 | | | | |

b) Herstellung einer Verbindung der allgemeinen Formel (I): 2-(4-Chlorphenyl)-2-(1H-imidazol-1-ylmethyl)-4-/2-chlor-4-(N,N-dimethylaminomethyl)-6-methylphenoxymethyl7-1,3-dioxolan

Zur Suspension aus 0,3 g = 10 mmol Natriumhydrid (80 %ige Öldispersion) in 20 ml Dimethylacetamid gibt man unter Rühren und Kühlen 0,68 g = 10 mmol Imidazol so zu, daß die Temperatur 20°C nicht übersteigt, rührt nach bis die Wasserstoffentwicklung beendet ist, gibt die Lösung von 4,9 g = 10 mmol der Verbindung aus Beispiel 35a in 5 ml Dimethylacetamid zu, kocht anschließend 24 Stunden am Rückfluß und arbeitet auf wie in Beispiel 1a beschrieben. Man erhält 1,7 g (35 % d.Th.) als hochviskoses, nicht kristallines Öl.

| | | | | |
|---|---|---|---|---|
| Analyse: | Ber.: | C: 60,56 | Gef.: | C: 60,6 |
| $C_{24}H_{27}Cl_2N_3O_3$ | | H: 5,71 | | H: 5,7 |
| | | N: 8,82 | | N: 8,9 |
| MG: 476,40 | | | | |

Beispiel 36

2-(4-Chlorphenyl)-2-(1H-imidazol-1-ylmethyl)-4-/4-(4-acetyl-piperazin-1-ylmethyl)-2-chlor-6-methylphenoxymethyl7-1,3-dioxolan

Zur Suspension aus 0,3 g = 10 mmol Natriumhydrid (80 %ige Öldispersion) in 20 ml Dimethylacetamid gibt man unter Rühren und Kühlen 2,8 g = 10 mmol 4-(4-Acetylpiperazin-1-ylmethyl)-2-chlor-6-methylphenol portionsweise so zu, daß die Temperatur 20°C nicht übersteigt. Anschließend trägt man 4,1 g = 10 mmol 2-Brommethyl-2-(4-chlorphenyl)-1,3-dioxolan-4-ylmethyl-methansulfonat portionsweise ein und rührt bei 80°C 6 Stunden nach. Bei dieser Temperatur werden 0,8 g = 5 mmol Kaliumjodid und 0,9 g = 10 mmol Imidazol-Natriumsalz portionsweise eingetragen, anschließend 48 Stunden bei 160°C gerührt und aufgearbeitet, wie in Beispiel 1a beschrieben. Man erhält 2 g (35 % d.Th.) als hochviskoses, nicht kristallines Öl.

| | | | | |
|---|---|---|---|---|
| Analyse: | Ber.: | C: 60,11 | Gef.: | C: 59,9 |
| $C_{28}H_{32}Cl_2N_4O_4$ | | H: 5,77 | | H: 5,5 |
| | | N: 10,01 | | N: 9,7 |
| MG: 559,6 | | | | |

Beispiel 37

Gemäß Beispiel 36, jedoch unter Einsatz äquivalenter Mengen von Verbindungen der allgemeinen Formel (IV), (III) und (VI) können die folgenden Verbindungen der allgemeinen Formel (I) hergestellt werden.

Tabelle 9:

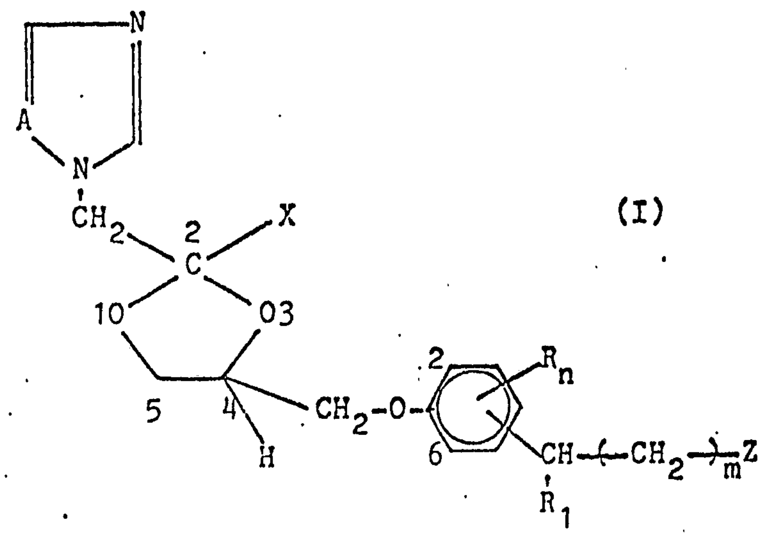

(I)

Für v und 2,4-Isomere gelten die Erklärungen der Tabelle 1.

| Verbdgs.-Nr. | A | X | n | $R_n$ | v | $R_1$ | m | Z | 2,4-Isomere | Base oder Salze | Fp.: [°C] | Analyse Ber.: | Gef.: |
|---|---|---|---|---|---|---|---|---|---|---|---|---|---|
| 9.1 | CH | $4-Cl-C_6H_4$ | 2 | 2,6-dimethyl | 4 | H | 0 | 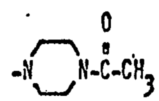 | | Base | | C: 64,61<br>H: 6,54<br>N: 10,39 | C: 64,3<br>H: 6,5<br>N: 10,1 |
| 9.2 | N | $2,4-Cl_2C_6H_3$ | 2 | 2,6-dichlor | 4 | H | 0 | $-N(C_2H_5)_2$ | cis | Base | | C: 50,27<br>H: 4,95<br>N: 10,20 | C: 50,0<br>H: 5,0<br>N: 9,8 |
| 9.3 | CH | $2,4-Cl_2C_6H_3$ | 0 | | 4 | H | 0 | $-N(C_2H_5)_2$ | cis | Base | - | C: 61,23<br>H: 5,96<br>N: 8,57 | C: 59,6<br>H: 5,5<br>N: 8,7 |

| Vorbdgs.-Nr. | A | X | n | $R_n$ | v | $R_1$ | m | Z | 2,4-Isomere | Base oder Salze | Fp.:/°C/ | Analyse Ber.: | Gef.: |
|---|---|---|---|---|---|---|---|---|---|---|---|---|---|
| 9.4 | CH | $2,4\text{-}Cl_2C_6H_3$ | 0 | | 4 | H | 0 | 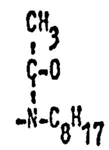 | cis | Base | - | C: 63,26<br>H: 6,68<br>N: 7,14 | C: 63,2<br>H: 6,9<br>N: 7,2 |
| 9.5 | N | $2,4\text{-}Cl_2C_6H_3$ | 2 | 2-Cl;<br>$6\text{-}CH_3$ | 4 | H | 0 | 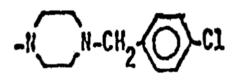 | cis | Base | | | |
| 9.6 | CH | $4\text{-}BrC_6H_4$ | 2 | 2-Cl;<br>$6\text{-}CH_3$ | 4 | H | 0 | $-N(C_2H_5)_2$ | | Base | | | |
| 9.7 | CH | $2,4\text{-}Cl_2C_6H_3$ | 0 | | 4 | H | 1 | 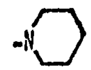 | cis | Base | 151-152 | C: 62,79<br>H: 6,05<br>N: 8,14 | C: 62,3<br>H: 6,1<br>N: 8,0 |

Weiter werden in Analogie zu Beispiel 36 die folgenden Verbindungen der allgemeinen Formel (I) hergestellt: Verbindungs-Nr.: 1.95, 1.135, 1.156, 1.172, und 1.207 (vgl. Tabelle 1).

Herstellung der Ausgangsstoffe:
Zur Herstellung von Verbindungen der allgemeinen Formel (IV):

Beispiel 38

a) Herstellung einer Verbindung der allgemeinen Formel (XVI) (vgl. Formelschema):
2-Brommethyl-2-(4-chlorphenyl)-4-hydroxymethyl-1,3-dioxolan

Die Lösung von 309,2 g = 2 mol 4-Chloracetophenon und 10 g = 0,06 mol p-Toluolsulfonsäure in einer Mischung aus 800 ml Benzol, 400 ml n-Butanol und 201,5 g = 2,2 mol Glycerin wird 48 Stunden am Wasserabscheider gekocht und in die abgekühlte Lösung bei 30°C unter Rühren 351,6 g = 112 ml = 2,2 mol $Br_2$ innerhalb 2,5 Stunden zugetropft und anschließend das Lösungsmittel im Vakuum am Rotationsverdampfer abgezogen. Der Rückstand wird in 800 ml Methylenchlorid aufgenommen und unter Kühlung mit 6 n Natronlauge gewaschen, die organische Phase mit Natriumsulfat getrocknet und das Lösungsmittel im Vakuum abgezogen. Man erhält 580 g (94 % d.Th.) viskoses Öl.

b) Herstellung einer Verbindung der allgemeinen Formel (IV):
2-Brommethyl-2-(4-chlorphenyl)-1,3-dioxolan-4-ylmethylmethansulfonat

Zur Lösung von 123 g = 0,4 mol der Verbindung aus Beispiel 38a in 150 ml Pyridin tropft man unter Rühren bei 0°C 50,4 g = 0,44 mol Methansulfochlorid zu, läßt auf 25°C

aufwärmen und rührt 4 Stunden nach. Das Reaktionsgemisch wird in 1 l Eiswasser gegossen, mit Methylenchlorid extrahiert, die organische Phase unter Kühlung mit halbkonzentrierter Salzsäure gewaschen, über Natriumcarbonat getrocknet, das Lösungsmittel im Vakuum abgezogen, der Rückstand in Methanol aufgenommen, der Niederschlag abgesaugt und aus Methanol umkristallisiert. Man erhält 64,7 g (42 % d.Th.) vom Fp.: 107 - 108°C.

Beispiel 39

Gemäß Beispiel 38b, jedoch unter Einsatz äquivalenter Mengen von Verbindungen der Formel (XVI) und entspechenden Sulfonsäurechloriden können die folgenden Verbindungen der allgemeinen Formel (IV) hergestellt werden:

Tabelle 10:

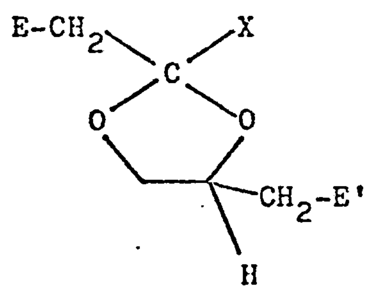

(IV)

Cis und Trans bezieht sich auf den $E-CH_2-$ und $E'-CH_2-$Rest in der 2- bzw. 4-Stellung des Dioxolanringes.

| Verbdgs.-Nr. | E | E' | X | 2,4-Isomere | Fp.:[°C] | Analyse Ber.: | Gef.: |
|---|---|---|---|---|---|---|---|
| 10.1. | Br | $-O-SO_2-CH_3$ | $2,4-Cl_2C_6H_3$ | Cis | 84 | C:34,31<br>H: 3,12 | C:34,1<br>H: 3,2 |
| 10.2. | Br | $-O-SO_2$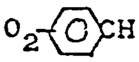$CH_3$ | $2,4-Cl_2C_6H_3$ | Cis | Öl | C:43,57<br>H: 3,45 | C:43,4<br>H: 3,5 |
| 10.3. | Br | $-O-SO_2-CH_3$ | $4-BrC_6H_4$ | | | | |
| 10.4. | Br | $-O-SO_2-CH_3$ | $2,4-Cl_2C_6H_3$ | Trans | | | |
| 10.5. | Br | $-O-SO_2$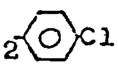Cl | $4-CH_3C_6H_4$ | | | | |
| 10.6. | Br | $-O-SO_2-CH_3$ | 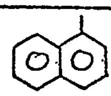 | | | | |
| 10.7. | Br | $-O-SO_2$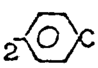Cl | 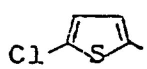 | | | | |
| 10.8. | Cl | $-O-SO_2-CH_3$ | $2,4Cl_2C_6H_3$ | Cis | | | |
| 10.9. | Br | $-O-SO_2$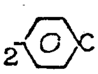Cl | $4-CH_3-O-C_6H_4$ | | | | |
| 10.10. | Br | $-O-SO_2-CH_3$ | $4-FC_6H_4$ | | 79-80 | C: 39,04<br>H: 3,82 | C: 39,0<br>H: 3,8 |
| 10.11. | Br | $-O-SO_2$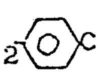Cl | $4-CF_3-C_6H_4$ | | | | |

Beispiele für das dritte Herstellverfahren nach Anspruch 4:

Beispiel 40

Cis-trans-2-(2,4-Dichlorphenyl)-2-(1H-imidazol-1-ylmethyl)-4-/2-chlor-4-(N,N-dimethylaminomethyl)-6-methylphenoxymethyl7-1,3-dioxolan

Die Mischung aus 30 ml Xylol, 20 ml Methylglykol und 8 g = 42 mmol p-Toluolsulfonsäure · $H_2O$ wird zunächst am Wasserabscheider wasserfrei gekocht. In die abgekühlte Lösung gibt man anschließend 5,1 g = 20 mmol 2,4-Dichlorphenylimidazol-1-ylmethylketon und 5,5 g = 20 mmol 1-/2-Chlor-4-(N,N-dimethylaminomethyl)-6-methylphenoxy7-2,3-propandiol und kocht weitere 48 Stunden am Wasserabscheider. Nach dem Abkühlen wird das Lösungsmittel im Vakuum abgezogen, der Rückstand in 50 ml 2 n Natronlauge aufgenommen, mit Methylenchlorid extrahiert, die organische Phase mit Natriumsulfat getrocknet und aufgearbeitet, wie in Beispiel 1a beschrieben. Man erhält hochviskoses, nicht kristallines Öl.

| | | | | |
|---|---|---|---|---|
| Analyse: | Ber.: | C: 56,42 | Gef.: | C: 55,8 |
| $C_{24}H_{26}Cl_3N_3O_3$ | | H: 5,13 | | H: 5,2 |
| | | N: 8,26 | | N: 8,0 |

MG: 510,85

Beispiel 41

Gemäß Beispiel 40, jedoch unter Einsatz äquivalenter Mengen 2,4-Dichlorphenyl-imidazol-1-ylmethylketon und entsprechender Propan-2,3-diole werden die folgenden Verbindungen der allgemeinen Formel (I) hergestellt:
Verbindungs-Nr.: 1.135, 1.172 und 1.207 (vgl. Tabelle 1).

Patentansprüche:

1. 1-(1,3-Dioxolan-2-ylmethyl)-azole der allgemeinen Formel (I)

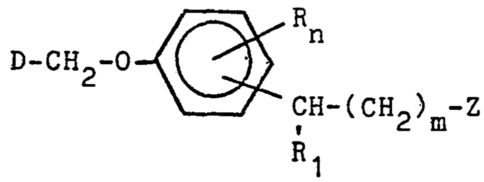

(I)

sowie ihre Stereoisomeren und ihre Salze mit physiologisch verträglichen Säuren,

in der

D einen 1-(1,3-Dioxolan-2-ylmethyl)-azol-Rest der folgenden Struktur bedeutet,

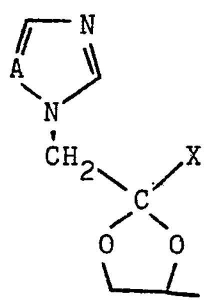

(D)

in welcher

A Stickstoff oder Methin,

X Naphthyl, Thienyl, Halothienyl, oder eine gegebenenfalls 1, 2 oder 3 Substituenten tragende Phenylgruppe, wobei die Substituenten gleich oder verschieden sind und Halogen, Trifluormethyl, $C_1$-$C_4$-Alkyl, $C_1$-$C_4$-Alkoxy bedeuten,

und wobei in Formel (I) weiterhin

$R_n$ unabhängig voneinander Halogen, Trifluormethyl, $C_1$-$C_8$-Alkyl, $C_1$-$C_4$-Alkoxy, $C_3$-$C_5$-Alkenyl, $C_1$-$C_4$-Alkoxycarbonyl, Carboxyl, Di-($C_1$-$C_4$)-Alkylaminomethyl oder Nitro bedeuten,

n = 0, 1, 2 oder 3 ist,

oder für den Fall, daß n = 2 ist,

$R_n$ einen $C_4H_4$-Rest bedeutet, der zusammen mit dem Phenylring einen Naphthylring bildet,

oder für den Fall, daß n = 1 ist,

$R_n$ eine gegebenenfalls 1 oder 2 Substituenten tragende Phenoxygruppe darstellt, wobei die Substituenten gleich oder verschieden sind und Halogen, Trifluormethyl, $C_1$-$C_4$-Alkyl, bevorzugt $C_1$-$C_2$-Alkyl oder $C_1$-$C_4$-Alkoxy, bevorzugt $C_1$-$C_2$-Alkoxy bedeuten,

$R_1$ Wasserstoff, $C_1$-$C_4$-Alkyl, oder eine gegebenenfalls 1 oder 2 Substituenten tragende Phenylgruppe, wobei die Substituenten gleich oder verschieden sind und Halogen, Trifluormethyl, Nitro, $C_1$-$C_4$-Alkyl oder $C_1$-$C_4$-Alkoxy bedeuten,

m = 0, 1 oder 2 bedeutet,

Z entweder

a) einen Aminorest der allgemeinen Formel Z(a)

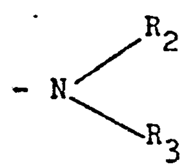 Z(a)

in der $R_2$ und $R_3$ gleich oder verschieden sind und jeweils Wasserstoff, $C_1$-$C_8$-Alkyl, $C_3$-$C_5$-Alkenyl, $C_3$-$C_8$-Cycloalkyl oder eine gegebenenfalls 1, 2 oder 3 Substituenten tragende Phenyl- bzw. Benzylgruppe bedeuten, wobei die Substituenten gleich oder verschieden sind und jeweils Halogen, $C_1$-$C_4$-Alkyl, $C_1$-$C_4$-Alkoxy oder Trifluormethyl bedeuten, oder einer der beiden Reste $R_2$ oder $R_3$ $C_1$-$C_5$-Alkanoyl oder $C_1$-$C_4$-Alkoxycarbonyl bedeutet, oder

b) einen Rest der allgemeinen Formel Z(b)

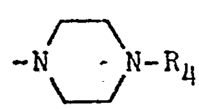 Z(b)

in der $R_4$ Wasserstoff, $C_1$-$C_4$-Alkyl, $C_3$-$C_5$-Alkenyl, Hydroxy-($C_2$-$C_3$)-alkyl, bevorzugt Hydroxyethyl, $C_1$-$C_4$-Alkoxy-($C_2$-$C_3$)-alkyl, bevorzugt $C_1$-$C_4$-Alkoxyethyl, $C_1$-$C_5$-Alkanoyl-, $C_2$-$C_5$-Alkanoylmethyl, $C_1$-$C_4$-Alkylsulfonyl, $C_1$-$C_4$-Alkyloxycarbonyl, $C_1$-$C_4$-Alkyloxycarbonylmethyl, Mono- und Di-($C_1$-$C_4$)-alkylaminocarbonylmethyl, Aminocarbonylmethyl-, Mono- und Di-($C_1$-$C_4$)alkylaminocarbonyl, $C_1$-$C_4$-Alkylaminothiocarbonyl, $C_1$-$C_4$-Alkylthiothiocarbonyl, Aminocarbonyl, $C_3$-$C_5$-Alkenylaminocarbonyl, bevorzugt N-Allylaminocarbonyl, $C_3$-$C_5$-Alkenylaminothiocarbonyl, bevorzugt N-Allylaminothiocarbonyl, oder eine Phenyl-, Phenylmethyl-, Phenylaminocarbonyl-, Phenylaminothiocarbonyl-, bzw. Benzoyl-Gruppe bedeutet, wobei jede der Phenylgruppen gegebenenfalls 1 oder 2 Substituenten tragen, die gleich oder verschieden sind und Halogen, Trifluormethyl, $C_1$-$C_4$-Alkyl, insbesondere $C_1$-$C_2$-Alkyl, oder $C_1$-$C_4$-Alkoxy, insbesondere $C_1$-$C_2$-Alkoxy bedeuten,
oder

c) einen 1-H-Imidazol-1-yl-, 1-H-1,2,4-Triazol-1-yl-, Pyrazol-1-yl-, Pyrrolidin-1-yl-, Piperidin-1-yl-, Morpholin-4-yl, Thiomorpholin-4-yl-, 2,6-Dimethylmorpholin-4-yl, 2,6-Dimethylthiomorpholin-4-yl-oder einen 1,2,3,4-Tetrahydrochinolin-1-yl oder 1,2,3,4-Tetrahydroisochinolin-2-yl-Rest bedeutet,
oder

d) eine Isocyangruppe der Formel Z(d)

$$-\bar{N} = \bar{C} \qquad Z(d)$$

oder

e) eine Isothiocyangruppe der Formel Z(e)

$$- N = C = S \qquad Z(e)$$

oder

f) einen Rest der allgemeinen Formel Z(f)

$$-NH-\overset{\overset{\large Y}{\|}}{C}-(G)_r-R_5 \qquad Z(f)$$

in der Y Sauerstoff oder Schwefel, G Sauerstoff oder eine NH-Gruppe, r 0 oder 1 und $R_5$ Wasserstoff, $C_1$-$C_4$-Alkyl, Mono-, Di- oder Trihalogenmethyl oder eine gegebenenfalls 1 oder 2 Substituenten tragende Phenylgruppe darstellen, wobei die Substituenten gleich oder verschieden sind und jeweils Halogen, Trifluormethyl, $C_1$-$C_4$-Alkyl, insbesondere $C_1$-$C_2$-Alkyl, oder $C_1$-$C_4$-Alkoxy, insbesondere $C_1$-$C_2$-Alkoxy bedeuten, mit der Maßgabe, daß für den Fall, daß Y ein Schwefelatom darstellt, G eine NH-Gruppe und r die Zahl 1 bedeuten, daß für den Fall, daß G ein Sauerstoffatom und r die Zahl 1 darstellen, $R_5$ keinen Wasserstoff bedeutet, und für den Fall, daß $R_5$ eine Mono-, Di- oder Trihalogenmethyl darstellt, r 0 und Y Sauerstoff, bedeuten.

2. Verfahren zur Herstellung der Verbindungen der allgemeinen Formel (I) in Anspruch 1, dadurch gekennzeichnet, daß man eine Verbindung der allgemeinen Formel (II)

$$D - CH_2 - E \qquad (II)$$

in der D die in Anspruch 1 angegebene Bedeutung hat und E einen reaktionsfähigen Esterrest bedeutet, mit einer Verbindung der allgemeinen Formel (III)

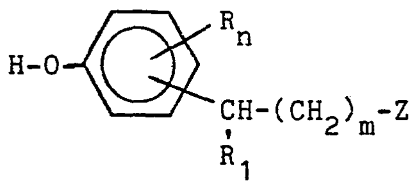

(III)

umsetzt, in der $R_n$, $R_1$, m und Z die in Anspruch 1 angegebenen Bedeutungen haben und gegebenenfalls die so erhaltenen Verbindungen der Formel (I) acyliert oder alkyliert, gewünschtenfalls in Gegenwart von Schwefelkohlenstoff.

3. Verfahren zur Herstellung von Verbindungen der allgemeinen Formel (I) in Anspruch 1, dadurch gekennzeichnet, daß man eine Verbindung der allgemeinen Formel (IV),

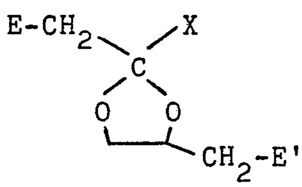

(IV)

in der X die in Anspruch 1 und E bzw. E' die zur Formel (II) in Anspruch 2 für E angegebenen Bedeutungen haben, zunächst mit einer Verbindung der allgemeinen Formel (III) in Anspruch 2 umsetzt und hierbei eine Verbindung der allgemeinen Formel (V) herstellt,

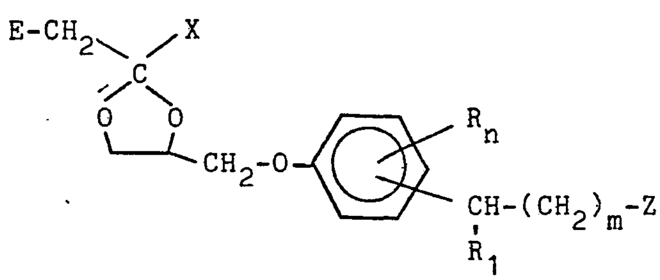

(V)

in der X, $R_n$, $R_1$, m und Z die in Anspruch 1, und E die zur Formel (II) in Anspruch 2 angegebenen Bedeutungen haben, und anschließend eine Verbindung der Formel (V) mit einer Verbindung der allgemeinen Formel (VI) umsetzt,

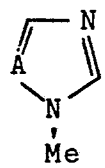

(VI)

in der A die in Anspruch 1 angegebenen Bedeutungen hat und Me Wasserstoff oder ein Metallatom bedeutet, und gegebenenfalls die so erhaltenen Verbindungen der Formel (I) acyliert oder alkyliert, gewünschtenfalls in Gegenwart von Schwefelkohlenstoff.

4. Verfahren zur Herstellung von Verbindungen der allgemeinen Formel (I) in Anspruch 1, dadurch gekennzeichnet, daß man eine Verbindung der allgemeinen Formel (VII)

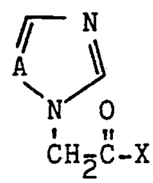

(VII)

in der A und X die in Anspruch 1 angegebenen Bedeutungen haben, mit einem 1,2-Diol der allgemeinen Formel (VIII) umsetzt,

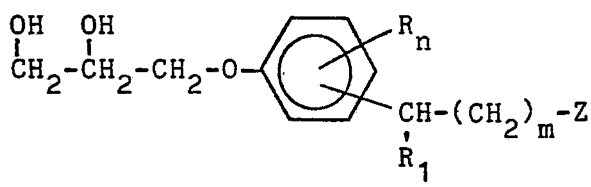

(VIII)

in der $R_n$, $R_1$, m und Z die zur Formel (I) in Anspruch 1 angegebenen Bedeutungen haben, oder mit dem Entsprechenden Epoxid umsetzt, und gegebenenfalls die so erhaltenen Verbindungen der Formel (I) acyliert oder alkyliert, gewünschtenfalls in Gegenwart von Schwefelkohlenstoff.

5. Pharmazeutische Zubereitung, dadurch gekennzeichnet, daß sie eine Verbindung gemäß Anspruch 1 enthält oder aus ihr besteht.

6. Verfahren zur Herstellung einer pharmazeutischen Zubereitung nach Anspruch 5, dadurch gekennzeichnet, daß man eine Verbindung der Formel (I) oder eines ihrer Salze, gegebenenfalls zusammen mit einem üblichen pharmazeutischen Trägerstoff und/oder Hilfsstoff in eine für pharmazeutische Zwecke geeignete Darreichungsform bringt.

7. Verwendung einer Verbindung gemäß Anspruch 1 zur Bekämpfung von Mykosen, Protozoen und grampositiver und gramnegativer Bakterien.

8. Verbindungen der allgemeinen Formel (III')

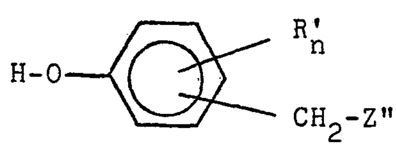

(III')

in der

$R'_n$ unabhängig voneinander Halogen, Trifluormethyl, $C_1$-$C_8$-Alkyl, $C_1$-$C_4$-Alkoxy, $C_3$-$C_5$-Alkenyl, $C_1$-$C_4$-Alkoxycarbonyl oder Nitro bedeuten,

n = 0, 1 oder 2 ist, und

Z" Acetylpiperazin, 2,6-Dimethylmorpholin-4-yl oder 2,6-Dimethylthiomorpholin-4-yl bedeutet, und für den Fall daß n = 2 ist, zusätzlich Z" Imidazol-1-yl oder 1,2,4-Trialzol-1-yl bedeutet.

9. Verfahren zur Herstellung von Verbindungen der allgemeinen Formel (III') in Anspruch 8, dadurch gekennzeichnet, daß man eine Verbindung der allgemeinen Formel (III")

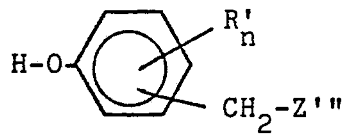

(III")

in der $R'_n$ die zur Formel (III') in Anspruch 8 angegebenen Bedeutungen haben und Z'" Dimethyl- oder Diäthylamino bedeutet, mit einer Verbindung der Formel (XII')

H - Z" (XII')

umsetzt, in der Z" die zur Formel (III') in Anspruch 8 angegebene Bedeutung hat.